# EUROPEAN PATENT SPECIFICATION

(11) **EP 2 524 049 B1**
(45) Date of publication and mention of the grant of the patent: **28.10.2015**
(21) Application number: 11702725.0
(22) Date of filing: 12.01.2011
(51) Int. Cl.: C12N 9/90, C12N 9/00, C12P 7/64

(54) **SCATTERED BRANCHED-CHAIN FATTY ACIDS AND BIOLOGICAL PRODUCTION THEREOF**
VERSTREUTE FETTSÄUREN MIT VERZWEIGTEN KETTEN UND BIOLOGISCHE HERSTELLUNG DAVON
ACIDES GRAS DISPERSÉS À CHAÎNE RAMIFIÉE ET PRODUCTION BIOLOGIQUE ASSOCIÉE

(30) Priority: 11.01.2011 US 201113004077; 12.01.2010 US 294274 P
(43) Date of publication of application: 21.11.2012
(73) Proprietor: THE PROCTER & GAMBLE COMPANY, Cincinnati, OH 45202 (US)
(72) Inventor: SAUNDERS, Charles, Winston, Fairfield, Ohio 45014 (US); XU, Jun, Mason, Ohio 45040 (US); LAUGHLIN, Leo, Timothy, II, Mason, Ohio 45040 (US); KHAMBATTA, Zubin, Sarosh, Fairfield, Ohio 45014 (US); GREEN, Phillip, Richard, Wyoming, Ohio 45231 (US)
(74) Representative: Pickford, James Lawrence
(86) International application number: PCT/US2011/020948
(87) International publication number: WO 2011/088088

(56) References cited:
- WO-A1-99/18217
- WO-A2-2007/136762
- KOLATTUKUDY P E ET AL: "Developmental pattern of the expression of malonyl-CoA decarboxylase gene and the production of unique lipids in the goose uropygial glands", ARCHIVES OF BIOCHEMISTRY AND BIOPHYSICS, ACADEMIC PRESS, US, vol. 256, no. 2, 1 August 1987 (1987-08-01), pages 446-454, XP024762632, ISSN: 0003-9861, DOI: DOI:10.1016/0003-9861(87)90601-1 [retrieved on 1987-08-01]
- LIN TING-WAN ET AL: "Structure-based inhibitor design of AccD5, an essential acyl-CoA carboxylase carboxyltransferase domain of Mycobacterium tuberculosis", PROCEEDINGS OF THE NATIONAL ACADEMY OF SCIENCES OF THE UNITED STATES OF AMERICA, vol. 103, no. 9, February 2006 (2006-02), pages 3072-3077, XP002632256, ISSN: 0027-8424
- BLOMQUIST G J ET AL: "Methyl-branched fatty acids and their biosynthesis in the housefly, Musca domestica L. (Diptera: Muscidae)", INSECT BIOCHEMISTRY AND MOLECULAR BIOLOGY, ELSEVIER SCIENCE LTD, GB, vol. 24, no. 8, 1 September 1994 (1994-09-01), pages 803-810, XP023550486, ISSN: 0965-1748, DOI: DOI:10.1016/0965-1748(94)90108-2 [retrieved on 1994-09-01]
- SCAIFE J R ET AL: "UTILIZATION OF METHYL MALONATE FOR THE SYNTHESIS OF BRANCHED CHAIN FATTY-ACIDS BY PREPARATIONS OF CHICKEN LIVER AND SHEEP ADIPOSE TISSUE", BIOCHEMICAL JOURNAL, vol. 176, no. 3, 1978, pages 799-804, XP002632257, ISSN: 0264-6021
- CARBALLEIRA N M ET AL: "Characterization of novel methyl-branched chain fatty acids from a halophilic Bacillus species.", JOURNAL OF NATURAL PRODUCTS FEB 2001 LNKD- PUBMED:11430016, vol. 64, no. 2, February 2001 (2001-02), pages 256-259, XP002632258, ISSN: 0163-3864
- KOMUNIECKI R ET AL: "Anaerobic metabolism in Ascaris suum: acyl CoA intermediates in isolated mitochondria synthesizing 2-methyl branched-chain fatty acids", MOLECULAR AND BIOCHEMICAL PARASITOLOGY, ELSEVIER SCIENCE PUBLISHERS, AMSTERDAM, NL, vol. 24, no. 2, 1 June 1987 (1987-06-01), pages 147-154, XP023700360, ISSN: 0166-6851, DOI: DOI:10.1016/0166-6851(87)90101-0 [retrieved on 1987-06-01]
- SAVVI SUZANA ET AL: "Functional characterization of a vitamin B-12-dependent methylmalonyl pathway in Mycobacterium tuberculosis: Implications for propionate metabolism during growth on fatty acids", JOURNAL OF BACTERIOLOGY, vol. 190, no. 11, June 2008 (2008-06), pages 3886-3895, XP002632259, ISSN: 0021-9193
- BURGAL, JULIE ET AL: "Metabolic engineering of hydroxy fatty acid production in plants: RcDGAT2 drives dramatic increases in ricinoleate levels in seed oil", PLANT BIOTECHNOLOGY JOURNAL, [Online] 1 October 2008 (2008-10-01), XP002632260,
- BUCKNER J S ET AL: "PURIFICATION AND PROPERTIES OF MALONYL COENZYME A DECARBOXYLASE EC-4.1.1.9 A REGULATORY ENZYME FROM THE UROPYGIAL GLAND OF GOOSE", ARCHIVES OF BIOCHEMISTRY AND BIOPHYSICS, vol. 117, 1 January 1976 (1976-01-01), pages 539-551, XP002632295,
- KIM Y S ET AL: "MALONYL COENZYME A DECARBOXYLASE FROM MYCOBACTERIUM-TUBERCULOSIS AND PSEUDOMONAS-FLUORESCENS", ARCHIVES OF BIOCHEMISTRY AND BIOPHYSICS, vol. 196, no. 2, 1979, pages 543-551, XP002632261, ISSN: 0003-9861

## Description

### FIELD OF THE INVENTION

The invention relates to cells and methods for producing fatty acids, and more particularly relates to cells and methods for producing scattered branched-chain fatty acids.

### BACKGROUND OF THE INVENTION

Branched-chain fatty acids are carboxylic acids with a methyl or ethyl branch on one or more carbons that can be either chemically synthesized or isolated from certain animals and bacteria. While certain bacteria, such as *Escherichia coli,* do not naturally produce branched-chain fatty acids, some bacteria, such as members of the genera *Bacillus* and *Streptomyces,* can naturally produce these fatty acids. For example, *Streptomyces avermitilis* and *Bacillus subtilis* both produce branched-chain fatty acids with from 14 to 17 total carbons, with the branches in the iso and anteiso positions (Cropp et al., Can. J. Microbiology 46: 506-14 (2000); De Mendoza et al., Biosynthesis and Function of Membrane Lipids, in Bacillus subtilis and Other Gram-Positive Bacteria, Sonenshein and Losick, eds., American Society for Microbiology (1993)). However, these organisms do not produce branched-chain fatty acids in amounts that are commercially useful. Another limitation of these natural organisms is that they apparently do not produce medium-chain branched-chain fatty acids, such as those with 11 or 13 carbons. In addition, if fatty acids having particular chain lengths, branches on particular carbons, or branches at positions other than the iso and anteiso positions are desired, these fatty acids may not be available or easily isolated from a natural organism in meaningful quantities.

WO9918217A1 discloses an improved method for the fermentative production of branched-chain fatty acids in transgenic plant calls which are forced to use the Me-malonyl-coA precursor.

As such, there remains a need for commercially useful, bacterially-produced, branched-chain fatty acids. In addition, there remains a need for a method of producing such branched-chain fatty acids.

### SUMMARY OF THE INVENTION

A method for producing branched-chain fatty acid comprising a methyl on one or more even number carbons, the method comprising culturing a cell comprising
(aa) an exogenous or overexpressed polynucleotide comprising a nucleic acid sequence encoding a polypeptide that catalyzes the conversion of propionyl-CoA to methylmalonyl-CoA and/or (bb) an exogenous or overexpressed polynucleotide comprising a nucleic acid sequence encoding a polypeptide that catalyzes the conversion of succinyl-CoA to methylmalonyl-CoA,
   under conditions allowing expression of the polynucleotide(s) and production of branched-chain fatty acid, wherein the cell produces more branched-chain fatty acid comprising a methyl on one or more even number carbons than an otherwise similar cell that does not comprise the polynucleotide(s);
wherein the cell is *Escherichia coli;*
and wherein the polypeptide that catalyzes the conversion of propionyl-CoA to methylmalonyl-CoA is a propionyl-CoA carboxylase and/or the polypeptide that catalyzes the conversion of succinyl-CoA to methylmalonyl-CoA is a methylmalonyl-CoA mutase.

A cell comprising:
(i) an exogenous or overexpressed polynucleotide comprising a nucleic acid sequence encoding an acyl transferase lacking polyketide synthesis activity, and
(ii) an exogenous or overexpressed polynucleotide comprising a nucleic acid sequence encoding a propionyl-CoA carboxylase and/or an exogenous or overexpressed polynucleotide comprising a nucleic acid sequence encoding a methylmalonyl-CoA mutase,
wherein the polynucleotide(s) are expressed and the cell produces more branched-chain fatty acid comprising a methyl on one or more even number carbons than an otherwise similar cell that does not comprise the polynucleotide(s);
wherein the cell is *Escherichia coli.*

### BRIEF DESCRIPTION OF THE DRAWINGS

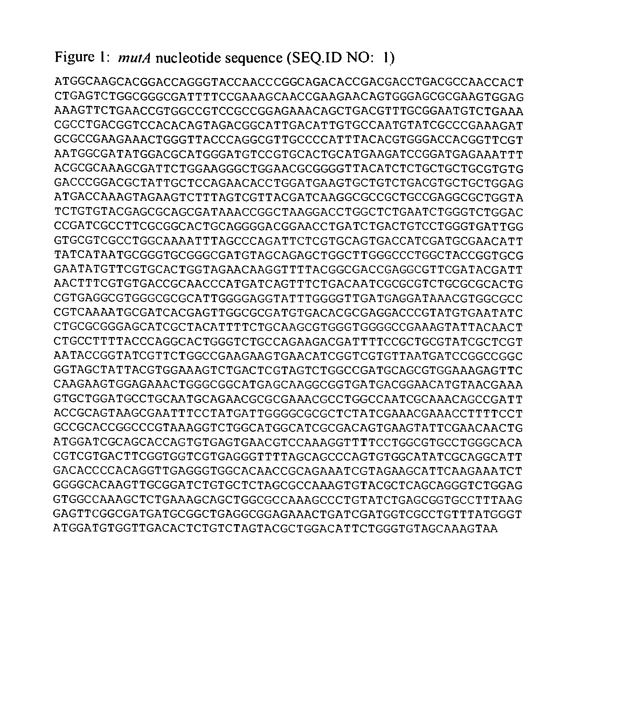

Figure 1 is a *mutA* nucleotide sequence (SEQ ID NO: 1).

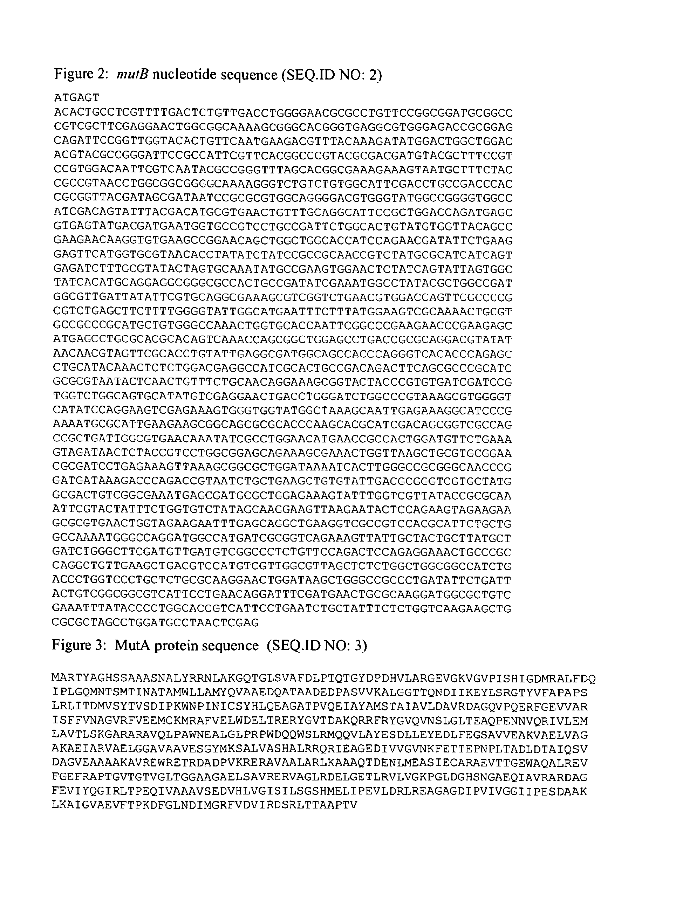

Figure 2 is a *mutB* nucleotide sequence (SEQ ID NO: 2).
Figure 3 is a MutA protein sequence (SEQ ID NO: 3).

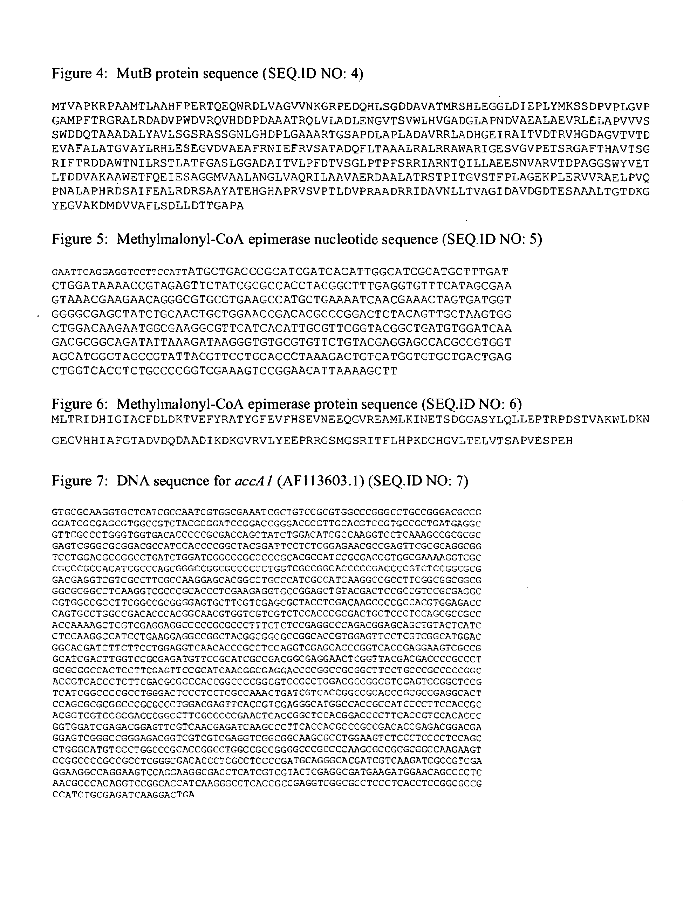

Figure 4 is a MutB protein sequence (SEQ ID NO: 4).
Figure 5 is a methylmalonyl-CoA epimerase nucleotide sequence (SEQ ID NO: 5).
Figure 6 is a methylmalonyl-CoA epimerase protein sequence (SEQ ID NO: 6).
Figure 7 is a DNA sequence for *accA1* (GenBank Accession No. AF113603.1) (SEQ ID NO: 7).

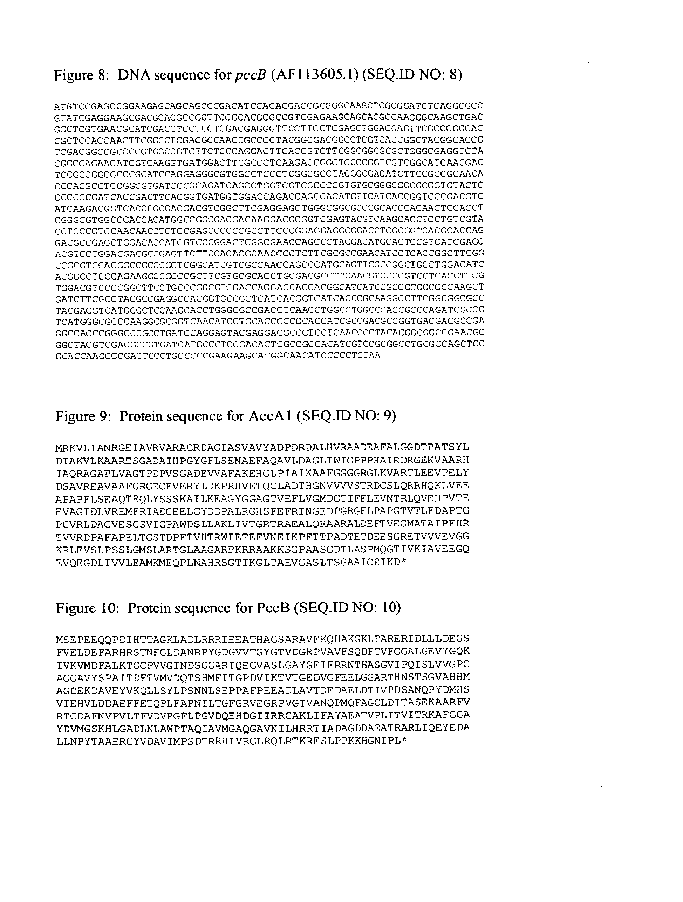

Figure 8 is a DNA sequence for *pccB* (GenBank Accession No. AF113605.1) (SEQ ID NO: 8).
Figure 9 is a protein sequence for AccA1 (SEQ ID NO: 9).
Figure 10 is a protein sequence for PccB (SEQ ID NO: 10).

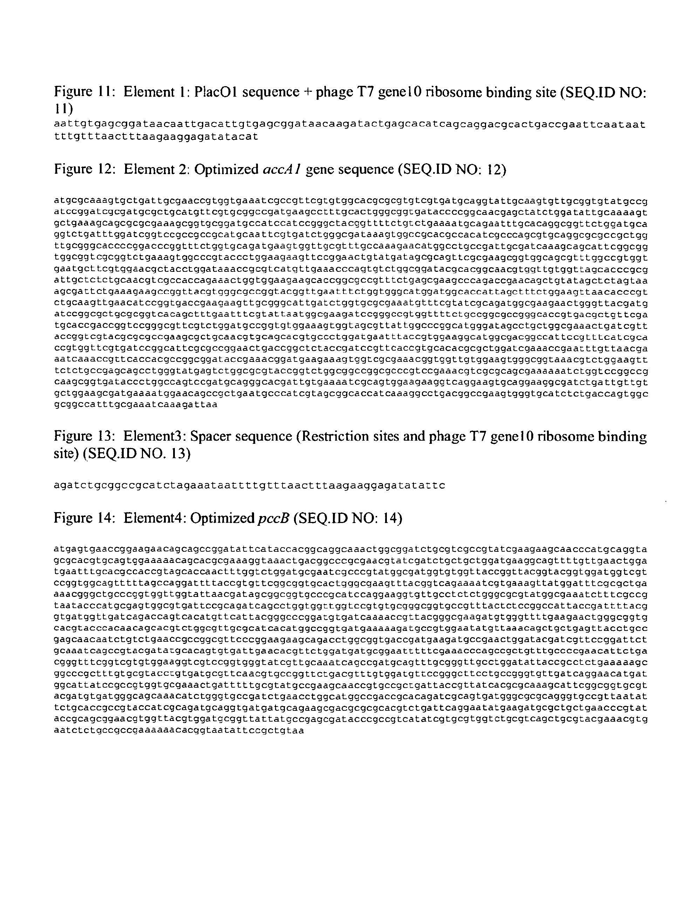

Figure 11 shows element 1 including the P_{Llac0-1} sequence and the phage T7 gene 10 ribosome binding site (SEQ ID NO: 11).
Figure 12 shows element 2 including the optimized *accA1* gene sequence (SEQ ID NO: 12).
Figure 13 shows element 3 including the spacer sequence (SEQ ID NO: 13).
Figure 14 shows element 4 including the optimized *pccB* sequence (SEQ ID NO: 14).

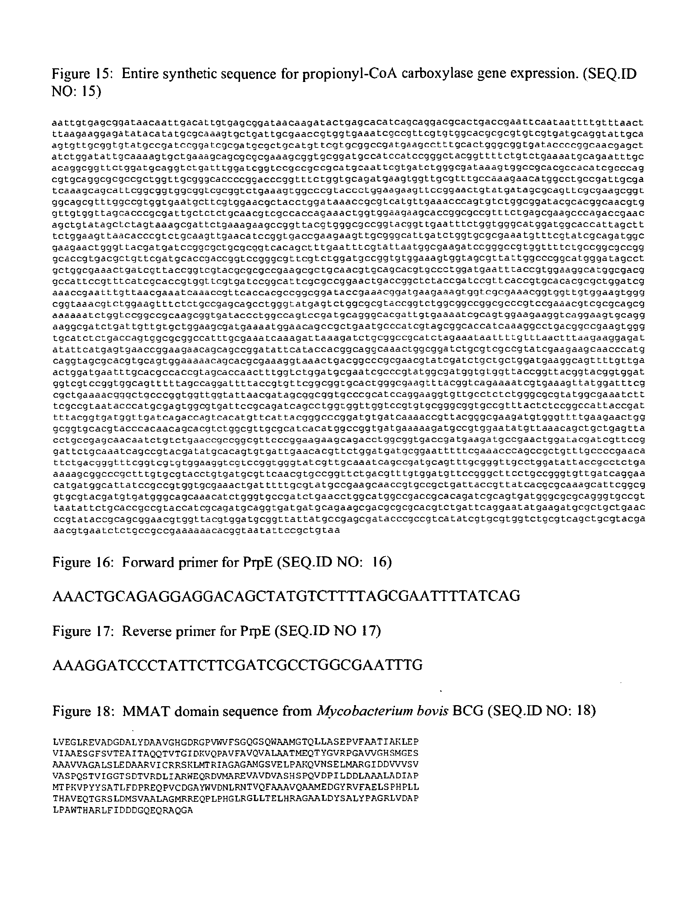

Figure 15 is a synthetic sequence for propionyl-CoA carboxylase gene expression (SEQ ID NO: 15).
Figure 16 is the forward primer sequence for PrpE (SEQ ID NO: 16).
Figure 17 is the reverse primer sequence for PrpE (SEQ ID NO: 17).
Figure 18 is the MMAT domain sequence from *Mycobacterium bovis* BCG (SEQ ID NO: 18).

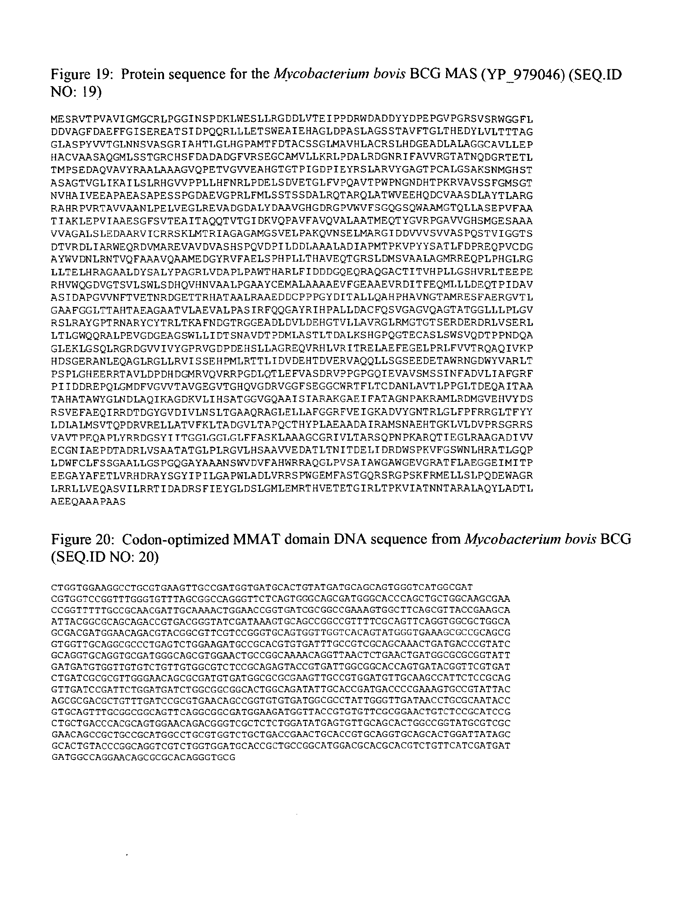

Figure 19 is a protein sequence for the *Mycobacterium bovis* BCG MAS (GenBank Accession No. YP_979046) (SEQ ID NO: 19).
Figure 20 is a codon-optimized MMAT domain DNA sequence from *Mycobacterium bovis* BCG (SEQ ID NO: 20).

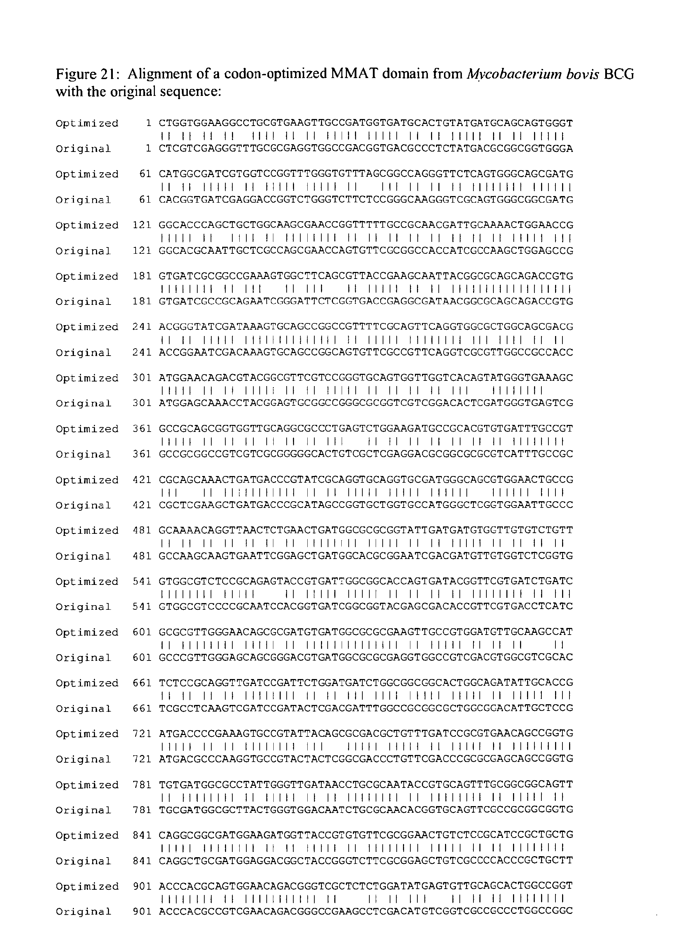

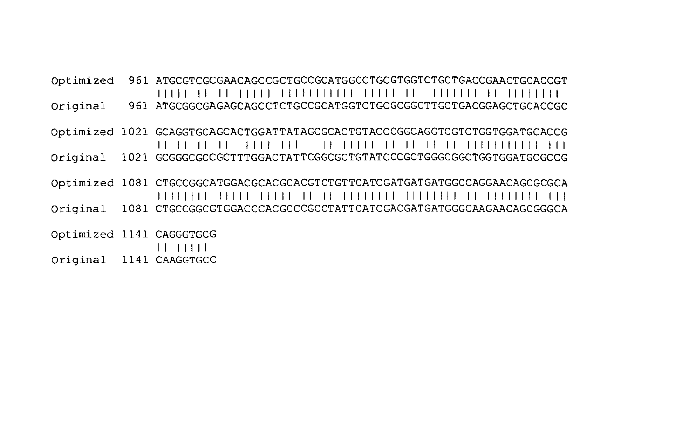

Figure 21 is an alignment of a codon-optimized MMAT domain from *Mycobacterium bovis* BCG with the original sequence (SEQ ID NOs: 20 and 21).

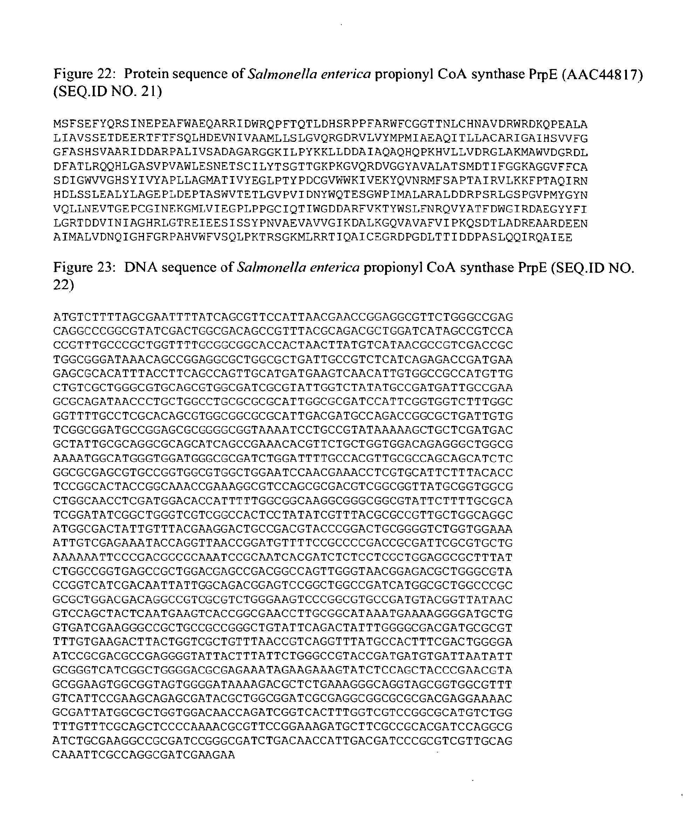

Figure 22 is the protein sequence of *Salmonella enterica* propionyl CoA synthase PrpE (GenBank Accession No. AAC44817) (SEQ ID NO: 22).
Figure 23 is the DNA sequence of *Salmonella enterica* propionyl CoA synthase PrpE (SEQ ID NO. 23).
Figure 24 is a bar graph illustrating methylmalonyl-CoA production (ng/ml) in *E. coli* strain K27-Z1 harboring pTrcHisA pZA31 (control), pZA31 *mutAB Ss epi* (MutAB Epi), pTrcHisA *Ec sbm* (Sbm), or pTrcHisA *Ec sbm* pZA31 *Mb mmat* (Sbm/Mmat). No methylmalonyl-CoA was identified in the control sample; the figure indicates the background level of detection.
Figure 25 is a bar graph illustrating methylmalonyl-CoA production (ng/ml) in *E. coli* BW25113 (control) and BW25113 harboring pZA31-*accA1-pccB* (Pcc). No methylmalonyl-CoA was identified in the control sample; the figure indicates the background level of detection. Two biological replicates are represented.
Figure 26 is a two-dimensional (2D) representation of the 2D Total Ion Chromatogram resulting from a sample of fatty acid produced by BL21 Star (DE3) *E. coli* harboring pTrcHisA *Ec sbm So ce epi* pZA31 *mmat.* Light areas on the figure indicate the presence of sample material. Peak names and arrows indicate samples that were further characterized by mass spectrometry.
Figure 27 is a two-dimensional (2D) representation of the 2D Total Ion Chromatogram resulting from a sample produced by a control strain, BL21 Star (DE3) *E. coli* harboring pTrcHisA pZA31. No branched-chain fatty acid was detected. Arrows indicate the presence of straight-chain fatty acid derivatives of the indicated chain length.
Figure 28 is a representation of the mass spectra of peaks 54, 55, and 57 identified in Figure 26. Eight- and ten-carbon branched-chain fatty acids are depicted in the top two profiles and were identified by the almost complete absence of the circled fragment. A twelve-branched fatty acid was tentatively identified and is depicted in the third profile.

### DETAILED DESCRIPTION OF THE INVENTION

The invention relates to improved biological production of scattered branched-chain fatty acids. In addition, in certain embodiments, the invention provides improved compositions of biologically produced scattered branched-chain fatty acids having defined chain lengths with methyl branches at one or more even-numbered carbons within the fatty acid. In addition, in certain embodiments, the fatty acid length can be tailored to a predetermined length, such as, for example, to produce fatty acids with a backbone of C12 to C16. In certain embodiments, the methods and/or cells can produce a mixture of fatty acids having varied numbers of methyl branches, varied positions of the methyl branches, and varied length of the fatty acids, such as, for example, a mixture of fatty acids having a chain length of C12 to C16 and from about 0 to about 3 methyl branches positioned on one or more even-numbered carbons.

As used herein, "amplify," "amplified," or "amplification" refers to any process or protocol for copying a polynucleotide sequence into a larger number of polynucleotide molecules, e.g., by reverse transcription, polymerase chain reaction, and ligase chain reaction.

As used herein, an "antisense sequence" refers to a sequence that specifically hybridizes with a second polynucleotide sequence. For instance, an antisense sequence is a DNA sequence that is inverted relative to its normal orientation for transcription. Antisense sequences can express an RNA transcript that is complementary to a target mRNA molecule expressed within the host cell (e.g., it can hybridize to target mRNA molecule through Watson-Crick base pairing).

As used herein, "cDNA" refers to a DNA that is complementary or identical to an mRNA, in either single stranded or double stranded form.

As used herein, the carbons in fatty acids are numbered with the first carbon as part of the carboxylic acid group, and the second carbon (C2) adjacent to the first. The numbers continue so that the highest number carbon is farthest from the carboxylic acid group. "Even number" carbons include C2, C4, C6, C8, C10, C12, C 14, and so on.

As used herein, "complementary" refers to a polynucleotide that can base pair with a second polynucleotide. Put another way, "complementary" describes the relationship between two single-stranded nucleic acid sequences that anneal by base-pairing. For example, a polynucleotide having the sequence 5'-GTCCGA-3' is complementary to a polynucleotide with the sequence 5'-TCGGAC-3'.

As used herein, a "conservative substitution" refers to the substitution in a polypeptide of an amino acid with a functionally similar amino acid. Put another way, a conservative substitution involves replacement of an amino acid residue with an amino acid residue having a similar side chain. Families of amino acid residues having similar side chains have been defined within the art, and include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, and cysteine), nonpolar side chains (e.g., alanine, valine, leucine, isoleucine, proline, phenylalanine, methionine, and tryptophan), beta-branched side chains (e.g., threonine, valine, and isoleucine) and aromatic side chains (e.g., tyrosine, phenylalanine, tryptophan, and histidine).

As used herein, "encoding" refers to the inherent property of nucleotides to serve as templates for synthesis of other polymers and macromolecules. Unless otherwise specified, a "nucleotide sequence encoding an amino acid sequence" includes all nucleotide sequences that are degenerate versions of each other and that encode the same amino acid sequence.

As used herein, "endogenous" refers to polynucleotides, polypeptides, or other compounds that are expressed naturally or originate within an organism or cell. That is, endogenous polynucleotides, polypeptides, or other compounds are not exogenous. For instance, an "endogenous" polynucleotide or peptide is present in the cell when the cell was originally isolated from nature.

As used herein, "expression vector" refers to a vector comprising a recombinant polynucleotide comprising expression control sequences operatively linked to a nucleotide sequence to be expressed. For example, suitable expression vectors include, without limitation, autonomously replicating vectors or vectors integrated into the chromosome. In some instances, an expression vector is a viral-based vector.

As used herein, "exogenous" refers to any polynucleotide or polypeptide that is not naturally expressed or produced in the particular cell or organism where expression is desired. Exogenous polynucleotides, polypeptides, or other compounds are not endogenous.

As used herein, "hybridization" includes any process by which a strand of a nucleic acid joins with a complementary nucleic acid strand through base-pairing. Thus, the term refers to the ability of the complement of the target sequence to bind to a test (i.e., target) sequence, or vice-versa.

As used herein, "hybridization conditions" are typically classified by degree of "stringency" of the conditions under which hybridization is measured. The degree of stringency can be based, for example, on the melting temperature (Tₘ) of the nucleic acid binding complex or probe. For example, "maximum stringency" typically occurs at about Tₘ -5° C (5° below the Tₘ of the probe); "high stringency" at about 5-10° C below the Tₘ; "intermediate stringency" at about 10-20° below the Tₘ of the probe; and "low stringency" at about 20-25° C below the Tₘ. Alternatively, or in addition, hybridization conditions can be based upon the salt or ionic strength conditions of hybridization and/or one or more stringency washes. For example, 6xSSC=very low stringency; 3xSSC=low to medium stringency; lxSSC=medium stringency; and 0.5xSSC=high stringency. Functionally, maximum stringency conditions may be used to identify nucleic acid sequences having strict (i.e., about 100%) identity or near-strict identity with the hybridization probe; while high stringency conditions are used to identify nucleic acid sequences having about 80% or more sequence identity with the probe.

As used herein, "identical" or percent "identity" in the context of two or more polynucleotide or polypeptide sequences refers to two or more sequences that are the same or have a specified percentage of nucleotides or amino acid residues that are the same, when compared and aligned for maximum correspondence, as measured using sequence comparison algorithms or by visual inspection.

As used herein, "long-chain fatty acids" refers to fatty acids with aliphatic tails longer than 14 carbons. In some embodiments of the invention, long-chain fatty acids are provided that comprise 15, 16, 17, 18, 19, 20, 21, or 22 carbons in the carbon backbone.

As used herein, "medium-chain fatty acids" refers to fatty acids with aliphatic tails between 6 and 14 carbons. In certain embodiments, the medium-chain fatty acids can have from 11 to 13 carbons.

As used herein, "naturally-occurring" refers to an object that can be found in nature. For example, a polypeptide or polynucleotide sequence that is present in an organism (including viruses) that can be isolated from a source in nature and which has not been intentionally modified by man in the laboratory is naturally-occurring.

As used herein, "operably linked," when describing the relationship between two DNA regions or two polypeptide regions, means that the regions are functionally related to each other. For example, a promoter is operably linked to a coding sequence if it controls the transcription of the sequence; a ribosome binding site is operably linked to a coding sequence if it is positioned so as to permit translation; and a signal sequence is operably linked to a peptide if it functions as a signal sequence, such as by participating in the secretion of the mature form of the protein.

As used herein, "overexpression" refers to expression of a polynucleotide to produce a product (e.g., a polypeptide or RNA) at a higher level than the polynucleotide is normally expressed in the host cell. An overexpressed polynucleotide is generally a polynucleotide native to the host cell, the product of which is generated in a greater amount than that normally found in the host cell. Overexpression is achieved by, for instance and without limitation, operably linking the polynucleotide to a different promoter than the polynucleotide's native promoter or introducing additional copies of the polynucleotide into the host cell.

As used herein, "polynucleotide" refers to a polymer composed of nucleotides. The polynucleotide may be in the form of a separate fragment or as a component of a larger nucleotide sequence construct, which has been derived from a nucleotide sequence isolated at least once in a quantity or concentration enabling identification, manipulation, and recovery of the sequence and its component nucleotide sequences by standard molecular biology methods, for example, using a cloning vector. When a nucleotide sequence is represented by a DNA sequence (i.e., A, T, G, C), this also includes an RNA sequence (i.e., A, U, G, C) in which "U" replaces "T." Put another way, "polynucleotide" refers to a polymer of nucleotides removed from other nucleotides (a separate fragment or entity) or can be a component or element of a larger nucleotide construct, such as an expression vector or a polycistronic sequence. Polynucleotides include DNA, RNA and cDNA sequences.

As used herein, "polypeptide" refers to a polymer composed of amino acid residues which may or may not contain modifications such as phosphates and formyl groups.

As used herein, "recombinant expression vector" refers to a DNA construct used to express a polynucleotide that encodes a desired polypeptide. A recombinant expression vector can include, for example, a transcriptional subunit comprising (i) an assembly of genetic elements having a regulatory role in gene expression, for example, promoters and enhancers, (ii) a structural or coding sequence which is transcribed into mRNA and translated into protein, and (iii) appropriate transcription and translation initiation and termination sequences. Recombinant expression vectors are constructed in any suitable manner. The nature of the vector is not critical, and any vector may be used, including plasmid, virus, bacteriophage, and transposon. Possible vectors for use in the invention include, but are not limited to, chromosomal, nonchromosomal and synthetic DNA sequences, e.g., bacterial plasmids; phage DNA; yeast plasmids; and vectors derived from combinations of plasmids and phage DNA, DNA from viruses such as vaccinia, adenovirus, fowl pox, baculovirus, SV40, and pseudorabies.

As used herein, "primer" refers to a polynucleotide that is capable of specifically hybridizing to a designated polynucleotide template and providing a point of initiation for synthesis of a complementary polynucleotide when the polynucleotide primer is placed under conditions in which synthesis is induced.

As used herein, "recombinant polynucleotide" refers to a polynucleotide having sequences that are not naturally joined together. A recombinant polynucleotide may be included in a suitable vector, and the vector can be used to transform a suitable host cell. A host cell that comprises the recombinant polynucleotide is referred to as a "recombinant host cell." The polynucleotide is then expressed in the recombinant host cell to produce, e.g., a "recombinant polypeptide."

As used herein, "specific hybridization" refers to the binding, duplexing, or hybridizing of a polynucleotide preferentially to a particular nucleotide sequence under stringent conditions.

As used herein, "stringent conditions" refers to conditions under which a probe will hybridize preferentially to its target subsequence, and to a lesser extent to, or not at all to, other sequences.

As used herein, "short-chain fatty acids" refers to fatty acids having aliphatic tails with fewer than 6 carbons.

As used herein, "substantially homologous" or "substantially identical" in the context of two nucleic acids or polypeptides, generally refers to two or more sequences or subsequences that have at least 40%, 60%, 80%, 90%, 95%, 96%, 97%, 98% or 99% nucleotide or amino acid residue identity, when compared and aligned for maximum correspondence, as measured using sequence comparison algorithms or by visual inspection. The substantial identity can exist over any suitable region of the sequences, such as, for example, a region that is at least about 50 residues in length, a region that is at least about 100 residues, or a region that is at least about 150 residues. In certain embodiments, the sequences are substantially identical over the entire length of either or both comparison biopolymers.

In one embodiment, the invention relates to a novel method of producing scattered branched-chain fatty acids (or products derived from scattered branched-chain fatty acid) using bacteria. In general, the method includes increasing the supply of methylmalonyl-CoA and/or the conversion of methylmalonyl-CoA to methylmalonyl-ACP within the cell, incorporating the branch from the methylmalonyl-CoA into the fatty acid, and, optionally, using a thioesterase to specify the range of size of the fatty acids. In certain embodiments, the method provides branched-chain fatty acids having a chain length of C12 to C16. In addition, in certain embodiments, the branched-chain fatty acids have from about 0 to about 3 methyl branches, such as from about 1 to about 3 methyl branches, such as, for example, from about 1 to about 2 methyl branches, or 1, 2, or 3 methyl branches positioned on one or more carbons. In certain embodiments, the methyl branches are positioned on even-numbered carbons.

In one embodiment, scattered branched-chain fatty acid production is increased by increasing the production of methylmalonyl-CoA within the cell via, e.g., propionyl-CoA and/or succinyl-CoA intermediates. Thus, in one aspect, the invention provides a method for producing branched-chain fatty acid comprising a methyl on one or more even number carbons. The method comprises culturing a cell comprising an exogenous or overexpressed polynucleotide comprising a nucleic acid sequence encoding a polypeptide that catalyzes the conversion of propionyl-CoA to methylmalonyl-CoA and/or an exogenous or overexpressed polynucleotide comprising a nucleic acid sequence encoding a polypeptide that catalyzes the conversion of succinyl-CoA to methylmalonyl-CoA. The cell is cultured under conditions allowing expression of the polynucleotide(s) and production of the branched-chain fatty acid. The cell produces more branched-chain fatty acid comprising a methyl branch on one or more even number carbons than an otherwise similar cell that does not comprise the polynucleotide(s) (e.g., a cell of the same cell type or derived from the same organism that does not comprise the polynucleotide(s)). Propionyl-CoA is converted to methylmalonyl-CoA by, e.g., the action of a propionyl-CoA carboxylase. Any propionyl-CoA carboxylase that catalyzes the conversion of propionyl-CoA to methylmalonyl-CoA is suitable for use in the inventive method. An exemplary propionyl-CoA carboxylase is a carboxylase from *Streptomyces coelicolor,* which comprises two heterologous subunits encoded by *pccB* and by either *accA1* or *accA2.* In certain embodiments, the cell of the inventive method is engineered to produce PccB and AccA1 or PccB and AccA2. In one aspect, the cell comprises one or more polynucleotides encoding polypeptide(s) comprising an amino acid sequence at least about 80% identical (e.g., 85%, 90%, 95%, or 100% identical) to the amino acid sequences set forth in SEQ ID NO: 9 and/or 10. Additional, non-limiting examples of polypeptides that catalyze the conversion of propionyl-CoA to methylmalonyl-CoA are propionyl-CoA carboxylases from *Mycobacterium smegmatis, Homo sapiens, Acinetobacter baumannii, Brucella suis, Saccharopolyspora erythraea, Burkholderia glumae,* and *Aedes aegypti,* as well as the propionyl-CoA carboxylases set forth in Table A.

**TABLE A**

| **Organism** | **GenBank Accession** | **Description** | **SEQ ID NO:** |
|---|---|---|---|
| *Ehrlichia chaffeensis* | YP_507303 | Propionyl-CoA carboxylase alpha subunit (PCCA) | 51 |
| *Ehrlichia chaffeensis* | YP_507410 | Propionyl-CoA carboxylase beta subunit (PCCB) | 52 |
| *Agrobacterium vitis* | YP_002547482 | Propionyl-CoA carboxylase alpha subunit (PCCA) | 53 |
| *Agrobacterium vitis* | YP_002547479 | Propionyl-CoA carboxylase beta subunit (PCCB) | 54 |
| *Methylobacterium extorquens* | YP_003069256 | Propionyl-CoA carboxylase alpha subunit (PCCA) | 55 |
| *Methylobacterium extorquens* | YP_003065890 | Propionyl-CoA carboxylase beta subunit (PCCB) | 56 |
| *Sinorhizobium meliloti* | NP_437988 | Propionyl-CoA carboxylase alpha subunit (PCCA) | 57 |
| *Sinorhizobium meliloti* | NP_437987 | Propionyl-CoA carboxylase beta subunit (PCCB) | 58 |
| *Ruegeria pomeroyi* | YP_166352 | Propionyl-CoA carboxylase alpha subunit (PCCA) | 59 |
| *Ruegeria pomeroyi* | YP_166345 | Propionyl-CoA carboxylase beta subunit (PCCB) | 60 |

Optionally, the cell is modified to increase carbon flow to propionyl-CoA (and then onward to methylmalonyl-CoA) by, for example, increasing expression of (i.e., overexpressing) *prpE* or other propionyl-CoA synthetase genes. Alternatively or in addition, an exogenous polynucleotide comprising a nucleic acid sequence encoding a propionyl-CoA synthetase is introduced into the host cell to upregulate propionyl-CoA production. Additionally, feeding host cells (e.g., microbes) large amounts of methionine, isoleucine, valine, threonine, propionic acid, and/or odd-chain length fatty acids (such as valeric acid) increases production of the propionyl-CoA precursor of methylmalonyl-CoA.

Methylmalonyl-CoA production via propionyl-CoA also is increased utilizing the metabolic pathway that converts pyruvate to propionyl-CoA, with lactate, lactoyl-CoA, and acrylyl-CoA as intermediates. Carbon flow to propionyl-CoA is upregulated by overproducing the enzymes of the pathway, producing exogenous enzymes catalyzing one or more conversions of the pathway, and/or by providing pyruvate or lactate in larger amounts than normally found in the host cell. For example, in any embodiment of the invention, the cell comprises an exogenous or overexpressed polynucleotide encoding lactate dehydrogenase, lactate CoA transferase, lactyl-CoA dehydratase, and/or acrylyl-CoA reductase.

In addition, in any aspect of the invention, carbon flow to branch pathways not contributing to formation of the desired branched-chain fatty acid is minimized by attenuation of endogenous enzyme activity responsible for the diversion of carbon. Complete abolishment of endogenous activity is not required; any reduction in activity is suitable in the context of the invention. Enzyme activity is attenuated (i.e., reduced or abolished) by, for example, mutating the coding sequence for the enzyme to create a non-functional or reduced-function polypeptide, by removing all or part of the coding sequence for the enzyme from the cellular genome, by interfering with translation of an RNA transcript encoding the enzyme (e.g., using antisense oligonucleotides), or by manipulating the expression control sequences influencing expression of the enzyme. For example, in one aspect, the cell is modified to prevent methylmalonyl-CoA degradation, thereby increasing the amount of methylmalonyl-CoA available for conversion to methylmalonyl-ACP. Methylmalonyl-CoA degradation is reduced by, for example, deleting or inactivating methylmalonyl-CoA decarboxylase from the host. Put another way, the cell is modified to attenuate endogenous methylmalonyl-CoA decarboxylase activity. In *E. coli,* for example, methylmalonyl-CoA decarboxylase activity is attenuated by, for example, deleting or mutating *ygfG.* Optionally, endogenous acyl transferase activity is attenuated. Alternatively or in addition, methylmalonyl-CoA production within the cell is increased by preventing alternative metabolism of propionyl-CoA to succinyl-CoA, such as, for example, by deleting or otherwise reducing (attenuating) the activity of an endogenous methylmalonyl-CoA mutase gene. Optionally, methylmalonyl-CoA levels are increased by increasing the degradation of valine directly to methylmalonyl-CoA. Valine degradation comprises the following intermediates: α-ketoisovalerate, isobutyryl-CoA, methacrylyl-CoA, β-hydroxyisobutyryl-CoA, β-hydroxyisobutyrate, and methylmalonate semialdehyde. Optionally, methylmalonate semialdehyde is converted directly to methylmalonyl-CoA or indirectly through a propionyl-CoA intermediate. In an exemplary embodiment, the cell of the invention comprises an overexpressed or exogenous polynucleotide comprising a nucleic acid sequence encoding one or more of the following enzymes: L-valine:2-oxoglutarate aminotransferase, 2-oxoisovalerate dehydrogenase, isobutyryl-CoA:FAD oxidoreductase, 3-hydroxy-isobutyryl-CoA hydro-lyase, 3-hydroxyisobutyryl-CoA hydrolase, 3-hydroxyisobutyrate dehydrogenase, and/or methylmalonate-semialdehyde dehydrogenase. Methylmalonate-semialdehyde dehydrogenase catalyzes the production of propanoyl-CoA, which can be converted to methylmalonyl-CoA by propanoyl-CoA carboxylase.

In one aspect, the cell comprises an exogenous or overexpressed polynucleotide comprising a nucleic acid sequence encoding a polypeptide that catalyzes the conversion of succinyl-CoA to methylmalonyl-CoA. An exemplary polypeptide that catalyzes the reaction is methylmalonyl-CoA mutase. In any embodiment of the invention, the cell is engineered to overexpress a methylmalonyl-CoA mutase gene, such as, for example, *sbm* (encoding Sleeping Beauty mutase) in *E. coli.* Alternatively or in addition, an exogenous polynucleotide comprising a nucleic acid sequence encoding a methylmalonyl-CoA mutase is expressed in the cell. Exemplary methylmalonyl-CoA mutases include, but are not limited to, Sbm from *E. coli,* MutA and/or MutB from *Streptomyces cinnamonensis,* and methylmalonyl-CoA mutases from *Janibacter* sp. HTCC2649, *Corynebacterium glutamicum, Euglena gracilis, Homo sapiens, Propionibacterium shermanii, Bacillus megaterium,* and *Mycobacterium smegmatis*. Additional, non-limiting examples of polypeptides that catalyze the conversion of succinyl-CoA to methylmalonyl-CoA are provided in Table B.

**TABLE B**

| **Organism** | **GenBank Accession** | **Description** | **SEQ ID NO.** |
|---|---|---|---|
| *Bacillus megaterium* | YP_003564880 | methylmalonyl-CoA mutase small subunit (mutA) | 61 |
| *Bacillus megaterium* | YP_003564879 | methylmalonyl-CoA mutase large subunit (mutB) | 62 |
| *Mycobacterium tuberculosis* | YP_001282809 | methylmalonyl-CoA mutase small subunit (mutA) | 63 |
| *Mycobacterium tuberculosis* | YP_001282810 | methylmalonyl-CoA mutase large subunit (mutB) | 64 |
| *Corynebacterium glutamicum* | YP_225814 | methylmalonyl-COA mutase small subunit (mutA) | 65 |
| *Corynebacterium glutamicum* | YP_225813 | methylmalonyl-CoA mutase large subunit (mutB) | 66 |
| *Rhodococcus erythropolis* | YP_002766535 | methylmalonyl-CoA mutase small subunit (mutA) | 67 |
| *Rhodococcus erythropolis* | YP_002766536 | methylmalonyl-CoA mutase large subunit (mutB) | 68 |
| *Porphyromonas gingivalis* | NP_905776 | methylmalonyl-CoA mutase small subunit (mutA) | 69 |
| *Porphyromonas gingivalis* | NP_905777 | methylmalonyl-CoA mutase large subunit (mutB) | 70 |

In one aspect, the cell comprises one or more polynucleotides encoding polypeptide(s) comprising an amino acid sequence at least about 80% identical (e.g., 85%, 90%, 95%, or 100% identical) to the amino acid sequences set forth in SEQ ID NO: 3, 4, and/or 28. The cell can comprise polynucleotides encoding a methylmalonyl-CoA mutase, a propionyl-CoA carboxylase, or both.

Depending on the substrate specificity of the fatty acid synthase produced by the cell, a methylmalonyl-CoA epimerase also may be desired to facilitate use of methylmalonyl-CoA as a precursor in fatty acid synthesis. Thus, in one aspect, the cell further comprises an exogenous or overexpressed polynucleotide comprising a nucleic acid sequence encoding a methylmalonyl-CoA epimerase. Methylmalonyl-CoA epimerases suitable for use in the invention include, but are not limited to, *Sorangium cellulosum* So ce 56 methylmalonyl-CoA epimerase, *Streptomyces sviceus* ATCC 29083 methylmalonyl-CoA epimerase, *Kribbella flavida* DSM 17836 methylmalonyl-CoA epimerase, and methylmalonyl-CoA epimerases from *Homo sapiens, Bacillus megaterium,* and *Mycobacterium smegmatis.*

Production of branched-chain fatty acid comprising a methyl branch on one or more even number carbons also is enhanced by upregulating conversion of methylmalonyl-CoA to methylmalonyl-ACP. In one or more embodiments, conversion of methylmalonyl-CoA to methylmalonyl-ACP is increased in the cell by engineering the cell to produce an acyl transferase (such as the acyl transferase encoded by *fabD* in *E. coli)* to catalyze the formation of methylmalonyl-ACP from methylmalonyl-CoA. Put another way, in one aspect, the cell further comprises an exogenous or overexpressed polynucleotide comprising a nucleic acid sequence encoding an acyl transferase. Any suitable acyl transferase can be used, such as, for example and without limitation, an acyl transferase domain from a polyketide synthase, such as those involved in the synthesis of monensin, epothilone, amphotericin, candicidin, nystatin, pimaricin, ascomycin, rapamycin, avermiectin, spinosad, mycinamicin, niddamycin, oleandomycin, megalomicin, nanchangmycin, picromycin, rifamycin, oligomycin erythromycin, polyenes, and macrolides, and an acyl transferase domain from *Mycobacterium* mycocerosic acid synthase. Acyl transferase domains from larger fatty acid synthase enzymes, such as *Mycobacterium* mycocerosic acid synthase, act upon methylmalonyl-CoA in the absence of other enzymatic domains of the larger synthase. Optionally, the acyl transferase lacks polyketide synthesis activity. By "polyketide synthesis activity" is meant enzymatic activity, other than acyl transferase activity, that catalyzes the production of polyketides in a host cell, such as, for example and without limitation, acyltransferase activity, ketoacyl synthase activity, ketoacyl reductase activity, dehydratase activity, enoyl reductase activity, acyl carrier protein activity, and thioesterase activity.

Alternatively, or in addition, in certain embodiments, a 3-ketoacyl-ACP synthase domain, such as, for example, a domain from a polyketide synthase or a mycocerosic acid synthase, is added to the fatty acid synthase of the host cell. In certain embodiments, the host cell (e.g., microbe) is engineered to include both acyl transferase and 3-ketoacyl-ACP synthase domains that can recognize methylmalonyl-CoA. In addition, in certain embodiments, genes for the endogenous acyl transferase and/or 3-ketoacyl-ACP synthase activities can be attenuated (e.g., deleted) to minimize the amount of malonyl-CoA incorporation in fatty acid synthesis.

In certain embodiments, the invention includes use of a thioesterase to specify the chain length of the fatty acid, such as, for example, to produce medium-chain fatty acids. In certain embodiments, the host cell further comprises an exogenous or overexpressed polynucleotide comprising a nucleic acid sequence encoding a thioesterase. In one aspect, the host cell (e.g., bacteria) is engineered to produce a thioesterase that assists in the production of medium-chain branched-chain fatty acids. Alternatively, the host cell is engineered to produce (or overproduce) a thioesterase that assists in the production of long-chain branched-chain fatty acids. Exemplary thioesterases include, for example, the mallard uropygial gland thioesterase, the California bay thioesterase, the rat mammary gland thioesterase II, *E. coli* TesA, the *Cuphea wrightii* thioesterase, and other thioesterases suitable for production of the desired chain-length fatty acids.

Optionally, the cell is modified to produce (or increase the production of) branched acyl-CoA, which is a substrate for elongase in the production of long chain fatty acid. In this regard, in an exemplary embodiment of the invention, the cell comprises an exogenous or overexpressed polynucleotide comprising a nucleic acid encoding a coenzyme-A synthetase, which converts branched-chain fatty acid to branched acyl-CoA. Examples of coenzyme-A synthetases include, but are not limited to, the coenzyme-A synthetase from *Leishmania braziliensis* (GenBank Accession No. XP_001561614), and the coenzyme-A synthetase from *Escherichia coli* (GenBank Accession No. YP_541006). Optionally, the cell comprises exogenous or overexpressed polynucleotide(s) comprising a nucleic acid sequence encoding an elongase to increase the length of the carbon backbone. Elongases are enzyme complexes that exhibit 3-ketoacyl-CoA synthase, 3-ketoacyl-CoA reductase, 3-hydroxyacyl-CoA dehydratase, and enoyl-CoA reductase activities, and generally utilize malonyl-CoA as an extension unit for extending the carbon chain. When a methyl-malonyl CoA is used as an extension unit by the enzyme complex, additional methyl branches are introduced at even carbon positions. Exemplary elongases include, but are not limited to, elongases comprising the one or more of the following subunits: *Saccharomyces cerevisiae* 3-ketoacyl-CoA synthase (GenBank Accession No. NP_013476), 3-ketoacyl-CoA reductase (GenBank Accession No. NP_009717), 3-hydroxyacyl-CoA dehydratase (GenBank Accession No. NP_012438) and enoyl-CoA reductase (GenBank Accession No. NP_010269); and *Arabidopsis thaliana col* 3-ketoacyl-CoA synthase (GenBank Accession No. NP_849861), 3-ketoacyl-CoA reductase (GenBank Accession No. NP_564905), 3-hydroxyacyl-CoA dehydratase (GenBank Accession No. NP_193180), and enoyl-CoA reductase (GenBank Accession No. NP_191096).

Tthe invention relates to *Escherichia* cells (e.g., *E. coli),* which naturally produce Type II fatty acid synthase and/or do not naturally produce scattered branched-chain fatty acid (i.e., branched-chain fatty acid comprising a methyl branch on one or more even numbered carbons). These cells are engineered to produce the branched-chain fatty acids as described herein. Alternatively, the cell naturally produces branched-chain fatty acid and is modified as described herein to produce higher levels of branched-chain fatty acid (or different proportions of different types of branched-chain fatty acid) compared to an unmodified cell. In certain embodiments, fatty acid is manufactured using bacteria known to make the methylmalonyl-CoA precursor, such as *Streptomyces*, *Mycobacterium* or *Corynebacterium.* These bacteria are, in one aspect, engineered to produce (i) an acyl transferase to increase carbon flux to methylmalonyl-ACP that is incorporated in the fatty acid synthesis pathway and/or (ii) a thioesterase to control the chain length.

In one aspect, the fatty acid produced by the inventive cell comprises about 80% to about 100% (wt.) (e.g., about 85%, about 90%, or about 95%) linear and branched-chain fatty acid. Of the linear and branched-chain fatty acids produced by the cell, approximately 1% to approximately 95% or more (e.g., 5%, 10%, 15%, 20%, 30%, 50%, 60%, 75%, 85%, or 100%) is branched-chain fatty acid comprising a methyl group on one or more even carbons. In some embodiments, the cell does not produce, or produces only trace amounts of, fatty acid comprising methyl branching on odd numbered carbons. By "trace amount" is meant less than 1% of the total fatty acid content produced by the cell. Alternatively or in addition, in one aspect, the mixture of fatty acids produced by the cell comprises no more than 50% end-terminal-branched fatty acid (i.e., fatty acids that contain branching on a carbon atom that is within 40% of the non-functionalized terminus of the longest carbon chain). Optionally, the inventive cell is modified to preferentially produce branched-chain fatty acid with desired chain lengths, e.g., about six to about 18 carbons or more in the carbon backbone (not including the methyl branch(es)). In some embodiments, the host cell preferentially generates long chain fatty acid, medium-length chain fatty acid, short chain fatty acid, or a desired combination fatty acids (e.g., 60%, 70%, 80%, 85%, 90%, 95% or more of the branched-chain fatty acid produced by the cell comprises the desired number of carbons). In addition, in certain embodiments, the engineered cells tolerate large amounts of branched-chain fatty acid in the growth medium, plasma membrane, or lipid droplets, and/or produce branched-chain fatty acid more economically than an unmodified cell by, e.g., using a less expensive feedstock, requiring less fermentation time, and the like.

The polynucleotide(s) encoding one or more polypeptides that catalyze the reaction(s) for producing branched-chain fatty acid may be derived from any source. Depending on the embodiment of the invention, the polynucleotide is isolated from a natural source such as bacteria, algae, fungi, plants, or animals; produced via a semi-synthetic route (e.g., the nucleic acid sequence of a polynucleotide is codon-optimized for expression in a particular host cell, such as *E. coli);* or synthesized *de novo.* In certain embodiments, it is advantageous to select an enzyme from a particular source based on, e.g., the substrate specificity of the enzyme, the type of branched-chain fatty acid produced by the source, or the level of enzyme activity in a given host cell. In one aspect of the invention, the enzyme and corresponding polynucleotide are naturally found in the host cell and overexpression of the polynucleotide is desired. In this regard, in some instances, additional copies of the polynucleotide are introduced in the host cell to increase the amount of enzyme available for fatty acid production. Overexpression of a native polynucleotide also is achieved by upregulating endogenous promoter activity, or operably linking the polynucleotide to a more robust promoter. Exogenous enzymes and their corresponding polynucleotides also are suitable for use in the context of the invention, and the features of the biosynthesis pathway or end product can be tailored depending on the particular enzyme used. If desired, the polynucleotide(s) is isolated or derived from the branched-chain fatty acid-producing organisms described herein.

In certain embodiments, the cell produces an analog or variant of a polypeptide described herein. Amino acid sequence variants of the polypeptide include substitution, insertion, or deletion variants, and variants may be substantially homologous or substantially identical to the unmodified polypeptides as set out above. In certain embodiments, the variants retain at least some of the biological activity, e.g., catalytic activity, of the polypeptide. Other variants include variants of the polypeptide that retain at least about 50%, preferably at least about 75%, more preferably at least about 90%, of the biological activity.

Substitution variants typically exchange one amino acid for another at one or more sites within the protein. Substitutions of this kind can be conservative, that is, one amino acid is replaced with one of similar shape and charge. Conservative substitutions include, for example, the changes of: alanine to serine; arginine to lysine; asparagine to glutamine; aspartate to glutamate; cysteine to serine; glutamine to asparagine; glutamate to aspartate; isoleucine to leucine or valine; leucine to valine or isoleucine; lysine to arginine; methionine to leucine or isoleucine; phenylalanine to tyrosine, leucine or methionine; serine to threonine; threonine to serine; tryptophan to tyrosine; tyrosine to tryptophan or phenylalanine; and valine to isoleucine or leucine.

In some instances, the recombinant cell comprises an analog or variant of the exogenous or overexpressed polynucleotide(s) described herein. Nucleic acid sequence variants include one or more substitutions, insertions, or deletions, and variants may be substantially homologous or substantially identical to the unmodified polynucleotide. Polynucleotide variants or analogs encode mutant enzymes having at least partial activity of the unmodified enzyme. Alternatively, polynucleotide variants or analogs encode the same amino acid sequence as the unmodified polynucleotide. Codon-optimized sequences, for example, generally encode the same amino acid sequence as the parent/native sequence but contain codons that are preferentially expressed in a particular host organism.

A polypeptide or polynucleotide "derived from" an organism contains one or more modifications to the native amino acid sequence or nucleotide sequence and exhibits similar, if not better, activity compared to the native enzyme (e.g., at least 70%, at least 80%, at least 90%, at least 95%, at least 100%, or at least 110% the level of activity of the native enzyme). For example, enzyme activity is improved in some contexts by directed evolution of a parent/native sequence. Additionally or alternatively, an enzyme coding sequence is mutated to achieve feedback resistance. Thus, in one or more embodiments of the invention, the polypeptide encoded by the exogenous polynucleotide is feedback resistant and/or is modified to alter the activity of the native enzyme. A polynucleotide "derived from" a reference polynucleotide encompasses, but is not limited to, a polynucleotide comprising a nucleic acid sequence that has been codon-optimized for expression in a desired host cell.

The cell of the invention may comprise any combination of polynucleotides described herein to produce branched-chain fatty acid comprising a methyl branch on one or more even number carbons. For example, the invention provides a cell comprising (i) an exogenous or overexpressed polynucleotide comprising a nucleic acid sequence encoding an acyl transferase lacking polyketide synthesis activity, and (ii) an exogenous or overexpressed polynucleotide comprising a nucleic acid sequence encoding a propionyl-CoA carboxylase and/or an exogenous or overexpressed polynucleotide comprising a nucleic acid sequence encoding a methylmalonyl-CoA mutase, wherein the polynucleotide(s) are expressed and the cell produces more branched-chain fatty acid comprising a methyl on one or more even number carbons than an otherwise similar cell that does not comprise the polynucleotide(s). Recombinant cells can be produced in any suitable manner to establish an expression vector within the cell. The expression vector can include the exogenous polynucleotide operably linked to expression elements, such as, for example, promoters, enhancers, ribosome binding sites, operators and activating sequences. Such expression elements may be regulatable, for example, inducible (via the addition of an inducer). Alternatively or in addition, the expression vector can include additional copies of a polynucleotide encoding a native gene product operably linked to expression elements. Representative examples of useful promoters include, but are not limited to: the LTR (long terminal 35 repeat from a retrovirus) or SV40 promoter, the *E. coli lac, tet,* or *trp* promoter, the phage Lambda P_{L} promoter, and other promoters known to control expression of genes in prokaryotic or eukaryotic cells or their viruses. In one aspect, the expression vector also includes appropriate sequences for amplifying expression. The expression vector can comprise elements to facilitate incorporation of polynucleotides into the cellular genome. Introduction of the expression vector or other polynucleotides into cells can be performed using any suitable method, such as, for example, transformation, electroporation, microinjection, microprojectile bombardment, calcium phosphate precipitation, modified calcium phosphate precipitation, cationic lipid treatment, photoporation, fusion methodologies, receptor mediated transfer, or polybrene precipitation. Alternatively, the expression vector or other polynucleotides can be introduced by infection with a viral vector, by conjugation, by transduction, or by other any other suitable method.

Cells, such as bacterial cells, containing the polynucleotides encoding the proteins described herein can be cultured under conditions appropriate for growth of the cells and expression of the polynucleotides. Cells expressing the protein can be identified by any suitable methods, such as, for example, by PCR screening, screening by Southern blot analysis, or screening for the expression of the protein. In certain embodiments, cells that contain the polynucleotide(s) can be selected by including a selectable marker in the DNA construct, with subsequent culturing of cells containing a selectable marker gene, under conditions appropriate for survival of only those cells that express the selectable marker gene. The introduced DNA construct can be further amplified by culturing genetically modified cells under appropriate conditions (e.g., culturing genetically modified cells containing an amplifiable marker gene in the presence of a concentration of a drug at which only cells containing multiple copies of the amplifiable marker gene can survive). Cells that contain and express polynucleotides encoding the exogenous proteins can be referred to herein as genetically modified cells. Bacterial cells that contain and express polynucleotides encoding the exogenous protein can be referred to as genetically modified bacterial cells.

Exemplary cells of the invention include *E. coli* BW25113 comprising pTrcHisA *mmat* and pZA31*-accA1-pccB,* which was deposited with American Type Culture Collection (ATCC), 10801 University Blvd., Manassas, VA, on December 14, 2010, under the provisions of the Budapest Treaty for the International Recognition of the Deposit of Microorganisms for the Purpose of Patent Procedure ("Budapest Treaty"), and assigned Deposit Accession No. [XXX] on [DATE], and *E. coli* BL21 Star (DE3) comprising pTrcHisA *Ec sbm So ce epi* and pZA31 *mmat* which was deposited with American Type Culture Collection (ATCC), 10801 University Blvd., Manassas, VA, on December 14, 2010, under the provisions of the Budapest Treaty for the International Recognition of the Deposit of Microorganisms for the Purpose of Patent Procedure ("Budapest Treaty"), and assigned Deposit Accession No. [XXX] on [DATE]. The invention also includes variants or progeny of the cells described herein that retain the phenotypic characteristics of the recombinant microbe. A substantially pure monoculture of the cell described herein (i.e., a culture comprising at least 80% or at least 90% of a desired cell) also is provided.

Any cell culture conditions appropriate for growing a host cell and synthesizing branched-chain fatty acid is suitable for use in the inventive method. Addition of fatty acid synthesis intermediates, precursors, and/or co-factors for the enzymes associated with branched-chain fatty acid synthesis to the culture is contemplated herein. In certain embodiments, the genetically modified cells (such as genetically modified bacterial cells) have an optimal temperature for growth, such as, for example, a lower temperature than normally encountered for growth and/or fermentation. For example, in certain embodiments, incorporation of branched-chain fatty acids into the membrane may increase membrane fluidity, a property normally associated with low growth temperatures. In addition, in certain embodiments, cells of the invention may exhibit a decline in growth at higher temperatures as compared to normal growth and/or fermentation temperatures as typically found in cells of the type.

The inventive method optionally comprises extracting branched-chain fatty acid from the culture. Fatty acids can be extracted from the culture medium and measured using any suitable manner. Suitable extraction methods include, for example, methods as described in: Bligh et al., A rapid method for total lipid extraction and purification, Can. J. Biochem. Physiol. 37:911-917 (1959). In certain embodiments, production of fatty acids in the culture supernatant or in the membrane fraction of recombinant cells can be measured. In this embodiment, cultures are prepared in the standard manner, although nutrients (e.g., 2-methylbutyrate, isoleucine) that may provide a boost in substrate supply can be added to the culture. Cells are harvested by centrifugation, acidified with hydrochloric or perchloric acid, and extracted with chloroform and methanol, with the fatty acids entering the organic layer. The fatty acids are converted to methyl esters, using methanol at 100° C. The methyl esters are separated by gas chromatography (GC) and compared with known standards of fatty acids (purchased from Larodan or Sigma). Confirmation of chemical identity is carried out by combined GC/mass spec, with further mass spec analysis of fragmented material carried out if necessary.

In one embodiment, the cell utilizes the branched-chain fatty acid as a precursor to make one or more other products. Products biosynthesized (i.e., derived) from branched-chain fatty acid include, but are not limited to, phospholipids, triglycerides, alkanes, olefins, wax esters, fatty alcohols, and fatty aldehydes. Some host cells naturally generate one or more products derived from branched-chain fatty acid; other host cells are genetically engineered to convert branched-chain fatty acid to, e.g., an alkane, olefin, wax ester, fatty alcohol, phospholipid, triglyceride, and/or fatty aldehyde. Organisms and genetic modifications thereof to synthesize products derived from branched-chain fatty acids are further described in, e.g., International Patent Publication Nos. WO 2007/136762, WO 2008/151149, and WO 2010/062480, and U.S. Patent Application Publication US 2010/0298612. In one aspect, the inventive method comprises extracting a product derived from branched-chain fatty acid (phospholipid, triglyceride, alkane, olefin, wax ester, fatty alcohol, and/or fatty aldehyde synthesized in the cell from branched-chain fatty acid) from the culture. Any extraction method is appropriate, including the extraction methods described in International Patent Publication Nos. WO 2007/136762, WO 2008/151149, and WO 2010/062480, and U.S. Patent Application Publication Nos. US 2010/0251601, US 20100242345, US 20100105963, and US 2010/0298612.

The inventive cell preferably produces more branched-chain fatty acid comprising a methyl branch on one or more even number carbons than an otherwise similar cell that does not comprise the polynucleotide(s). Methods of measuring fatty acid released into the fermentation broth or culture media or liberated from cellular fractions are described herein. Branched-chain fatty acid production is not limited to fatty acid accumulated in the culture, however, but also includes fatty acid used as a precursor for downstream reactions yielding products derived from branched-chain fatty acid. Thus, products derived from branched-chain fatty acid (e.g., phospholipids, triglycerides, fatty alcohols, olefins, wax esters, fatty aldehydes, and alkanes) are, in some embodiments, surrogates for measuring branched-chain fatty acid production in a host cell. Methods of measuring fatty acid content in phospholipid in the cell membrane are described herein. Similarly, measurement of degradation products of branched-chain fatty acids also is instructive as to the amount of branched-chain fatty acid is produced in a host cell. Depending on the particular embodiment of the invention, the inventive cell produces at least 3%, at least 5%, at least 10%, at least 20%, at least 25%, or at least 50% more branched-chain fatty acid than an otherwise similar cell that does not comprise the polynucleotide(s).

The invention further provides a composition comprising the branched-chain fatty acids described herein. For example, the invention provides a composition comprising a branched-chain fatty acid comprising between 10-18 carbons in the carbon backbone, such as fatty acids comprising between 10 and 16 carbons (e.g., fatty acids comprising 10, 11, 12, 13, 14, 15, or 16 carbons), with branching on one or more even numbered carbons (e.g., C2, C4, C6, C8, C10, C12, C 14, and/or C16). A composition comprising longer-chain fatty acid also is provided, such as a composition comprising between 19 and 22 carbons in the longest carbon chain. A composition comprising a combination of any of the fatty acids described herein also is provided (e.g., a composition comprising fatty acids of varying lengths and/or branch locations along the carbon backbone).

The following examples further describe and demonstrate embodiments within the scope of the invention. The examples are given solely for the purpose of illustration and are not to be construed as limitations of the invention, as many variations thereof are possible without departing from the scope of the invention.

### Example 1. Construction of methylmalonyl-CoA mutase expression vector.

There are numerous genes annotated to encode the two subunits of methylmalonyl-CoA mutase. *Janibacter* sp. HTCC2649 encodes two such genes. Synthetic versions of these genes were prepared, with the codon usage altered to match that used by many *E. coli* genes (i.e., the coding sequence was codon-optimized for expression in *E. coli).* By analogy to other methylmalonyl-CoA mutase genes, these synthetic genes were named *mutA* (SEQ ID NO: 1) and *mutB* (SEQ ID NO: 2), corresponding to the MutA (SEQ ID NO: 3) and MutB (SEQ ID NO: 4) protein subunits. In the synthetic DNA, an extra three base pairs were added (encoding an alanine residue immediately after the initiation methionine) in *mutA* to facilitate introduction of an *Nco*I site. An *Xho*I restriction site was also placed after the coding sequence of *mutB* for insertion into the pBAD vector (Invitrogen). The *Nco*I/*Xho*I fragment was cloned into pBAD.

### Example 2. Construction of methylmalonyl-CoA epimerase expression vector.

There are numerous genes annotated to encode methylmalonyl-CoA mutase. One such gene is from *Streptomyces sviceus.* A synthetic gene can be constructed (SEQ ID NO: 5) using codon usage similar to *E. coli* genes and with *Eco*RI and *Hind* III sites flanking the coding region. An *E. coli* Shine-Dalgarno sequence can be added between the *Eco*RI site and the initiation codon for the epimerase gene. The predicted protein product is the same as the predicted protein product from the *S. sviceus* gene (SEQ ID NO: 6). The epimerase gene can be cloned into the pBAD-*mutAB* construct using the *Eco*RI and *Hin*d III restriction sites (downstream of *mutB*) to form the pBAD-*mutAB*-epimerase gene plasmid. *E. coli* cultures can be grown at 27° C after induction with arabinose and supplemented with hydroxycobalamin to achieve expression of functional methylmalonyl-CoA mutase and branched-chain fatty acid production.

### Example 3. Construction of propionyl-CoA carboxylase expression vector.

Nucleotide sequences (SEQ ID NO: 7 and SEQ ID NO: 8) encoding the two propionyl-CoA carboxylase subunits AccA1 (GenBank Accession NO. AF113603.1; SEQ ID NO: 9) and PccB (GenBank Accession No. AF113605.1; SEQ ID NO: 10)), respectively, from the *Streptomyces coelicolor A3(2)* propionyl-CoA carboxylase (Rodriguez E., Gramajo H., Microbiology. 1999 Nov;145:3109-19), were codon-optimized for *E. coli* expression. A gene construct for expressing propionyl-CoA carboxylase was constructed with the following elements sequentially 1) P_{L1ac0-1} promoter and operator plus T7 gene10 ribosomal binding site (SEQ ID NO: 11); 2) optimized *accA1* (SEQ ID NO: 12); 3) three restriction site sequences including *Bgl*II, *Not*I and *Xba*I and a T7 gene10 ribosome binding site (SEQ ID NO: 13); and 4) codon-optimized *pccB* (SEQ ID NO: 14). The synthesized DNA fragments were cloned into the *Xho*I and *Pst*I sites of expression vector pZA31-MCS (Expressys, Ruelzheim, Germany), resulting in plasmid pZA31*-accA1-pccB* (SEQ ID NO: 15).

### Example 4. Construction of propionyl-CoA synthetase expression vector.

The *Salmonella enterica* propionyl-CoA synthetase gene, *prpE,* was amplified using PCR and the primers set forth in SEQ ID NO: 16 and SEQ ID NO: 17, and placed behind a Shine-Dalgarno sequence in the plasmid pZA31*-accA1-pccB* (SEQ ID NO: 15) using the restriction enzymes *Pst*I and *Bam*HI. Enhanced propionyl-CoA synthetase production is expected to increase synthetic flux to propionyl-CoA.

### Example 5. Reduction of propionylation of propionyl-CoA synthetase.

In *S. enterica,* propionyl-CoA synthetase is subject to inhibition by propionylation at lysine 592 when propionyl-CoA levels accumulate. (Garrity et al, J. Biol. Chem., Vol. 282, Issue 41, 30239-30245, October 12, 2007). Similar enzyme modulation may occur in other species, although the position of the modified lysine may be different. Several strategies to overcome this inhibition will be tested and compared. First, the propionyl-CoA synthetase gene will be mutated to change the coding capacity from lysine (at the site of propionylation) to arginine or other amino acids to prevent propionylation. Second, a source of resveratrol or other sirtuin activators will be introduced into the culture medium to activate sirtuin to depropionylate PrpE. Third, the endogenous *N*-acetyltransferase enzyme responsible for the propionylation reaction will be knocked out. For example, if working with *S*. *enterica, pat* could be deleted. As another example, if working with *B. subtilis, acuA* could be deleted. Fourth, the flux of propionyl-CoA into fatty acid synthesis will be increased by increasing propionyl-CoA carboxylase activity to keep free propionyl-CoA levels down. Fifth, the sirtuin activity will be increased, thus increasing deacetylation of propionyl-CoA carboxylase. For example, the *S. enterica cobB* expression could be increased.

### Example 6. Creation of an expression vector comprising the coding sequence of the MMAT (methylmalonyl-CoA acyl transferase) domain from Mycobacterium mycocerosic acid synthase (MAS).

*Mycobacterium* MAS is a multifunctional protein that catalyzes the synthesis of mycocerosic acid and that contains a domain with MMAT activity. The MMAT domain (amino acids 508-890) (SEQ ID NO: 18) of MAS from *Mycobacterium bovis BCG* (YP_979046) (SEQ ID NO: 19) was codon optimized for *E. coli* expression (SEQ ID NO: 20). The optimized sequence was synthesized and cloned into vector pTrcHisA (Invitrogen) between the *Bam*HI and *Hind*III sites. The resulting construct fused the MMAT domain with the His tag leader peptide encoded by the vector. The expression vector was introduced into a recombinant *E. coli* host that produces methylmalonyl-CoA. MMAT activity catalyzes the formation of methylmalonyl-ACP, which subsequently can be incorporated into the type II fatty acid synthesis pathway to form methyl branches at even positions of the fatty acid chain.

### Example 7. Method for detecting acyl-CoA.

This example describes an exemplary method for detecting and quantifying an acyl-CoA (e.g., methylmalonyl-CoA) in a sample, such as a sample of recombinant host cells producing branched-chain fatty acid.

A stable, labeled (deuterium) internal standard-containing master mix was prepared comprising d₃-3-hydroxymethylglutaryl-CoA (200 µl of 50 µg/ml stock in 10 ml of 15% trichloroacetic acid). An aliquot (500 µl) of the master mix was added to a 2 ml tube. Silicone oil (AR200; Sigma catalog number 85419; 800 µl) was layered onto the master mix. An *E. coli* culture (800 µl) was layered gently on top of the silicone oil, and the resulting sample was subjected to centrifugation at 20,000xg for five minutes at 4° C in an Eppendorf 5417C centrifuge. A portion (300 µl) of the master mix-containing layer was transferred to an empty tube and frozen on dry ice for 30 minutes.

The acyl-CoA content of samples was determined using HPLC/MS/MS. Individual coenzyme-A standards (propionyl-CoA, methylmalonyl-CoA, succinyl-CoA, malonyl-CoA, isobutyryl-CoA, isovaleryl-CoA, and acetyl-CoA) were purchased from Sigma Chemical Company (St. Louis, MO) and prepared as 500 µg/ml stocks in methanol. The analytes were pooled, and standards with all of the analytes were prepared by dilution with 15% trichloroacetic acid. Standards for regression were prepared by transferring 500 µl of the working standards to an autosampler vial containing 10 µL of the 50 µg/ml internal standard. Sample peak areas (or heights) were normalized to the stable-labeled internal standard (d₃-3-hydroxymethylglutaryl-CoA, Cayman Chemical Co.). Samples were assayed by HPLC/MS/MS on a Sciex API5000 mass spectrometer in positive ion Turbo Ion Spray. Separation was carried out by reversed-phase high performance liquid chromatography using a Phenomenex Onyx Monolithic C18 column (2 x 50 mm) and mobile phases of (1) 5 mM ammonium acetate, 5 mM dimethylbutylamine, 6.5 mM acetic acid and (2) acetonitrile with 0.1% formic acid, with the gradient set forth in Table C.

**TABLE C**

| Time | Mobile Phase A (%) | Mobile Phase B (%) |
|---|---|---|
| 0 min | 97.5 | 2.5 |
| 1.0 min | 97.5 | 2.5 |
| 2.5 min | 91.0 | 9.0 |
| 5.5 min | 45 | 55 |
| 6.0 min | 45 | 55 |
| 6.1 min | 97.5 | 2.5 |
| 7.5 min | - | - |
| 9.5 min | End Run | |

The conditions on the mass spectrometer were: DP 160, CUR 30, GS1 65, GS2 65, IS 4500, CAD 7, TEMP 650 C. The transitions set forth in Table D were used for the multiple reaction monitoring (MRM).

**TABLE D**

| Compound | Precursor Ion* | Product Ion* | Collision Energy | CXP |
|---|---|---|---|---|
| n-Propionyl-CoA | 824.3 | 317.2 | 41 | 32 |
| Methylmalonyl-CoA | 868.1 | 317.1 | 42 | 31 |
| Succinyl-CoA | 868.2 | 361.1 | 49 | 38 |
| Malonyl-CoA | 854.2 | 347.2 | 41 | 36 |
| Isobutyryl-CoA | 838.3 | 345.2 | 45 | 34 |
| Isovaleryl-CoA | 852.2 | 345.2 | 45 | 34 |
| Acetyl-CoA | 810.3 | 303.2 | 43 | 30 |
| d3-3-Hydroxymethylglutaryl-CoA | 915.2 | 408.2 | 49 | 13 |

| | | | | |
|---|---|---|---|---|
| *Energy (Volts) for MS/MS analysis | | | | |

### Example 8. Analysis of fatty acids produced by host cells.

This example illustrates a method of analyzing branched-chain fatty acids produced by cells (e.g., recombinant microbes).

Cell cultures (approximately 1.5 ml) were frozen in 2.0 ml glass vials and stored at -20° C until ready for processing. Samples were chilled on dry ice for 30 minutes and lyophilized overnight (∼16 hours) until dry. A 10 µl aliquot of internal standard (glyceryl trinonadecanoate (Sigma catalog number T4632-1G)) was added to each vial, followed by 400 µL of 0.5 N NaOH (in methanol). The vial was capped and vortexed for 10 seconds. Samples were incubated at 65° C for 30-50 minutes. Samples were then removed from the incubator, and 500 µl of boron trifluoride reagent (Aldrich catalog number B1252) was added. The samples were vortexed again for 10 seconds, incubated at 65° C for 10-15 minutes, and cooled to room temperature (approximately 20 minutes). Hexane (350 µl) was added, and the samples were again vortexed for 10 seconds. If the phases did not separate, 50-100 µl of saturated salt solution (5 g NaCl to 5 ml water) was added, and the sample was vortexed for 10 seconds. At least 100 µl of the top hexane layer was placed into the gas chromatography vial. The vial was capped and stored at 4° C until analyzed by gas chromatography.

Gas chromatography was performed as described in Table E below. A bacterial acid methyl ester standard (Sigma catalog number 47080-U) and a fatty acid methyl ester standard (Sigma catalog number 47885-U) were used to identify peaks in samples. A sample check standard using glyceryl tripalmitate (Sigma catalog number T5888-1G) was used to confirm esterification of samples. A blank standard (internal standard only) was used to assess background noise.

**TABLE E**

| | | | | | | | |
|---|---|---|---|---|---|---|---|
| Gas Chromatograph | HP 5890 GC Series II | | | | | | |
| Detector | FID 360° C 40 ml/min Hydrogen, 400 ml/min Air | | | | | | |
| Carrier Gas | Helium | | | | | | |
| Quantitative Program | GC Chemstation A.09.03. (Agilent) | | | | | | |
| Column | VF-5ms 15 M x 0.150 mm x 0.15 µm Varian catalog number CP9035 | | | | | | |
| Injection Liner | Gooseneck (with glass wool packing) | | | | | | |
| Injector | HP 7673 | | | | | | |
| Injection Syringe | 10 µL | | | | | | |
| Injection Mode | Split 25:1 | | | | | | |
| Injection volume | 4 µL (Plunger Speed = fast; 5 sample pumps) | | | | | | |
| Pre Injection Solvent Washes | 2 samples | | | | | | |
| Post Injection Solvent Washes | 3 for both acetone and hexane | | | | | | |
| Injector Temperature | 325° C | | | | | | |
| Total Program Time | 16 minutes | | | | | | |
| Thermal Program | | Initial Temp. (°C) | Initial Time (min) | Rate (°C/min) | Final Temp (°C) | Final Time (min) | |
| | | 90 | 0.75 | 20.0 | 325 | 1.0 | |
| | | | | 25.0 | 350 | 2.5 | |

### Example 9. Construction of expression vectors comprising S. cinnamonensis mutA and mutB and S. sviceus epi.

A synthetic DNA construct was generated comprising *Streptomyces cinnamonensis mutA* (SEQ ID NO: 24) (GenBank Accession No. AAA03040.1), *S*. *cinnamonensis mutB* (SEQ ID NO: 25) (GenBank Accession No. AAA03041.1), and a *Streptomyces sviceus* ATCC 29083 methylmalonyl-CoA epimerase gene (SEQ ID NO: 26) (GenBank Accession No. ZP_06919825.1). The genes were codon-optimized for expression in *E. coli.* An *Eco*RI restriction site was placed on the 5' end, and a *Bam*HI site was placed on the 3' end of the synthesized gene construct. These sites were subsequently used for cloning into a pZA31 vector (Expressys, Ruelzheim, Germany). A ribosome binding sequence and spacer was placed before the *mutA* and epimerase gene start codons (SEQ ID NO: 27). The plasmid was designated pZA31 *mutAB Ss epi.*

### Example 10. Construction of expression vectors comprising sbm and malE/sbm polynucleotides.

Sleeping beauty mutase (Sbm) (also known as methylmalonyl-CoA mutase (MCM)) is an enzyme that catalyzes the rearrangement of succinyl-CoA to L-methylmalonyl-CoA. The enzyme is vitamin B12 (cobalamin) dependent. Methylmalonyl-CoA is a building block for scattered branch-chain fatty acids (sBCFA) (i.e., branched-chain fatty acid comprising a methyl branch on one or more even number carbons of the fatty acid backbone). Plasmids comprising a polynucleotide encoding Sbm were generated to introduce multiple copies of the Sbm coding sequence, downstream of a regulatable promoter, into *E. coli* host cells.

A polynucleotide was synthesized based on the sequence of *E. coli sbm* (SEQ ID NO: 28) (GenBank Accession No. NP_417392.1) from *E. coli* strain MG1655. The nucleic acid sequence was codon-optimized to match the pattern of highly expressed *E. coli* genes while maintaining the native amino acid sequence of the enzyme. The generated nucleic acid sequence is set forth in SEQ ID NO: 29. A *Bam*HI and an *Xba*I site were added at the 5' end of the synthetic Sbm coding sequence with the sequence GGATCCATGTCTAGA (SEQ ID NO: 49) adjacent to the ATG translation initiation sequence. A *Sac*I restriction site sequence was added to the 3' end of the synthetic Sbm coding sequence. The gene was synthesized, cloned into a pUC57 vector, and sequenced (GenScript, Piscataway, NJ). The synthetic *sbm* was then released from pUC57 by restriction enzymes *Bam*HI and *Sac*I, and sub-cloned into plasmid pTrcHisA (Invitrogen, Carlsbad, CA) in frame with the poly-histidine sequence (GenScript, Piscataway, NJ). The plasmid was designated pTrcHisA *Ec sbm.* The sequence was confirmed by sequencing (GenScript, Piscataway, NJ). The recombinant protein encoded by the sequence contained a poly-histidine sequence (Met-Gly-Gly-Ser-His-His-His-His-His-His-Gly-Met-Ala-Ser-Met-Thr-Gly-Gly-Gln-Gln-Met-Gly-Arg-Thr-Asp-Asp-Asp-Asp-Lys-Asp-Arg-Trp-Gly-Ser (SEQ ID NO: 50)) and a full-length native Sbm amino acid sequence.

A recombinant methylmalonyl-CoA mutase has been reported to be insoluble in *E. coli* (Korotkova, N., and M. E. Lidstrom. J. Biological Chemistry 279: 13652-8 (2004)). Translation fusion with maltose-binding protein (MBP, encoded by *malE*) prevents aggregation of recombinant proteins (Kapust, R. B., and D. S. Waugh. Protein Science 8: 1668-74 (1999)). A recombinant construct was generated by inserting *malE* upstream of *sbm.* The *malE* polynucleotide was synthesized based on the sequence of maltose binding protein *(E. coli* MG1655 GenBank NC_000913.2 (GenScript, Piscataway, NJ)). A *Bam*HI site was placed adjacent to the translation initiation codon of *malE,* and an *Xba*I site was placed immediately 5' to the stop codon of the *malE* sequence (SEQ ID NO: 30). Also, one nucleotide was changed (T438 to C438) to remove a restriction site recognition sequence for *Bgl*II.

The MalE coding sequence (SEQ ID NO: 30) was first synthesized and cloned into a pUC57 plasmid. After confirming its sequence, the *malE* polynucleotide was released using restriction enzymes *Bam*HI and *Xba*I. The released *malE* was then re-cloned into plasmid pTrcHisA *Ec sbm* at *Bam*HI and *Xba*I sites (GenScript, Piscataway, NJ). The resulting plasmid was designated pTrcHisA *Ec malE Ec sbm.* The recombinant protein encoded by pTrcHisA *Ec malE Ec sbm* contains three peptides: the poly-histidine tag, full-length MBP, and full-length Sbm.

### Example 11. Construction of a recombinant expression vector comprising a polynucleotide encoding the methylmalonyl-CoA acyl transferase (MMAT) domain from Mycobacterium mycocerosic acid synthase (MAS).

*Mycobacterium* MAS is a multifunctional protein containing MMAT activity that catalyzes the synthesis of mycocerosic acid. The nucleic acid sequence encoding the MMAT domain (amino acids 508-890) (SEQ ID NO: 18) of MAS from *Mycobacterium bovis* BCG (GenBank Accession No. YP_979046) (SEQ ID NO: 19) was codon-optimized for *E. coli* expression (SEQ ID NO: 20). The optimized sequence, designated *"mmat,"* was synthesized and cloned into vector pTrcHisA (Invitrogen) between the *Bam*HI and *Hind*III sites. The resulting construct fused the MMAT domain with the poly-histidine tag encoded by the vector. The expression vector (pTrcHisA *mmat*) was introduced into a recombinant *E. coli* host that produces methylmalonyl-CoA. MMAT activity catalyzes the formation of methylmalonyl-ACP, which is incorporated by Type II fatty acid synthase into fatty acid, forming methyl branches at even positions of the fatty acid chain.

An expression vector encoding *Mycobacterium bovis* BCG fused to a poly-histidine tag also was generated. The pTrcHisA *mmat* plasmid DNA described above was amplified by PCR using oligonucleotides synthesized to include 5'*-Kpn*I (SEQ ID NO: 31) and 3'-*Hind*III restriction sites (SEQ ID NO: 32) (Integrated DNA Technologies, Inc., Coralville, IA). PCR was run on samples having 1 µl (2 ng) pTrcHisA *mmat* DNA, 1.5 µl of a 10 µM stock of each primer, 5 µl of 10X Pfx reaction mix (Invitrogen Carlsbad, CA), 0.5 µl of Pfx DNA polymerase (1.25 units), and 41 µl of water. PCR conditions were as follows: the samples were initially incubated at 95° C for three minutes, followed by 30 cycles at 95° C for 30 seconds (strand separation), 58° C for 30 seconds (primer annealing), and 68° C primer extension for 1.5 minutes. Following the cycles, the samples were incubated for 10 minutes at 68° C, and the samples were then held at 4° C.

The PCR products were purified using a QIAquick® PCR Purification Kit (Qiagen), digested with restriction enzymes *Kpn*I and *Hind*III and ligated (Fast-Link Epicentre Biotechnologies, Madison, WI) with *Kpn*I/*Hind*III-digested pZA31MCS (Expressys, Ruelzheim, Germany). The ligation mix was used to transform *E. coli* DH5α™ (Invitrogen Carlsbad, CA). Isolated colonies were screened by PCR using a sterile pipette tip stab as an inoculum into a reaction tube containing only water, followed by addition of the remaining PCR reaction cocktail (AccuPrime™ SuperMixII, Invitrogen Carlsbad, CA) and primers as described above.

Recombinant plasmids were isolated and purified using the QIAPrep® Spin Miniprep Kit (Qiagen) and characterized by restriction enzyme digestion (*Dra*I*, Kpn*I and *Hind*III from New England Biolabs, Beverly, MA). The plasmids were subsequently used to transform BW25113 *(E. coli* Genetics Stock Center, New Haven, CT) made competent using the calcium chloride method. Transformants were selected on Luria agar plates containing 34 µg/ml chloramphenicol. Plasmid DNA was isolated and purified using the QIAfilter™ Plasmid Midi Kit (Qiagen). DNA sequencing confirmed that the insert was *mmat* (SEQ ID NO: 34). The resulting plasmid incorporating a poly-histidine tag was designated pZA31 *mmat.*

### Example 12. Method of generating a recombinant host cell comprising an exogenous polynucleotide encoding a propionyl-CoA carboxylase and an exogenous polynucleotide encoding a methylmalonyl-CoA acyl transferase (MMAT) domain from Mycobacterium mycocerosic acid synthase (MAS).

This example describes an exemplary method for making a cell comprising an exogenous polynucleotide comprising a nucleic acid sequence encoding a polypeptide that catalyzes the conversion of propionyl-CoA to methylmalonyl-CoA and an exogenous polynucleotide comprising a nucleic acid sequence encoding a polypeptide that catalyzes the conversion of methylmalonyl-CoA to methylmalonyl-ACP. The method entails co-transduction *of E. coli* with plasmids containing a propionyl-CoA carboxylase gene from *Streptomyces coelicolor* and a gene encoding a MMAT domain from *Mycobacterium* MAS.

*E. coli* BW25113 cells *(E. coli* Genetic Stock Center, New Haven, CT) were made chemically competent for plasmid DNA transformation by a calcium chloride method. Actively growing 50 ml *E. coli* cultures were grown to an optical density (at 600 nm) of ∼0.4. Cultures were quickly chilled on ice, and the bacteria were recovered by centrifugation at 2700xg for 10 minutes. The supernatant was discarded and pellets were gently suspended in 30 ml of an ice-cold 80 mM MgCl₂, 20 mM CaCl₂ solution. Cells were again recovered by centrifugation at 2700xg for 10 minutes. The supernatant was discarded and pellets were gently resuspended in 2 ml of an ice-cold 0.1 M CaCl₂ solution.

Cells were transformed on ice in pre-chilled 14 ml round-bottom centrifuge tubes. Approximately 25 ng of each of pTrcHisA *mmat* and pZA31*-accA1-pccB* (described above) was incubated on ice with 100 µl of competent cells for 30 minutes. The cells were heat shocked at 42° C for 90 seconds and immediately placed on ice for two minutes. Pre-warmed SOC medium (500 µl; Invitrogen, Carlsbad, CA) was added and the cells allowed to recover at 37° C with 225 rpm shaking. A portion (50 µl) of the transformed cell mix was spread onto selective LB agar 100 µg/ml ampicillin and 34 µg/ml chloramphenicol plates to select for cells carrying the pTrcHisA *mmat* and pZA31/32-*accA1-pccB* plasmids. Individual colonies were picked from each plate and streaked onto LB agar (with ampicillin and chloramphenicol) to confirm the antibiotic resistance phenotype. Restriction endonuclease digestion analysis of isolated plasmid DNA with *Hae*II verified the plasmid DNA pool for each strain. A sample *of E. coli* BW25113 comprising pTrcHisA *mmat* and pZA31-*accA1-pccB* was deposited with American Type Culture Collection (ATCC), 10801 University Blvd., Manassas, VA, on December 14, 2010, under the provisions of the Budapest Treaty for the International Recognition of the Deposit of Microorganisms for the Purpose of Patent Procedure ("Budapest Treaty"), and assigned Deposit Accession No. [XXX] on [DATE].

### Example 13: Construction of an expression vector encoding Sorangium cellulosum So ce 56 methylmalonyl-CoA epimerase.

A *S. cellulosum* methylmalonyl-CoA epimerase synthetic gene *(So ce epi)* was designed and synthesized (SEQ ID NO: 37). The coding sequence was codon-optimization for expression in *E. coli* and modified to remove restriction sites (GenScript, Piscataway, NJ). The nucleic acid sequence was flanked with a *Sac*I site and a synthetic ribosome binding site from the pBAD vector (Invitrogen, Carlsbad, CA) adjacent to the translation initiation sequence (SEQ ID NO: 39). The synthetic gene was cloned as a *Sac*I/*Pst*I fragment into pTrcHisA *Ec sbm* and pTrcHisA *Ec malE Ec Sbm,* with the resulting plasmids designated as pTrcHisA *Ec sbm So ce epi* and pTrcHisA *Ec malE Ec sbm So ce epi,* respectively.

### Example 14. Construction of an expression vector encoding Kribbella flavida DSM 17836 methylmalonyl-CoA epimerase.

A *K. flavida* methylmalonyl-CoA epimerase gene *(Kf epi)* was designed and synthesized (SEQ ID NO: 35). The coding sequence was optimized for expression in *E. coli* and restriction sites were removed (GenScript, Piscataway, NJ). The gene was flanked with a *Sac*I site and a synthetic ribosome binding site from the pBAD vector adjacent to the translation initiation sequence (SEQ ID NO: 39). The synthetic gene was cloned as a *Sac*I/*Pst*I fragment into pTrcHisA *Ec sbm* and pTrcHisA *Ec malE Ec sbm.* The resulting plasmids were designated pTrcHisA *Ec sbm Kf epi* and pTrcHisA *Ec malE Ec sbm Kf epi,* respectively.

### Example 15. Production of host cells producing branched-chain fatty acid.

This example describes the production of branched-chain fatty acid using a recombinant host cell (e.g., *E. coli)* expressing polynucleotides encoding a propionyl-CoA carboxylase or a methylmalonyl-CoA mutase and a methylmalonyl-CoA epimerase, in some instances in conjunction with a polynucleotide encoding an acyl transferase and/or thioesterase.

It is useful to have the capacity to tailor the fatty acid chain length. Branched fatty acids of different lengths have different physical properties suitable for different commercial applications. To demonstrate the capacity to tailor the chain length of branched fatty acids, *E*. *coli* 'TesA (Cho, H., and J.E. Cronan, Jr. J. Biological Chemistry 270: 4216-9 (1995)) was incorporated into expression vectors described above and inserted into host cells. To create a pTrc *Ec 'tesA* expression vector, a truncated *E. coli tesA* (*'tesA*) cDNA (SEQ ID NO: 40) was created by PCR amplification of the *E. coli tesA* gene (GenBank Accession No. L06182). A 5' primer (SEQ ID NO: 41) was designed to anneal after the 26th codon of *tesA,* modifying the 27th codon from an alanine to a methionine and creating a *Nco*I restriction site. A 3' primer (SEQ ID NO: 43) incorporating a *Bam*HI restriction site was designed. PCR was performed with 50 µl of Pfu Ultra II Hotstart 2X master mix (Agilent Technologies, Santa Clara, CA), 1 µl of a mix of the two primers (10 µmoles of each), 1 µl of *E. coli* BW25113 genomic DNA, and 48 µl of water. PCR began with a two minute incubation at 95° C, followed by 30 cycles of 20 seconds at 95° C for denaturation, 20 seconds for annealing at 58° C, and 15 seconds at 72° C for extension. The sample was incubated at 72° C for three minutes and then held at 4° C. The PCR product (*Ec 'tesA*) was purified using a QIAquick® PCR Purification Kit (Qiagen, Valencia, CA). The bacterial expression vector pTrcHisA and the *'tesA* PCR product were digested with *Nco*I and *Bam*HI*.* The digested vector and insert were ligated using Fast-Link (Epicentre Biotechnologies, Madison, WI). The ligation mix was then used to transform *E. coli* TOP10 cells (Invitrogen, Carlsbad, CA). Recombinant plasmids were isolated using a QIAPrep® Spin Miniprep Kit (Qiagen) and characterized by gel electrophoresis of restriction digests with *Hae*II. DNA sequencing confirmed that the *'tesA* insert had been cloned and that the insert encoded the expected amino acid sequence (SEQ ID NO: 45). The resulting plasmid was designated pTrc *Ec 'tesA.*

To limit gene expression, the truncated *E. coli 'tesA* gene was subcloned into the low-copy bacterial expression vector pZS21-MCS (Expressys, Ruelzheim, Germany). The expression vector pTrc *Ec 'tesA* was a template in a PCR reaction using a 5' primer designed to create a flanking *Xho*I restriction site and include the pTrcHisA *lac* promoter (to replace the pZS21-MCS vector *tet* promoter) (SEQ ID NO: 46) and a 3' primer incorporating a *Hind*III restriction site (SEQ ID NO: 47). PCR was performed with 50 µl of Pfu Ultra II Hotstart 2X master mix (Agilent Technologies, Santa Clara, CA), 1 µl of a mix of the two primers (10 µmoles of each), 1µl of pTrc Ec 'tesA plasmid DNA (6 ng), and 48 µl of water. PCR began with a two minute incubation at 95° C, followed by 30 cycles of 20 seconds at 95° C for denaturation, 20 seconds for annealing at 57° C, and 20 seconds at 72° C for extension. The sample was incubated at 72° C for three minutes and then held at 4° C. The PCR product was purified using a QIAquick® PCR Purification Kit (Qiagen, Valencia, CA). The bacterial expression vector pZS21-MCS and the *Ec 'tesA* PCR product were digested with *Xho*I and *Hind*III. The digested vector and insert were ligated using Fast-Link (Epicentre Biotechnologies, Madison, WI). The ligation mix was then used to transform *E. coli* TOP10 cells (Invitrogen, Carlsbad, CA). Recombinant plasmids were isolated using a QIAPrep® Spin Miniprep Kit (Qiagen) and characterized by gel electrophoresis of restriction digests with *Hae*II. DNA sequencing confirmed that the *'tesA* insert had been cloned and that the insert encoded the expected amino acid sequence (SEQ ID NO: 45). The resulting plasmid was designated pZS22 *Ec 'tesA.*

An *E. coli* strain deficient in fatty acid degradation (Voelker, T. A., and H. M. Davies. J. Bacteriology 176: 7320-7 (1994)) and able to regulate transcription of recombinant genes was generated as follows. An *E. coli* K-12 strain (K27) defective in *fadD* lacks the fatty acyl-CoA synthetase responsible for an initial step in fatty acid degradation. The strain K27 (F-, *tyrT58(AS), fadD88, mel-1;* CGSC Strain # 5478) was obtained from the *E. coli* Genetic Stock Center (New Haven, CT). A genomic regulation cassette from strain DH5αZ1 [*lacl^{q},* PN25-tetR, Sp^{R}, deoR, supE44, Δ(lacZYA-argFV169), ϕ80 lacZΔM15 (Expressys, Ruelzheim, Germany)] was introduced into the host strain. The transducing phage P1vir was charged with DH5αZ1 DNA as follows. A logarithmically growing culture (5 ml LB broth containing 0.2% glucose and 5 mM CaCl₂) of donor strain, DH5αZ1, was infected with a 100 µl of a lysate stock of P1vir phage. The culture was further incubated three hours for the infected cells to lyse. The debris was pelleted, and the supernatant was further cleared through a 0.45 µm syringe filter unit. The fresh lysate was titered by spotting 10 µl of serial 1:10 dilutions of lysate in TM buffer (10 mM MgSO₄/10 mM Tris•Cl, pH 7.4) onto a 100 mm LB (with 2.5 mM CaCl₂) plate overlayed with a cultured lawn *of E. coli* in LB top agar (with 2.5 mM CaCl₂). The process was repeated using the newly created phage stock until the phage titer surpassed 10⁹ pfu/ml.

The higher titer phage stock was used to transduce fragments of the DH5αZ1 genome into a recipient K27 strain. An overnight culture (1.5 ml) of K27 was pelleted and resuspended in 750 µl of a PI salts solution (10 mM CaCl₂/5 mM MgSO₄). 100 µl of the suspended cells was inoculated with varying amounts of DH5αZ1 donor P1vir lysate (1, 10, and 100 µl) in sterile test tubes. The phage was allowed to adsorb to the cells for 30 minutes at 37° C. Absorption was terminated by addition of 1 ml LB broth plus 200 µl of 1 M sodium citrate, and the cultures were further incubated for 1 hour at 37° C with aeration. The cultures were pelleted, and the cells suspended in 100 µl of LB broth (plus 0.2 M sodium citrate) and spread onto LB agar plates with 50 µg/mL spectinomycin. Spectinomycin-resistant strains were isolated, and genomic DNAs were screened by PCR for the presence of *tetR, lacI^{q}* and *fadD88.* One such transductant was named K27-Z1 and used in further studies.

To transform K27-Z1, competent cells were placed on ice in pre-chilled 14 ml round bottom centrifuge tubes. Each plasmid was incubated with 50 µl of chemically competent K27-Z1 cells (Cohen, S. N., Change, A. C. Y., and L. Hsu. Proceedings National Academy Sciences U.S.A. 69: 2110-4 (1972)) for 30 minutes. The cells were heat shocked at 42° C for 90 seconds and immediately placed on ice for two minutes. Pre-warmed SOC medium (250 µl) (Invitrogen, Carlsbad, CA) was added, and the cells were allowed to recover at 37 °C with 125 rpm shaking for one hour. Transformed cell mix (20 µl) was spread onto selective LB agar with 100 µg/ml ampicillin to select for cells carrying the pTrcHisA-based plasmids. Transformed cell mix (50 µl) was spread onto LB agar with 34 µg/ml chloramphenicol to select for cells carrying the pZA31-based plasmids. Transformed cell mix (150 µl) was spread onto LB agar with 100 µg/ml ampicillin and 34 µg/ml chloramphenicol to select for cells carrying both the pTrcHisA-based and pZA31-based plasmids. In some cases, the creation of triple transformants required two transformations: a double transformant was originally created, made competent, and transformed by a third plasmid.

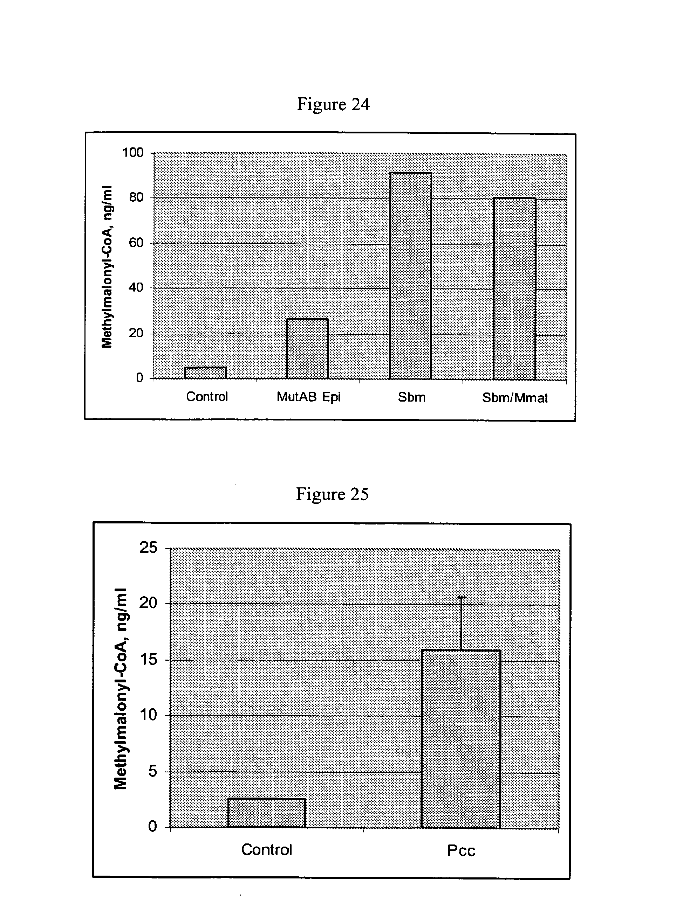

Using the methods described above, *E. coli* strain K27-Z1 was transduced with pTrcHisA pZA31 (control), pZA31 *mutAB Ss epi,* pTrcHisA *Ec sbm*, and pTrcHisA *Ec sbm*/*pZA31 Mb mmat.* The bacteria were cultured in M9 with glycerol (0.2%) at 22° C in flasks that were coated with black Scotch duct tape. After the bacteria reached an optical density (600 nm) of 0.4, a mix of IPTG, anhydrotetracycline, arabinose and hydroxocobalamin hydrochloride was added to the culture, giving final concentrations of 1 mM, 100 ng/ml, 0.2%, and 20 µM, respectively. Twenty-four hours later, the bacteria were harvested for coenzyme A analysis. Methylmalonyl-CoA production is illustrated in Figure 24. Host cells producing exogenous methylmalonyl-CoA mutase and methylmalonyl-CoA epimerase (encoded by pZA31 *mutAB Ss epi)* produced over 25 ng methylmalonyl-CoA per ml culture. Host cells comprising additional copies of the Sbm (methylmalonyl-CoA mutase) coding sequence produced over three times the amount of methylmalonyl-CoA per ml of culture, and co-expression of an methylmalonyl-CoA acyl transferase reduced the amount of methylmalonyl-CoA present in the culture medium.

Production of methylmalonyl-CoA in host cells expressing exogenous propionyl-CoA carboxylase also was studied and is illustrated in Figure 25. BW25113 (control) and BW25113 containing pZA31*-accA1-pccB* (labeled as Pcc in the figure) were cultured in LB, and the coenzyme-A thioesters were isolated and characterized as described above. Host cells comprising a polynucleotide encoding an exogenous propionyl-CoA carboxylase produced over about 15 ng methylmalonyl-CoA per ml of culture.

When *Ec 'tesA* was present, less longer-chain (fifteen and seventeen carbons) and more mid-chain (thirteen carbons) branched fatty acids were produced by the host cell, indicating that production of thioesterase increases the proportion of medium chain-length branched fatty acids produced by the inventive method.

### Example 16. Analysis of scattered branched fatty acid by two-dimensional (2D) gas chromatography.

To identify branched fatty acids produced by recombinant *E. coli* produced as described herein, fatty acids were isolated from bacterial cultures and derivatives were generated to facilitate identification. The fatty acid derivatives were separated by 2D gas chromatography and mass spectrometry was used to characterize fragmented samples. Derivatization of fatty acids to their 4,4' dimethyloxazoline derivatives prior to analysis via mass spectrometry has been described (Zhang, J.Y., QT. Yu, B.N. Liu and Z.H. Huang, Biomed Env. Mass Spectrom. 15:33 (1988)). By careful examination of minor spectral differences, it possible to determine the location of branch points on the backbones of fatty acid derivatives.

One liter of bacterial samples in LB (modified to contain only 0.5 mg/ml sodium chloride, unless otherwise indicated) with cyanocobalamin (20 µM) were cultured at 22° C for 25 hours following induction with IPTG, anhydrotetracycline, and arabinose. A cell pellet was collected by centrifugation at 3500 rpm, and the supernatant was discarded. The cell pellet was suspended in the remaining liquid, and the slurry was transferred into Pyrex tubes (#9826, Corning Inc., Lowell, MA). An equal volume of chloroform was added, and the sample was dried at room temperature overnight.

To produce samples for analysis, cell pellets (0.5 grams) were placed in a round bottom flask, and 0.5 grams of KOH pellets and 25 ml of water were added. The *E. coli* pellets and KOH solution were refluxed for three hours, and the sample was allowed to cool. Concentrated HCl was added drop-wise, using a methyl orange endpoint to ensure fatty carboxylic acids were in the acid form. The acidified aqueous solution was then extracted three times with 25 ml aliquots of hexane to extract the fatty acids into the organic layer.

To convert fatty acid to oxazoline derivatives, the hexane extract was evaporated to dryness and reconstituted into 5 ml of hexane to which sodium sulfate was added as a drying agent. After evaporating the sample to a 1 ml volume, a portion (0.6 ml) was decanted into a Reactitherm™ vial. The hexane in the Reactitherm™ vial was again evaporated to dryness, and 2 ml of 2-methyl-2-aminopropanol was added. The vial was capped and heated for 4 hours at 200° C. The cooled 2-methyl-2-aminopropanol solution was transferred to a scintillation vial, to which 5 ml of methylene chloride was added. The sample was washed with three 5 ml volumes of water. Sodium sulfate was added to the methylene chloride to remove any residual water, and an aliquot was transferred to a GC vial for analysis.

The derivatized samples were analyzed on a Leco Pegasus 4D Comprehensive 2D gas chromatograph time-of-flight mass spectrometer equipped with a 30M Supelco GammaDex 120 (Supelco 24307) column in the first dimension and a 2M Varian VF5-MS (Varian CP9034) column in the second dimension. Retention times of key chain-length fatty acids (in both first and second dimensions) in test samples were confirmed by identical preparation and analysis of a Supleco (47080-U) BAME (bacterial acid methyl ester) standard mixture. Using these columns, 4,4'dimethyloxazoline-derivatized branched-chain fatty acids were expected to elute prior to their linear chain-length homologs in the first dimension, and this was confirmed by the iso and anteiso structural isomers of C15 methyl esters (derivatized to their 4,4'-dimethyloxazoline derivatives) in the BAME standard reference above.

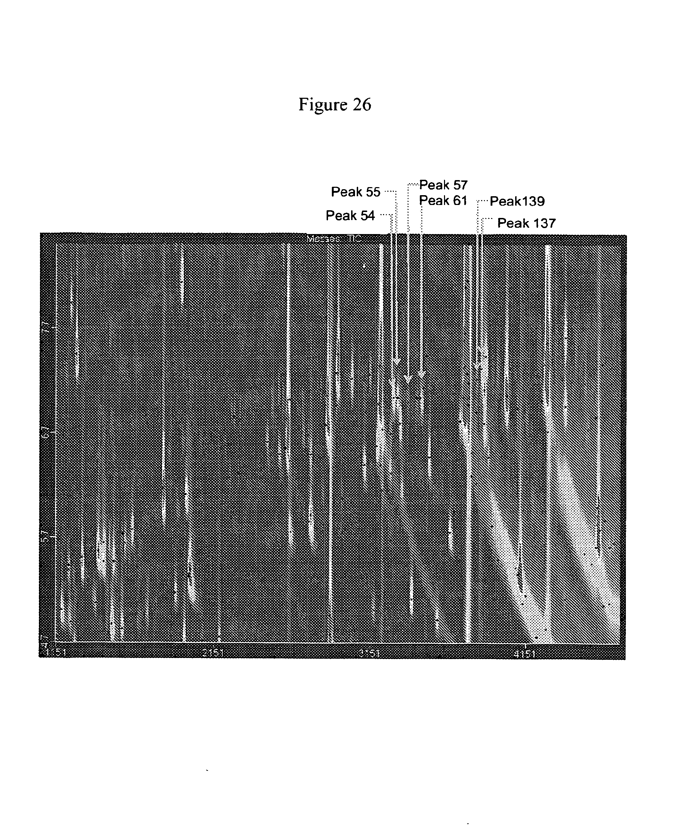

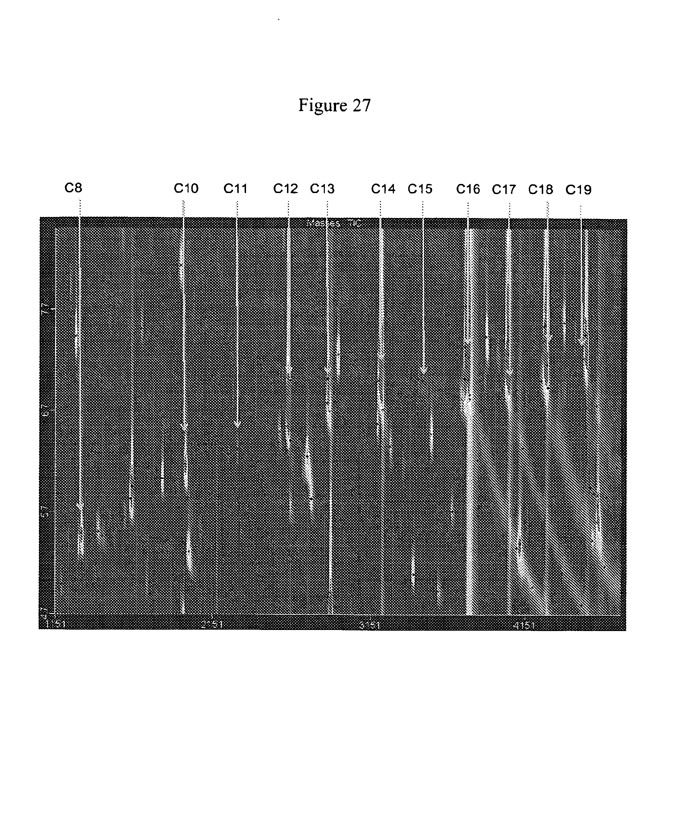

The profile of fatty acids produced by two strains was compared. The first strain was engineered to produce branched fatty acids [BL21 Star (DE3) (pTrcHisA *Ec sbm So ce epi* pZA31 *mmat*)] and the second was a control strain [BL21 Star (DE3) (pTrcHisA pZA31)]. A sample *of E. coli* BL21 Star (DE3) comprising pTrcHisA *Ec sbm So ce epi* and pZA31 *mmat* was deposited with American Type Culture Collection (ATCC), 10801 University Blvd., Manassas, VA, on December 14, 2010, under the provisions of the Budapest Treaty for the International Recognition of the Deposit of Microorganisms for the Purpose of Patent Procedure ("Budapest Treaty"), and assigned Deposit Accession No. [XXX] on [DATE]. The sample from the first strain revealed several peaks in the region where branched fatty acids were expected (Figure 26), whereas the sample from the control strain revealed no such peaks (Figure 27). For example, several peaks (labeled 54, 55, and 57) were in a position consistent with branched C15 acids, and peaks 137 and 139 were in a position expected for branched C17 acids. Mass spectrometry established that these peaks comprise branched fatty acids.

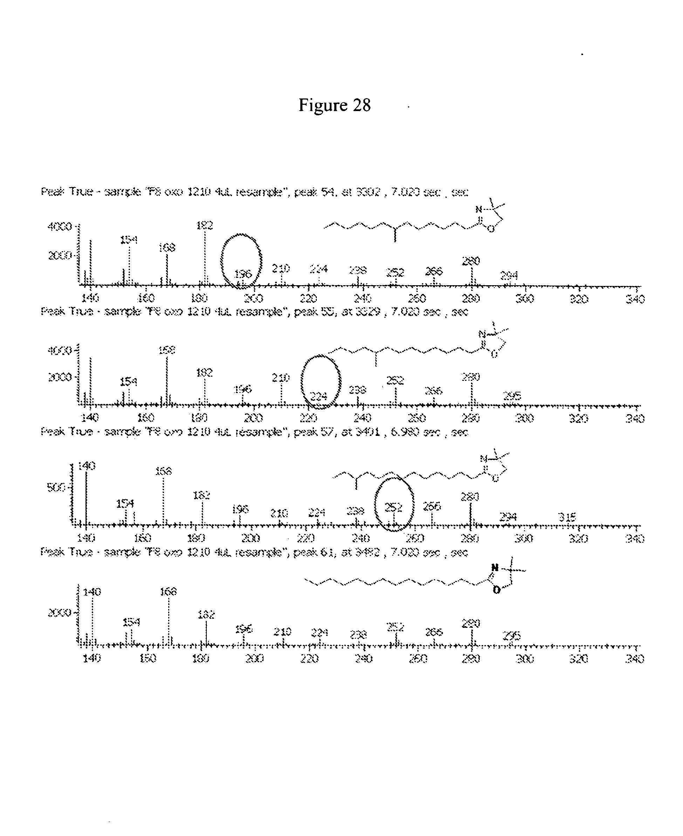

The mass spectral fragmentation pattern of oxazoline derivatives was used to confirm that the fatty acids identified using 2D GC contained branches. Oxazoline derivatives fragment along the length of the carbon chain starting from the functional end of the molecule. If a branch point occurs along the backbone, there is a gap in the mass spectrum pattern; which peak is missing (or reduced) depends on the location of the branch. Figure 28 depicts the mass spectra of the peaks labeled 54, 55, and 57 in Figure 26 as oxazoline derivatives of methyl-branched tetradecanoic fatty acids. The ions circled exhibit reduced or no intensity relative to the reference spectrum of linear pentadecanoic fatty acid (bottom spectrum), and were assigned as 8-methyl, 10-methyl, and 12-methyl (anteiso) tetradecanoic fatty acid (all as oxazoline derivatives). Peak 57 was tentatively identified as the anteiso C15 oxazoline derivative despite the similarity to the mass spec data for the linear sample because 1) peak 61 migrated at the position of an anteiso C15 standard on 2D gas chromatography, 2) the 252 molecular weight ion is present in slightly lower amounts relative to the nearby 238 and 266 molecular weight ions, and 3) anteiso compounds can be difficult to identify by this technique. The 8- and 10-branched fatty acids are shown in the top two profiles of Figure 28, readily identified by the almost complete absence of the fragment circled. Peaks 137 and 139 in Figure 26 were assigned as 8-methylhexadecanoic acid and 12-methylhexadecanoic acids (as oxazoline derivatives). Thus, B132 Star (DE3) (pTrcHisA *Ec sbm So ce epi* pZA31 *mmat*) (i.e., a recombinant microbe comprising overexpressed or recombinant polynucleotides encoding a methylmalonyl-CoA mutase, a methylmalonyl-CoA epimerase, and an acyl transferase) generated branched-chain C15 and C17 fatty acids comprising methyl branches on even-number carbons.

Branched fatty acid production also was observed in host cells producing exogenous propionyl-CoA carboxylase and *Streptomyces coelicolor* methylmalonyl-CoA mutase. The propionyl-CoA carboxylase gene-containing strain produced the branched fatty acids shown in Table F.

**TABLE F**

| | | | Molecular Weight | |
|---|---|---|---|---|
| Peak # | Proposed Compound ID | Formula | DMOX | as fatty acid |
| 38 | 6-methyl, dodecanoic acid (DMOX) | C₁₃H₃₃ (C₄H₈NO) | 267 | 214 |
| 40 | 8-methyl, dodecanoic acid (DMOX) | C₁₃H₃₃ (C₄H₈NO) | 267 | 214 |
| 61 | 6-methyl, tridecanoic acid (DMOX) | C₁₄H₃₅ (C₄H₈NO) | 281 | 228 |
| 62 | 8-methyl, tridecanoic acid (DMOX) | C₁₄H₃₅ (C₄H₈NO) | 281 | 228 |
| 101 | 6-methyl, tetradecanoic acid (DMOX) | C₁₅H₃₇ (C₄H₈NO) | 295 | 242 |
| 103 | 10-methyl, tetradecanoic acid (DMOX) | C₁₅H₃₇ (C₄H₈NO) | 295 | 242 |
| 140 | 10-methyl, pentadecanoic acid (DMOX) | C₁₆H₃₉ (C₄H₈NO) | 309 | 256 |
| 182 | 8-methyl, hexadecanoic acid (DMOX) | C₁₇H₄₁ (C₄H₈NO) | 323 | 270 |
| 189 | 12-methyl, hexadecanoic acid (DMOX) | C₁₇H₄₁ (C₄H₈NO) | 323 | 270 |

The *S. coelicolor* methylmalonyl-CoA mutase gene-containing microbe (BL21 Star (DE3) harboring pZA31 *mutAB Ss epi* pTrcHisA *mmat*) produced four branched fatty acids: 6-methyltetradecanoic acid, 10-methyltetradecanoic acid, 6-methylhexadecanoic acid, and 12-methylhexadecanoic acid.

Using 2D gas chromatography and mass spectrometry, fatty acid profiles were compared for two recombinant strains comprising *Ec sbm, So ce epi, Mb mmat* and containing or lacking a thioesterase coding sequence ('*tesA*). The amount of branched C15 fatty acids relative to branched C17 fatty acids was greater in the '*tesA*-containing strain. The area percent ratio of branched C15 fatty acid to branched C17 fatty acids in K27-Z1 (pTrcHisA *Ec sbm So ce epi pZA31 mmat*) was 1.4, while the ratio produced by K27-Z1 (pTrcHisA *Ec sbm So ce epi* pZA31 *mmat* pZS22 *Ec 'tesA*) was 7.0. Expression of a thioesterase shortened the chain length of branched fatty acids.

These results demonstrate that a cell of the invention producing propionyl-CoA carboxylase or producing methylmalonyl-CoA mutase, methylmalonyl-CoA epimerase, and acyl transferase generates branched-chain fatty acids comprising methyl branches on even-number carbons. Recombinant host cells further comprising a polynucleotide encoding a thioesterase preferentially produce fatty acid comprising shorter chain length.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method for producing branched-chain fatty acid comprising a methyl on one or more even number carbons, the method comprising culturing a cell comprising
(aa) an exogenous or overexpressed polynucleotide comprising a nucleic acid sequence encoding a polypeptide that catalyzes the conversion of propionyl-CoA to methylmalonyl-CoA and/or (bb) an exogenous or overexpressed polynucleotide comprising a nucleic acid sequence encoding a polypeptide that catalyzes the conversion of succinyl-CoA to methylmalonyl-CoA,
under conditions allowing expression of the polynucleotide(s) and production of branched-chain fatty acid, wherein the cell produces more branched-chain fatty acid comprising a methyl on one or more even number carbons than an otherwise similar cell that does not comprise the polynucleotide(s);
wherein the cell is *Escherichia coli;*
and wherein the polypeptide that catalyzes the conversion of propionyl-CoA to methylmalonyl-CoA is a propionyl-CoA carboxylase and/or the polypeptide that catalyzes the conversion of succinyl-CoA to methylmalonyl-CoA is a methylmalonyl-CoA mutase.

2. The method of claim 1 further comprising extracting from culture the branched-chain fatty acid or a product of the branched-chain fatty acid.

3. The method of any of the preceding claims, wherein the cell comprises an exogenous or overexpressed polynucleotide comprising a nucleic acid sequence encoding a methylmalonyl-CoA mutase and further comprises an exogenous or overexpressed polynucleotide comprising a nucleic acid sequence encoding a methylmalonyl-CoA epimerase.

4. The method of any of the preceding claims, wherein the cell further comprises an exogenous or overexpressed polynucleotide encoding an acyl transferase lacking polyketide synthesis activity and/or an exogenous or overexpressed polynucleotide comprising a nucleic acid sequence encoding a thioesterase.

5. The method of any of the preceding claims, wherein the cell produces a Type II fatty acid synthase.

6. The method of any of the preceding claims, wherein (i) the propionyl-CoA carboxylase is *Streptomyces coelicolor* PccB and AccA1 or PccB and AccA2 and/or (ii) the methylmalonyl-CoA mutase is *Janibacter* sp. HTCC2649 methylmalonyl-CoA mutase, *S. cinnamonensis* MutA and MutB, or *E. coli* Sbm.

7. The method of any of the preceding claims, wherein (i) the methylmalonyl-CoA mutase comprises an amino acid sequence having at least about 80% sequence identity to the amino acid sequence set forth in SEQ ID NOs: 3, 4, or 28 and/or (ii) the propionyl-CoA carboxylase comprises an amino acid sequence having at least about 80% sequence identity to the amino acid sequence set forth in SEQ ID NOs: 9 and 10.

8. The method of claim 4, wherein the acyl transferase is FabD, an acyl transferase domain of a polyketide synthase, or an acyl transferase domain of *Mycobacterium* mycocerosic acid synthase.

9. A cell comprising:
(i) an exogenous or overexpressed polynucleotide comprising a nucleic acid sequence encoding an acyl transferase lacking polyketide synthesis activity, and
(ii) an exogenous or overexpressed polynucleotide comprising a nucleic acid sequence encoding a propionyl-CoA carboxylase and/or an exogenous or overexpressed polynucleotide comprising a nucleic acid sequence encoding a methylmalonyl-CoA mutase,
wherein the polynucleotide(s) are expressed and the cell produces more branched-chain fatty acid comprising a methyl on one or more even number carbons than an otherwise similar cell that does not comprise the polynucleotide(s);
wherein the cell is *Escherichia coli.*

10. The cell of claim 9, wherein (i) the propionyl-CoA carboxylase is *Streptomyces coelicolor* PccB and AccA1 or PccB and AccA2 and/or (ii) the methylmalonyl-CoA mutase is *Janibacter* sp. HTCC2649 methylmalonyl-CoA mutase, *S. cinnamonensis* MutA and MutB, or E. *coli* Sbm.

11. The cell of claim 9, wherein (i) the methylmalonyl-CoA mutase comprises an amino acid sequence having at least about 80% sequence identity to the amino acid sequence set forth in SEQ ID NOs: 3, 4, or 28 and/or (ii) the propionyl-CoA carboxylase comprises an amino acid sequence having at least about 80% sequence identity to the amino acid sequence set forth in SEQ ID NOs: 9 and 10.

12. The cell of any one of claims 9-11, wherein the cell comprises an exogenous or overexpressed polynucleotide comprising a nucleic acid sequence encoding a methylmalonyl-CoA mutase and further comprises an exogenous or overexpressed polynucleotide comprising a nucleic acid sequence encoding a methylmalonyl-CoA epimerase.

13. The cell of any one of claims 9-12, wherein the acyl transferase is FabD, an acyl transferase domain of a polyketide synthase, or an acyl transferase domain of *Mycobacterium* mycocerosic acid synthase.

14. The cell of any one of claims 9-13, wherein the cell further comprises an exogenous or overexpressed polynucleotide comprises a nucleic acid sequence encoding a thioesterase.

15. The cell of any one of claims 9-14, wherein the cell has been modified to attenuate endogenous methylmalonyl-CoA mutase activity, endogenous methylmalonyl-CoA decarboxylase activity, and/or endogenous acyl transferase activity.

## Patentansprüche

1. Verfahren zur Herstellung einer verzweigtkettigen Fettsäure, die ein Methyl auf einem oder mehreren geradzahligen Kohlenstoffen umfasst, wobei das Verfahren das Kultivieren einer Zelle umfasst, umfassend
(aa) ein exogenes oder überexprimiertes Polynucleotid, das eine Nucleinsäuresequenz umfasst, die für ein Polypeptid codiert, das die Konvertierung von Propionyl-CoA zu Methylmalonyl-CoA katalysiert, und/oder
(bb) ein exogenes oder überexprimiertes Polynucleotid, das eine Nucleinsäuresequenz umfasst, die für ein Polypeptid codiert, das die Konvertierung von Succinyl-CoA zu Methylmalonyl-CoA katalysiert,
unter Bedingungen, die die Expression des Polynucleotids/der Polynucleotide und die Produktion verzweigtkettiger Fettsäure erlauben, wobei die Zelle mehr verzweigtkettige Fettsäure ein Methyl auf einem oder mehreren geradzahligen Kohlenstoffen umfassend als eine ansonsten ähnliche Zelle produziert, die das Polynucleotid/die Polynucleotide nicht umfasst;
wobei die Zelle *Escherichia coli* ist;
und wobei das Polypeptid, das die Konvertierung des Propionyl-CoA zu Methylmalonyl-CoA katalysiert, eine Propionyl-CoA-Carboxylase ist und/oder das Polypeptid, das die Konvertierung von Succinyl-CoA zu Methylmalonyl-CoA katalysiert, eine Methylmalonyl-CoA-Mutase ist.

2. Verfahren nach Anspruch 1, ferner umfassend das Extrahieren der verzweigtkettigen Fettsäure oder eines Produkts der verzweigtkettigen Fettsäure aus der Kultur.

3. Verfahren nach einem der vorstehenden Ansprüche, wobei die Zelle ein exogenes oder überexprimiertes Polynucleotid umfasst, umfassend eine Nucleinsäuresequenz, die für eine Methylmalonyl-CoA-Mutase codiert, und ferner ein exogenes oder überexprimiertes Polynucleotid umfasst, umfassend eine Nucleinsäuresequenz, die für eine Methylmalonyl-CoA-Epimerase codiert.

4. Verfahren nach einem der vorstehenden Ansprüche, wobei die Zelle ferner ein exogenes oder überexprimiertes Polynucleotid umfasst, das für eine Acyltransferase ohne Polyketidsynthese-Aktivität codiert, und/oder ein exogenes oder überexprimiertes Polynucleotid, umfassend eine Nucleinsäuresequenz, die für eine Thioesterase codiert.

5. Verfahren nach einem der vorstehenden Ansprüche, wobei die Zelle eine Typ-II-Fettsäure-Synthase produziert.

6. Verfahren nach einem der vorstehenden Ansprüche, wobei (i) die Propionyl-CoA-Carboxylase *Streptomyces coelicolor* PccB und AccA1 oder PccB und AccA2 ist und/oder (ii) die Methylmalonyl-CoA-Mutase *Janibactersp.* HTCC2649 Methyl-malonyl-CoA-Mutase, S. *cinnamonensis* MutA und MutB oder E. *coli* Sbm ist.

7. Verfahren nach einem der vorstehenden Ansprüche, wobei (i) die Methylmalonyl-CoA-Mutase eine Aminosäure-Sequenz mit wenigstens etwa 80 % Sequenzidentität zu der in SEQ ID Nr 3, 4 oder 28 dargestellten Aminosäure-sequenz umfasst, und/oder (ii) die Propionyl-CoA-Carboxylase eine Aminosäure-Sequenz mit wenigstens etwa 80 % Sequenzidentität zu der in SEQ ID 9 und 10 dargestellten Aminosäuresequenz umfasst.

8. Verfahren nach Anspruch 4, wobei die Acyltransferase FabD, eine Acyltransferase-Domäne einer Polyketidsynthase oder eine Acyltransferase-Domäne einer *Mycobacterium*-Mycocerosesäure-Synthase ist.

9. Zelle, umfassend:
(i) ein exogenes oder überexprimiertes Polynucleotid, das eine Nucleinsäuresequenz umfasst, die für eine Acyltransferase ohne Polyketidsynthese-Aktivität codiert, und
(ii) ein exogenes oder überexprimiertes Polynucleotid, das eine Nucleinsäuresequenz umfasst, die für eine Propionyl-CoA-Carboxylase codiert, und/oder ein exogenes oder überexprimiertes Polynucleotid, das eine Nuclein-säuresequenz umfasst, die für eine Methylmalonyl-CoA-Mutase codiert,
wobei das Polynucleotid/die Polynucleotide exprimiert werden und die Zelle mehr verzweigtkettige Fettsäure produziert, die ein Methyl auf einem oder mehreren geradzahligen Kohlenstoffen umfasst, als eine ansonsten ähnliche Zelle, die das Polynucleotid/die Polynucleotide nicht umfasst,
wobei die Zelle *Escherichia coli* ist;

10. Zelle nach Anspruch 9, wobei (i) die Propionyl-CoA-Carboxylase *Streptomyces coelicolor* PccB und AccA1 oder PccB und AccA2 ist und/oder (ii) die Methylmalonyl-CoA-Mutase *Janibactersp.* HTCC2649 Methylmalonyl-CoA-Mutase, S. *Cinnamonensis* MutA und MutB oder E. coli Sbm ist.

11. Zelle nach Anspruch 9, wobei (i) die Methylmalonyl-CoA-Mutase eine Aminosäure-Sequenz mit wenigstens etwa 80 % Sequenzidentität zu der in SEQ ID Nr 3, 4 oder 28 dargestellten Aminosäuresequenz umfasst, und/oder (ii) die Propionyl-CoA-Carboxylase eine Aminosäure-Sequenz mit wenigstens etwa 80 % Sequenzidentität zu der in SEQ ID 9 und 10 dargestellten Aminosäuresequenz umfasst.

12. Zelle nach einem der Ansprüche 9 bis 11, wobei die Zelle ein exogenes oder überexprimiertes Polynucleotid umfasst, umfassend eine Nucleinsäuresequenz, die für eine Methylmalonyl-CoA-Mutase codiert, und ferner ein exogenes oder überexprimiertes Polynucleotid umfasst, umfassend eine Nucleinsäuresequenz, die für eine Methylmalonyl-CoA-Epimerase codiert.

13. Zelle nach Anspruch 9 bis 12, wobei die Acyltransferase FabD, eine Acyltransferase-Domäne einer Polyketidsynthase oder eine Acyltransferase-Domäne einer *Mycobacterium*-Mycocerosesäure-Synthase ist.

14. Zelle nach einem der Ansprüche 9 bis 13, wobei die Zelle ferner ein exogenes oder überexprimiertes Polynucleotid umfasst, umfassend eine Nucleinsäuresequenz, die für eine Thioesterase codiert.

15. Zelle nach einem der Ansprüche 9 bis 14, wobei die Zelle modifiziert wurde, um endogene Methylmalonyl-CoA-Mutase-Aktivität, endogene Methylmalonyl-CoA-Decarboxylase-Aktivität und/oder endogene Acyltransferase-Aktivität zu verringern.

## Revendications

1. Procédé pour produire un acide gras à chaîne ramifiée comprenant un méthyle sur un ou plusieurs carbones à nombre pair, le procédé comprenant la culture d'une cellule comprenant
(aa) un polynucléotide surexprimé ou exogène comprenant une séquence d'acide nucléique codant pour un polypeptide qui catalyse la conversion du propionyl-coenzyme A en méthylmalonyl-coenzyme A et/ou (bb) un polynucléotide surexprimé ou exogène comprenant une séquence d'acide nucléique codant pour un polypeptide qui catalyse la conversion du succinyl-coenzyme A en méthylmalonyl-coenzyme A,
dans des conditions permettant l'expression du ou des polynucléotide(s) et la production d'acide gras à chaîne ramifiée, dans laquelle la cellule produit plus d'acides gras à chaîne ramifiée comprenant un méthyle sur un ou plusieurs carbones à nombre pair qu'une cellule par ailleurs similaire qui ne comprend pas le ou les polynucléotide(s) ;
dans lequel la cellule est *Escherichia coli* ;
et dans lequel le polypeptide qui catalyse la conversion du propionyl-coenzyme A en méthylmalonyl-coenzyme A est un propionyl-coenzyme A carboxylase et/ou le polypeptide qui catalyse la conversion du succinyl-coenzyme A en méthylmalonyl-coenzyme A est un méthylmalonyl-coenzyme A mutase.

2. Procédé selon la revendication 1, comprenant en outre l'extraction de la culture de l'acide gras à chaîne ramifiée ou d'un produit dérivé de l'acide gras à chaîne ramifiée.

3. Procédé selon l'une quelconque des revendications précédentes, dans lequel la cellule comprend un polynucléotide surexprimé ou exogène comprenant une séquence d'acide nucléique codant pour un méthylmalonyl-coenzyme A mutase et comprend en outre un polynucléotide surexprimé ou exogène comprenant une séquence d'acide nucléique codant pour une méthylmalonyl-coenzyme A épimérase.

4. Procédé selon l'une quelconque des revendications précédentes, dans lequel la cellule comprend en outre un polynucléotide surexprimé ou exogène codant pour une acyl transférase dépourvue d'activité de synthèse de polycétides et/ou un polynucléotide surexprimé ou exogène comprenant une séquence d'acide nucléique codant pour une thioestérase.

5. Procédé selon l'une quelconque des revendications précédentes, dans lequel la cellule produit une acide gras synthase de type II.

6. Procédé selon l'une quelconque des revendications précédentes, dans lequel (i) la propionyl-coenzyme A carboxylase est *Streptomyces coelicolor* PccB et AccA1 ou PccB et AccA2 et/ou (ii) la méthylmalonyl-coenzyme A mutase est *Janibacter* sp. HTCC2649 méthylmalonyl-coenzyme A mutase, S. *cinnamonensis* MutA et MutB, ou *E. coli* Sbm.

7. Procédé selon l'une quelconque des revendications précédentes, dans lequel (i) la méthylmalonyl-coenzyme A mutase comprend une séquence d'acides aminés ayant une identité de séquence d'au moins environ 80 % avec la séquence d'acides aminés indiquée dans les NO ID SÉQ : 3, 4, ou 28 et/ou (ii) la propionyl-coenzyme A carboxylase comprend une séquence d'acides aminés ayant une identité de séquence d'au moins environ 80 % avec la séquence d'acides aminés indiquée dans les NO ID SÉQ : 9 et 10.

8. Procédé selon la revendication 4, dans lequel l'acyl transférase est FabD, un domaine d'acyl transférase d'une polycétide synthase, ou un domaine d'acyl transférase de l'acide mycocérosique *Mycobacterium* synthase.

9. Cellule comprenant :
(i) un polynucléotide exogène ou surexprimé comprenant une séquence d'acide nucléique codant pour une acyl transférase dépourvue d'activité de synthèse de polycétides, et
(ii) un polynucléotide surexprimé ou exogène comprenant une séquence d'acide nucléique codant pour une propionyl-coenzyme A carboxylase et/ou un polynucléotide surexprimé ou exogène comprenant une séquence d'acide nucléique codant pour une méthylmalonyl-coenzyme A mutase,
dans laquelle le ou les polynucléotide(s) sont exprimés et la cellule produit plus d'acides gras à chaîne ramifiée comprenant un méthyle sur un ou plusieurs carbones à nombre pair qu'une cellule par ailleurs similaire qui ne comprend pas le ou les polynucléotide(s).
dans lequel la cellule est *Escherichia coli.*

10. Cellule selon la revendication 9, dans laquelle (i) la propionyl-coenzyme A carboxylase est *Streptomyces coelicolor* PccB et AccA1 ou PccB et AccA2 et/ou (ii) la méthylmalonyl-coenzyme A mutase est *Janibactersp.* HTCC2649 méthylmalonyl-coenzyme A mutase, S. *cinnamonensis* MutA et MutB, ou *E. coli* Sbm.

11. Cellule selon la revendication 9, dans laquelle (i) la méthylmalonyl-coenzyme A mutase comprend une séquence d'acides aminés ayant une identité de séquence d'au moins environ 80 % avec la séquence d'acides aminés indiquée dans les NO ID SÉQ : 3, 4, ou 28 et/ou (ii) la propionyl-coenzyme A carboxylase comprend une séquence d'acides aminés ayant une identité de séquence d'au moins environ 80 % avec la séquence d'acides aminés indiquée dans le NO ID SÉQ : 9 et 10.

12. Cellule selon l'une quelconque des revendications 9-11, dans laquelle la cellule comprend un polynucléotide surexprimé ou exogène comprenant une séquence d'acide nucléique codant pour un méthylmalonyl-coenzyme A mutase et comprend en outre un polynucléotide surexprimé ou exogène comprenant une séquence d'acide nucléique codant pour un méthylmalonyl-coenzyme A épimérase.

13. Cellule selon l'une quelconque des revendications 9-12, dans laquelle l'acyl transférase est FabD, un domaine d'acyl transférase d'une polycétide synthase, ou un domaine d'acyl transférase de l'acide mycocérosique *Mycobacterium* synthase.

14. Cellule selon l'une quelconque des revendications 9-13, dans laquelle la cellule comprend en outre un polynucléotide surexprimé ou exogène qui comprend une séquence d'acide nucléique codant pour un thioestérase.

15. Cellule selon l'une quelconque des revendications 9-14, dans laquelle la cellule a été modifiée pour atténuer l'activité de la méthylmalonyl-coenzyme A mutase endogène, l'activité de la méthylmalonyl-coenzyme A décarboxylase, et/ou l'activité de l'acyl transférase endogène.


### SEQUENCE LISTING

<110> Saunders, et al.
<120> Scattered Branched-Chain Fatty Acid and Biological Production Thereof
<130> 30766/45912
<150> 61/294274 <151> 2010-01-12
<160> 70
<170> PatentIn version 3.5
<210> 1
   <211> 1917
   <212> DNA
   <213> Escherichia coli
<400> 1
   atggcaagca cggaccaggg taccaacccg gcagacaccg acgacctgac gccaaccact      60
   ctgagtctgg cgggcgattt tccgaaagca accgaagaac agtgggagcg cgaagtggag     120
   aaagttctga accgtggccg tccgccggag aaacagctga cgtttgcgga atgtctgaaa     180
   cgcctgacgg tccacacagt agacggcatt gacattgtgc caatgtatcg cccgaaagat     240
   gcgccgaaga aactgggtta cccaggcgtt gccccattta cacgtgggac cacggttcgt     300
   aatggcgata tggacgcatg ggatgtccgt gcactgcatg aagatccgga tgagaaattt     360
   acgcgcaaag cgattctgga agggctggaa cgcggggtta catctctgct gctgcgtgtg     420
   gacccggacg ctattgctcc agaacacctg gatgaagtgc tgtctgacgt gctgctggag     480
   atgaccaaag tagaagtctt tagtcgttac gatcaaggcg ccgctgccga ggcgctggta     540
   tctgtgtacg agcgcagcga taaaccggct aaggacctgg ctctgaatct gggtctggac     600
   ccgatcgcct tcgcggcact gcaggggacg gaacctgatc tgactgtcct gggtgattgg     660
   gtgcgtcgcc tggcaaaatt tagcccagat tctcgtgcag tgaccatcga tgcgaacatt     720
   tatcataatg cgggtgcggg cgatgtagca gagctggctt gggccctggc taccggtgcg     780
   gaatatgttc gtgcactggt agaacaaggt tttacggcga ccgaggcgtt cgatacgatt     840
   aactttcgtg tgaccgcaac ccatgatcag tttctgacaa tcgcgcgtct gcgcgcactg     900
   cgtgaggcgt gggcgcgcat tggggaggta tttggggttg atgaggataa acgtggcgcc     960
   cgtcaaaatg cgatcacgag ttggcgcgat gtgacacgcg aggacccgta tgtgaatatc    1020
   ctgcgcggga gcatcgctac attttctgca agcgtgggtg gggccgaaag tattacaact    1080
   ctgcctttta cccaggcact gggtctgcca gaagacgatt ttccgctgcg tatcgctcgt    1140
   aataccggta tcgttctggc cgaagaagtg aacatcggtc gtgttaatga tccggccggc    1200
   ggtagctatt acgtggaaag tctgactcgt agtctggccg atgcagcgtg gaaagagttc    1260
   caagaagtgg agaaactggg cggcatgagc aaggcggtga tgacggaaca tgtaacgaaa    1320
   gtgctggatg cctgcaatgc agaacgcgcg aaacgcctgg ccaatcgcaa acagccgatt 1380
   accgcagtaa gcgaatttcc tatgattggg gcgcgctcta tcgaaacgaa accttttcct 1440
   gccgcaccgg cccgtaaagg tctggcatgg catcgcgaca gtgaagtatt cgaacaactg 1500
   atggatcgca gcaccagtgt gagtgaacgt ccaaaggttt tcctggcgtg cctgggcaca 1560
   cgtcgtgact tcggtggtcg tgagggtttt agcagcccag tgtggcatat cgcaggcatt 1620
   gacaccccac aggttgaggg tggcacaacc gcagaaatcg tagaagcatt caagaaatct 1680
   ggggcacaag ttgcggatct gtgctctagc gccaaagtgt acgctcagca gggtctggag 1740
   gtggccaaag ctctgaaagc agctggcgcc aaagccctgt atctgagcgg tgcctttaag 1800
   gagttcggcg atgatgcggc tgaggcggag aaactgatcg atggtcgcct gtttatgggt 1860
   atggatgtgg ttgacactct gtctagtacg ctggacattc tgggtgtagc aaagtaa 1917
<210> 2
   <211> 2193
   <212> DNA
   <213> Escherichia coli
<400> 2
   atgagtacac tgcctcgttt tgactctgtt gacctgggga acgcgcctgt tccggcggat      60
   gcggcccgtc gcttcgagga actggcggca aaagcgggca cgggtgaggc gtgggagacc     120
   gcggagcaga ttccggttgg tacactgttc aatgaagacg tttacaaaga tatggactgg     180
   ctggacacgt acgccgggat tccgccattc gttcacggcc cgtacgcgac gatgtacgct     240
   ttccgtccgt ggacaattcg tcaatacgcc gggtttagca cggcgaaaga aagtaatgct     300
   ttctaccgcc gtaacctggc ggcggggcaa aagggtctgt ctgtggcatt cgacctgccg     360
   acccaccgcg gttacgatag cgataatccg cgcgtggcag gggacgtggg tatggccggg     420
   gtggccatcg acagtattta cgacatgcgt gaactgtttg caggcattcc gctggaccag     480
   atgagcgtga gtatgacgat gaatggtgcc gtcctgccga ttctggcact gtatgtggtt     540
   acagccgaag aacaaggtgt gaagccggaa cagctggctg gcaccatcca gaacgatatt     600
   ctgaaggagt tcatggtgcg taacacctat atctatccgc cgcaaccgtc tatgcgcatc     660
   atcagtgaga tctttgcgta tactagtgca aatatgccga agtggaactc tatcagtatt     720
   agtggctatc acatgcagga ggcgggcgcc actgccgata tcgaaatggc ctatacgctg     780
   gccgatggcg ttgattatat tcgtgcaggc gaaagcgtcg gtctgaacgt ggaccagttc     840
   gccccgcgtc tgagcttctt ttggggtatt ggcatgaatt tctttatgga agtcgcaaaa     900
   ctgcgtgccg cccgcatgct gtgggccaaa ctggtgcacc aattcggccc gaagaacccg     960
   aagagcatga gcctgcgcac gcacagtcaa accagcggct ggagcctgac cgcgcaggac    1020
   gtatataaca acgtagttcg cacctgtatt gaggcgatgg cagccaccca gggtcacacc    1080
   cagagcctgc atacaaactc tctggacgag gccatcgcac tgccgacaga cttcagcgcc    1140
   cgcatcgcgc gtaatactca actgtttctg caacaggaaa gcggtactac ccgtgtgatc 1200
   gatccgtggt ctggcagtgc atatgtcgag gaactgacct gggatctggc ccgtaaagcg 1260
   tggggtcata tccaggaagt cgagaaagtg ggtggtatgg ctaaagcaat tgagaaaggc 1320
   atcccgaaaa tgcgcattga agaagcggca gcgcgcaccc aagcacgcat cgacagcggt 1380
   cgccagccgc tgattggcgt gaacaaatat cgcctggaac atgaaccgcc actggatgtt 1440
   ctgaaagtag ataactctac cgtcctggcg gagcagaaag cgaaactggt taagctgcgt 1500
   gcggaacgcg atcctgagaa agttaaagcg gcgctggata aaatcacttg ggccgcgggc 1560
   aacccggatg ataaagaccc agaccgtaat ctgctgaagc tgtgtattga cgcgggtcgt 1620
   gctatggcga ctgtcggcga aatgagcgat gcgctggaga aagtatttgg tcgttatacc 1680
   gcgcaaattc gtactatttc tggtgtctat agcaaggaag ttaagaatac tccagaagta 1740
   gaagaagcgc gtgaactggt agaagaattt gagcaggctg aaggtcgccg tccacgcatt 1800
   ctgctggcca aaatgggcca ggatggccat gatcgcggtc agaaagttat tgctactgct 1860
   tatgctgatc tgggcttcga tgttgatgtc ggccctctgt tccagactcc agaggaaact 1920
   gcccgccagg ctgttgaagc tgacgtccat gtcgttggcg ttagctctct ggctggcggc 1980
   catctgaccc tggtccctgc tctgcgcaag gaactggata agctgggccg ccctgatatt 2040
   ctgattactg tcggcggcgt cattcctgaa caggatttcg atgaactgcg caaggatggc 2100
   gctgtcgaaa tttatacccc tggcaccgtc attcctgaat ctgctatttc tctggtcaag 2160
   aagctgcgcg ctagcctgga tgcctaactc gag 2193
<210> 3
   <211> 671
   <212> PRT
   <213> Janibacter sp. HTCC2649
<400> 3
   Met Ala Arg Thr Tyr Ala Gly His Ser Ser Ala Ala Ala Ser Asn Ala
   1 5 10 15
   Leu Tyr Arg Arg Asn Leu Ala Lys Gly Gln Thr Gly Leu Ser Val Ala
   20 25 30
   Phe Asp Leu Pro Thr Gln Thr Gly Tyr Asp Pro Asp His Val Leu Ala
   35 40 45
   Arg Gly Glu Val Gly Lys Val Gly Val Pro Ile Ser His Ile Gly Asp
   50 55 60
   Met Arg Ala Leu Phe Asp Gln Ile Pro Leu Gly Gln Met Asn Thr Ser
   65 70 75 80
   Met Thr Ile Asn Ala Thr Ala Met Trp Leu Leu Ala Met Tyr Gln Val
   85 90 95
   Ala Ala Glu Asp Gln Ala Thr Ala Ala Asp Glu Asp Pro Ala Ser Val
   100 105 110
   Val Lys Ala Leu Gly Gly Thr Thr Gln Asn Asp Ile Ile Lys Glu Tyr
   115 120 125
   Leu Ser Arg Gly Thr Tyr Val Phe Ala Pro Ala Pro Ser Leu Arg Leu
   130 135 140
   Ile Thr Asp Met Val Ser Tyr Thr Val Ser Asp Ile Pro Lys Trp Asn
   145 150 155 160
   Pro Ile Asn Ile Cys Ser Tyr His Leu Gln Glu Ala Gly Ala Thr Pro
   165 170 175
   Val Gln Glu Ile Ala Tyr Ala Met Ser Thr Ala Ile Ala Val Leu Asp
   180 185 190
   Ala Val Arg Asp Ala Gly Gln Val Pro Gln Glu Arg Phe Gly Glu Val
   195 200 205
   Val Ala Arg Ile Ser Phe Phe Val Asn Ala Gly Val Arg Phe Val Glu
   210 215 220
   Glu Met Cys Lys Met Arg Ala Phe Val Glu Leu Trp Asp Glu Leu Thr
   225 230 235 240
   Arg Glu Arg Tyr Gly Val Thr Asp Ala Lys Gln Arg Arg Phe Arg Tyr
   245 250 255
   Gly Val Gln Val Asn Ser Leu Gly Leu Thr Glu Ala Gln Pro Glu Asn
   260 265 270
   Asn Val Gln Arg Ile Val Leu Glu Met Leu Ala Val Thr Leu Ser Lys
   275 280 285
   Gly Ala Arg Ala Arg Ala Val Gln Leu Pro Ala Trp Asn Glu Ala Leu
   290 295 300
   Gly Leu Pro Arg Pro Trp Asp Gln Gln Trp Ser Leu Arg Met Gln Gln
   305 310 315 320
   Val Leu Ala Tyr Glu Ser Asp Leu Leu Glu Tyr Glu Asp Leu Phe Glu
   325 330 335
   Gly Ser Ala Val Val Glu Ala Lys Val Ala Glu Leu Val Ala Gly Ala
   340 345 350
   Lys Ala Glu Ile Ala Arg Val Ala Glu Leu Gly Gly Ala Val Ala Ala
   355 360 365
   Val Glu Ser Gly Tyr Met Lys Ser Ala Leu Val Ala Ser His Ala Leu
   370 375 380
   Arg Arg Gln Arg Ile Glu Ala Gly Glu Asp Ile Val Val Gly Val Asn
   385 390 395 400
   Lys Phe Glu Thr Thr Glu Pro Asn Pro Leu Thr Ala Asp Leu Asp Thr
   405 410 415
   Ala Ile Gln Ser Val Asp Ala Gly Val Glu Ala Ala Ala Ala Lys Ala
   420 425 430
   Val Arg Glu Trp Arg Glu Thr Arg Asp Ala Asp Pro Val Lys Arg Glu
   435 440 445
   Arg Ala Val Ala Ala Leu Ala Arg Leu Lys Ala Ala Ala Gln Thr Asp
   450 455 460
   Glu Asn Leu Met Glu Ala Ser Ile Glu Cys Ala Arg Ala Glu Val Thr
   465 470 475 480
   Thr Gly Glu Trp Ala Gln Ala Leu Arg Glu Val Phe Gly Glu Phe Arg
   485 490 495
   Ala Pro Thr Gly Val Thr Gly Thr Val Gly Leu Thr Gly Gly Ala Ala
   500 505 510
   Gly Ala Glu Leu Ser Ala Val Arg Glu Arg Val Ala Gly Leu Arg Asp
   515 520 525
   Glu Leu Gly Glu Thr Leu Arg Val Leu Val Gly Lys Pro Gly Leu Asp
   530 535 540
   Gly His Ser Asn Gly Ala Glu Gln Ile Ala Val Arg Ala Arg Asp Ala
   545 550 555 560
   Gly Phe Glu Val Ile Tyr Gln Gly Ile Arg Leu Thr Pro Glu Gln Ile
   565 570 575
   Val Ala Ala Ala Val Ser Glu Asp Val His Leu Val Gly Ile Ser Ile
   580 585 590
   Leu Ser Gly Ser His Met Glu Leu Ile Pro Glu Val Leu Asp Arg Leu
   595 600 605
   Arg Glu Ala Gly Ala Gly Asp Ile Pro Val Ile Val Gly Gly Ile Ile
   610 615 620
   Pro Glu Ser Asp Ala Ala Lys Leu Lys Ala Ile Gly Val Ala Glu Val
   625 630 635 640
   Phe Thr Pro Lys Asp Phe Gly Leu Asn Asp Ile Met Gly Arg Phe Val
   645 650 655
   Asp Val Ile Arg Asp Ser Arg Leu Thr Thr Ala Ala Pro Thr Val
   660 665 670
<210> 4
   <211> 571
   <212> PRT
   <213> Escherichia coli
<400> 4
   Met Thr Val Ala Pro Lys Arg Pro Ala Ala Met Thr Leu Ala Ala His
   1 5 10 15
   Phe Pro Glu Arg Thr Gln Glu Gln Trp Arg Asp Leu Val Ala Gly Val
   20 25 30
   Val Asn Lys Gly Arg Pro Glu Asp Gln His Leu Ser Gly Asp Asp Ala
   35 40 45
   Val Ala Thr Met Arg Ser His Leu Glu Gly Gly Leu Asp Ile Glu Pro
   50 55 60
   Leu Tyr Met Lys Ser Ser Asp Pro Val Pro Leu Gly Val Pro Gly Ala
   65 70 75 80
   Met Pro Phe Thr Arg Gly Arg Ala Leu Arg Asp Ala Asp Val Pro Trp
   85 90 95
   Asp Val Arg Gln Val His Asp Asp Pro Asp Ala Ala Ala Thr Arg Gln
   100 105 110
   Leu Val Leu Ala Asp Leu Glu Asn Gly Val Thr Ser Val Trp Leu His
   115 120 125
   Val Gly Ala Asp Gly Leu Ala Pro Asn Asp Val Ala Glu Ala Leu Ala
   130 135 140
   Glu Val Arg Leu Glu Leu Ala Pro Val Val Val Ser Ser Trp Asp Asp
   145 150 155 160
   Gln Thr Ala Ala Ala Asp Ala Leu Tyr Ala Val Leu Ser Gly Ser Arg
   165 170 175
   Ala Ser Ser Gly Asn Leu Gly His Asp Pro Leu Gly Ala Ala Ala Arg
   180 185 190
   Thr Gly Ser Ala Pro Asp Leu Ala Pro Leu Ala Asp Ala Val Arg Arg
   195 200 205
   Leu Ala Asp His Gly Glu Ile Arg Ala Ile Thr Val Asp Thr Arg Val
   210 215 220
   His Gly Asp Ala Gly Val Thr Val Thr Asp Glu Val Ala Phe Ala Leu
   225 230 235 240
   Ala Thr Gly Val Ala Tyr Leu Arg His Leu Glu Ser Glu Gly Val Asp
   245 250 255
   Val Ala Glu Ala Phe Arg Asn Ile Glu Phe Arg Val Ser Ala Thr Ala
   260 265 270
   Asp Gln Phe Leu Thr Ala Ala Ala Leu Arg Ala Leu Arg Arg Ala Trp
   275 280 285
   Ala Arg Ile Gly Glu Ser Val Gly Val Pro Glu Thr Ser Arg Gly Ala
   290 295 300
   Phe Thr His Ala Val Thr Ser Gly Arg Ile Phe Thr Arg Asp Asp Ala
   305 310 315 320
   Trp Thr Asn Ile Leu Arg Ser Thr Leu Ala Thr Phe Gly Ala Ser Leu
   325 330 335
   Gly Gly Ala Asp Ala Ile Thr Val Leu Pro Phe Asp Thr Val Ser Gly
   340 345 350
   Leu Pro Thr Pro Phe Ser Arg Arg Ile Ala Arg Asn Thr Gln Ile Leu
   355 360 365
   Leu Ala Glu Glu Ser Asn Val Ala Arg Val Thr Asp Pro Ala Gly Gly
   370 375 380
   Ser Trp Tyr Val Glu Thr Leu Thr Asp Asp Val Ala Lys Ala Ala Trp
   385 390 395 400
   Glu Thr Phe Gln Glu Ile Glu Ser Ala Gly Gly Met Val Ala Ala Leu
   405 410 415
   Ala Asn Gly Leu Val Ala Gln Arg Ile Leu Ala Ala Val Ala Glu Arg
   420 425 430
   Asp Ala Ala Leu Ala Thr Arg Ser Thr Pro Ile Thr Gly Val Ser Thr
   435 440 445
   Phe Pro Leu Ala Gly Glu Lys Pro Leu Glu Arg Val Val Arg Ala Glu
   450 455 460
   Leu Pro Val Gln Pro Asn Ala Leu Ala Pro His Arg Asp Ser Ala Ile
   465 470 475 480
   Phe Glu Ala Leu Arg Asp Arg Ser Ala Ala Tyr Ala Thr Glu His Gly
   485 490 495
   His Ala Pro Arg Val Ser Val Pro Thr Leu Asp Val Pro Arg Ala Ala
   500 505 510
   Asp Arg Arg Ile Asp Ala Val Asn Leu Leu Thr Val Ala Gly Ile Asp
   515 520 525
   Ala Val Asp Gly Asp Thr Glu Ser Ala Ala Ala Leu Thr Gly Thr Asp
   530 535 540
   Lys Gly Tyr Glu Gly Val Ala Lys Asp Met Asp Val Val Ala Phe Leu
   545 550 555 560
   Ser Asp Leu Leu Asp Thr Thr Gly Ala Pro Ala
   565 570
<210> 5
   <211> 146
   <212> PRT
   <213> Escherichia coli
<400> 5
   Met Leu Thr Arg Ile Asp His Ile Gly Ile Ala Cys Phe Asp Leu Asp
   1               5                   10                  15
   Lys Thr Val Glu Phe Tyr Arg Ala Thr Tyr Gly Phe Glu Val Phe His
               20                  25                  30
   Ser Glu Val Asn Glu Glu Gln Gly Val Arg Glu Ala Met Leu Lys Ile
           35                  40                  45
   Asn Glu Thr Ser Asp Gly Gly Ala Ser Tyr Leu Gln Leu Leu Glu Pro
       50                  55                  60
   Thr Arg Pro Asp Ser Thr Val Ala Lys Trp Leu Asp Lys Asn Gly Glu
   65                  70                  75                  80
   Gly Val His His Ile Ala Phe Gly Thr Ala Asp Val Asp Gln Asp Ala
                   85                  90                  95
   Ala Asp Ile Lys Asp Lys Gly Val Arg Val Leu Tyr Glu Glu Pro Arg
               100                 105                 110
   Arg Gly Ser Met Gly Ser Arg Ile Thr Phe Leu His Pro Lys Asp Cys
           115                 120                 125
   His Gly Val Leu Thr Glu Leu Val Thr Ser Ala Pro Val Glu Ser Pro
       130                 135                 140
   Glu His
   145
<210> 6
   <211> 146
   <212> PRT
   <213> Streptomyces sviceus
<400> 6 145
<210> 7
   <211> 1773
   <212> DNA
   <213> Streptomyces coelicolor
<300>
   <308> GenBank / AF113603.1
   <309> 1999-12-08
   <313> (1)..(1773)
<400> 7
   gtgcgcaagg tgctcatcgc caatcgtggc gaaatcgctg tccgcgtggc ccgggcctgc 60
   cgggacgccg ggatcgcgag cgtggccgtc tacgcggatc cggaccggga cgcgttgcac 120
   gtccgtgccg ctgatgaggc gttcgccctg ggtggtgaca cccccgcgac cagctatctg 180
   gacatcgcca aggtcctcaa agccgcgcgc gagtcgggcg cggacgccat ccaccccggc 240
   tacggattcc tctcggagaa cgccgagttc gcgcaggcgg tcctggacgc cggcctgatc 300
   tggatcggcc cgcccccgca cgccatccgc gaccgtggcg aaaaggtcgc cgcccgccac 360
   atcgcccagc gggccggcgc ccccctggtc gccggcaccc ccgaccccgt ctccggcgcg 420
   gacgaggtcg tcgccttcgc caaggagcac ggcctgccca tcgccatcaa ggccgccttc 480
   ggcggcggcg ggcgcggcct caaggtcgcc cgcaccctcg aagaggtgcc ggagctgtac 540
   gactccgccg tccgcgaggc cgtggccgcc ttcggccgcg gggagtgctt cgtcgagcgc 600
   tacctcgaca agccccgcca cgtggagacc cagtgcctgg ccgacaccca cggcaacgtg 660
   gtcgtcgtct ccacccgcga ctgctccctc cagcgccgcc accaaaagct cgtcgaggag 720
   gcccccgcgc cctttctctc cgaggcccag acggagcagc tgtactcatc ctccaaggcc 780
   atcctgaagg aggccggcta cggcggcgcc ggcaccgtgg agttcctcgt cggcatggac 840
   ggcacgatct tcttcctgga ggtcaacacc cgcctccagg tcgagcaccc ggtcaccgag 900
   gaagtcgccg gcatcgactt ggtccgcgag atgttccgca tcgccgacgg cgaggaactc 960
   ggttacgacg accccgccct gcgcggccac tccttcgagt tccgcatcaa cggcgaggac 1020
   cccggccgcg gcttcctgcc cgcccccggc accgtcaccc tcttcgacgc gcccaccggc 1080
   cccggcgtcc gcctggacgc cggcgtcgag tccggctccg tcatcggccc cgcctgggac 1140
   tccctcctcg ccaaactgat cgtcaccggc cgcacccgcg ccgaggcact ccagcgcgcg 1200
   gcccgcgccc tggacgagtt caccgtcgag ggcatggcca ccgccatccc cttccaccgc 1260
   acggtcgtcc gcgacccggc cttcgccccc gaactcaccg gctccacgga ccccttcacc 1320
   gtccacaccc ggtggatcga gacggagttc gtcaacgaga tcaagccctt caccacgccc 1380
   gccgacaccg agacggacga ggagtcgggc cgggagacgg tcgtcgtcga ggtcggcggc 1440
   aagcgcctgg aagtctccct cccctccagc ctgggcatgt ccctggcccg caccggcctg 1500
   gccgccgggg cccgccccaa gcgccgcgcg gccaagaagt ccggccccgc cgcctcgggc 1560
   gacaccctcg cctccccgat gcagggcacg atcgtcaaga tcgccgtcga ggaaggccag 1620
   gaagtccagg aaggcgacct catcgtcgta ctcgaggcga tgaagatgga acagcccctc 1680
   aacgcccaca ggtccggcac catcaagggc ctcaccgccg aggtcggcgc ctccctcacc 1740
   tccggcgccg ccatctgcga gatcaaggac tga 1773
<210> 8
   <211> 1593
   <212> DNA
   <213> Streptomyces coelicolor
<300>
   <308> GenBank / AF113605.1
   <309> 1999-12-08
   <313> (1)..(1593)
<400> 8
   atgtccgagc cggaagagca gcagcccgac atccacacga ccgcgggcaa gctcgcggat      60
   ctcaggcgcc gtatcgagga agcgacgcac gccggttccg cacgcgccgt cgagaagcag     120
   cacgccaagg gcaagctgac ggctcgtgaa cgcatcgacc tcctcctcga cgagggttcc     180
   ttcgtcgagc tggacgagtt cgcccggcac cgctccacca acttcggcct cgacgccaac     240
   cgcccctacg gcgacggcgt cgtcaccggc tacggcaccg tcgacggccg ccccgtggcc     300
   gtcttctccc aggacttcac cgtcttcggc ggcgcgctgg gcgaggtcta cggccagaag     360
   atcgtcaagg tgatggactt cgccctcaag accggctgcc cggtcgtcgg catcaacgac     420
   tccggcggcg cccgcatcca ggagggcgtg gcctccctcg gcgcctacgg cgagatcttc     480
   cgccgcaaca cccacgcctc cggcgtgatc ccgcagatca gcctggtcgt cggcccgtgt     540
   gcgggcggcg cggtgtactc ccccgcgatc accgacttca cggtgatggt ggaccagacc     600
   agccacatgt tcatcaccgg tcccgacgtc atcaagacgg tcaccggcga ggacgtcggc     660
   ttcgaggagc tgggcggcgc ccgcacccac aactccacct cgggcgtggc ccaccacatg     720
   gccggcgacg agaaggacgc ggtcgagtac gtcaagcagc tcctgtcgta cctgccgtcc     780
   aacaacctct ccgagccccc cgccttcccg gaggaggcgg acctcgcggt cacggacgag     840
   gacgccgagc tggacacgat cgtcccggac tcggcgaacc agccctacga catgcactcc     900
   gtcatcgagc acgtcctgga cgacgccgag ttcttcgaga cgcaacccct cttcgcgccg     960
   aacatcctca ccggcttcgg ccgcgtggag ggccgcccgg tcggcatcgt cgccaaccag    1020
   cccatgcagt tcgccggctg cctggacatc acggcctccg agaaggcggc ccgcttcgtg    1080
   cgcacctgcg acgccttcaa cgtccccgtc ctcaccttcg tggacgtccc cggcttcctg    1140
   cccggcgtcg accaggagca cgacggcatc atccgccgcg gcgccaagct gatcttcgcc    1200
   tacgccgagg ccacggtgcc gctcatcacg gtcatcaccc gcaaggcctt cggcggcgcc    1260
   tacgacgtca tgggctccaa gcacctgggc gccgacctca acctggcctg gcccaccgcc    1320
   cagatcgccg tcatgggcgc ccaaggcgcg gtcaacatcc tgcaccgccg caccatcgcc      1380
   gacgccggtg acgacgccga ggccacccgg gcccgcctga tccaggagta cgaggacgcc      1440
   ctcctcaacc cctacacggc ggccgaacgc ggctacgtcg acgccgtgat catgccctcc      1500
   gacactcgcc gccacatcgt ccgcggcctg cgccagctgc gcaccaagcg cgagtccctg      1560
   cccccgaaga agcacggcaa catccccctg taa                                   1593
<210> 9
   <211> 590
   <212> PRT
   <213> Streptomyces coelicolor
<300>
   <308> GenBank / AF113603.1
   <309> 1999-12-08
   <313> (1)..(590)
<400> 9
   Met Arg Lys Val Leu Ile Ala Asn Arg Gly Glu Ile Ala Val Arg Val
   1               5                   10                  15
   Ala Arg Ala Cys Arg Asp Ala Gly Ile Ala Ser Val Ala Val Tyr Ala
               20                  25                  30
   Asp Pro Asp Arg Asp Ala Leu His Val Arg Ala Ala Asp Glu Ala Phe
           35                  40                  45
   Ala Leu Gly Gly Asp Thr Pro Ala Thr Ser Tyr Leu Asp Ile Ala Lys
       50                  55                  60
   Val Leu Lys Ala Ala Arg Glu Ser Gly Ala Asp Ala Ile His Pro Gly
   65                  70                  75                  80
   Tyr Gly Phe Leu Ser Glu Asn Ala Glu Phe Ala Gln Ala Val Leu Asp
                   85                  90                  95
   Ala Gly Leu Ile Trp Ile Gly Pro Pro Pro His Ala Ile Arg Asp Arg
               100                 105                 110
   Gly Glu Lys Val Ala Ala Arg His Ile Ala Gln Arg Ala Gly Ala Pro
           115                 120                 125
   Leu Val Ala Gly Thr Pro Asp Pro Val Ser Gly Ala Asp Glu Val Val
       130                 135                 140
   Ala Phe Ala Lys Glu His Gly Leu Pro Ile Ala Ile Lys Ala Ala Phe
   145                 150                 155                 160
   Gly Gly Gly Gly Arg Gly Leu Lys Val Ala Arg Thr Leu Glu Glu Val
                   165                 170                 175
   Pro Glu Leu Tyr Asp Ser Ala Val Arg Glu Ala Val Ala Ala Phe Gly
                   180                 185                 190
   Arg Gly Glu Cys Phe Val Glu Arg Tyr Leu Asp Lys Pro Arg His Val
               195                 200                 205
   Glu Thr Gln Cys Leu Ala Asp Thr His Gly Asn Val Val Val Val Ser
       210                 215                 220
   Thr Arg Asp Cys Ser Leu Gln Arg Arg His Gln Lys Leu Val Glu Glu
   225                 230                 235                 240
   Ala Pro Ala Pro Phe Leu Ser Glu Ala Gln Thr Glu Gln Leu Tyr Ser
                   245                 250                 255
   Ser Ser Lys Ala Ile Leu Lys Glu Ala Gly Tyr Gly Gly Ala Gly Thr
               260                 265                 270
   Val Glu Phe Leu Val Gly Met Asp Gly Thr Ile Phe Phe Leu Glu Val
           275                 280                 285
   Asn Thr Arg Leu Gln Val Glu His Pro Val Thr Glu Glu Val Ala Gly
       290                 295                 300
   Ile Asp Leu Val Arg Glu Met Phe Arg Ile Ala Asp Gly Glu Glu Leu
   305                 310                 315                 320
   Gly Tyr Asp Asp Pro Ala Leu Arg Gly His Ser Phe Glu Phe Arg Ile
                   325                 330                 335
   Asn Gly Glu Asp Pro Gly Arg Gly Phe Leu Pro Ala Pro Gly Thr Val
               340                 345                 350
   Thr Leu Phe Asp Ala Pro Thr Gly Pro Gly Val Arg Leu Asp Ala Gly
           355                 360                 365
   Val Glu Ser Gly Ser Val Ile Gly Pro Ala Trp Asp Ser Leu Leu Ala
       370                 375                 380
   Lys Leu Ile Val Thr Gly Arg Thr Arg Ala Glu Ala Leu Gln Arg Ala
   385                 390                 395                 400
   Ala Arg Ala Leu Asp Glu Phe Thr Val Glu Gly Met Ala Thr Ala Ile
                   405                 410                 415
   Pro Phe His Arg Thr Val Val Arg Asp Pro Ala Phe Ala Pro Glu Leu
               420                 425                 430
   Thr Gly Ser Thr Asp Pro Phe Thr Val His Thr Arg Trp Ile Glu Thr
           435                 440                 445
   Glu Phe Val Asn Glu Ile Lys Pro Phe Thr Thr Pro Ala Asp Thr Glu
       450                 455                 460
   Thr Asp Glu Glu Ser Gly Arg Glu Thr Val Val Val Glu Val Gly Gly
   465                 470                 475                 480
   Lys Arg Leu Glu Val Ser Leu Pro Ser Ser Leu Gly Met Ser Leu Ala
                   485                 490                 495
   Arg Thr Gly Leu Ala Ala Gly Ala Arg Pro Lys Arg Arg Ala Ala Lys
               500                 505                 510
   Lys Ser Gly Pro Ala Ala Ser Gly Asp Thr Leu Ala Ser Pro Met Gln
           515                 520                 525
   Gly Thr Ile Val Lys Ile Ala Val Glu Glu Gly Gln Glu Val Gln Glu
       530                 535                 540
   Gly Asp Leu Ile Val Val Leu Glu Ala Met Lys Met Glu Gln Pro Leu
   545                 550                 555                 560
   Asn Ala His Arg Ser Gly Thr Ile Lys Gly Leu Thr Ala Glu Val Gly
                   565                 570                 575
   Ala Ser Leu Thr Ser Gly Ala Ala Ile Cys Glu Ile Lys Asp
               580                 585                 590
<210> 10
   <211> 530
   <212> PRT
   <213> Streptomyces coelicolor
<300>
   <308> GenBank / AF113605.1
   <309> 1999-12-08
   <313> (1)..(530)
<400> 10
   Met Ser Glu Pro Glu Glu Gln Gln Pro Asp Ile His Thr Thr Ala Gly
   1               5                   10                  15
   Lys Leu Ala Asp Leu Arg Arg Arg Ile Glu Glu Ala Thr His Ala Gly
               20                  25                  30
   Ser Ala Arg Ala Val Glu Lys Gln His Ala Lys Gly Lys Leu Thr Ala
           35                  40                  45
   Arg Glu Arg Ile Asp Leu Leu Leu Asp Glu Gly Ser Phe Val Glu Leu
   50 55 60
   Asp Glu Phe Ala Arg His Arg Ser Thr Asn Phe Gly Leu Asp Ala Asn
   65 70 75 80
   Arg Pro Tyr Gly Asp Gly Val Val Thr Gly Tyr Gly Thr Val Asp Gly
   85 90 95
   Arg Pro Val Ala Val Phe Ser Gln Asp Phe Thr Val Phe Gly Gly Ala
   100 105 110
   Leu Gly Glu Val Tyr Gly Gln Lys Ile Val Lys Val Met Asp Phe Ala
   115 120 125
   Leu Lys Thr Gly Cys Pro Val Val Gly Ile Asn Asp Ser Gly Gly Ala
   130 135 140
   Arg Ile Gln Glu Gly Val Ala Ser Leu Gly Ala Tyr Gly Glu Ile Phe
   145 150 155 160
   Arg Arg Asn Thr His Ala Ser Gly Val Ile Pro Gln Ile Ser Leu Val
   165 170 175
   Val Gly Pro Cys Ala Gly Gly Ala Val Tyr Ser Pro Ala Ile Thr Asp
   180 185 190
   Phe Thr Val Met Val Asp Gln Thr Ser His Met Phe Ile Thr Gly Pro
   195 200 205
   Asp Val Ile Lys Thr Val Thr Gly Glu Asp Val Gly Phe Glu Glu Leu
   210 215 220
   Gly Gly Ala Arg Thr His Asn Ser Thr Ser Gly Val Ala His His Met
   225 230 235 240
   Ala Gly Asp Glu Lys Asp Ala Val Glu Tyr Val Lys Gln Leu Leu Ser
   245 250 255
   Tyr Leu Pro Ser Asn Asn Leu Ser Glu Pro Pro Ala Phe Pro Glu Glu
   260 265 270
   Ala Asp Leu Ala Val Thr Asp Glu Asp Ala Glu Leu Asp Thr Ile Val
   275 280 285
   Pro Asp Ser Ala Asn Gln Pro Tyr Asp Met His Ser Val Ile Glu His
   290 295 300
   Val Leu Asp Asp Ala Glu Phe Phe Glu Thr Gln Pro Leu Phe Ala Pro
   305 310 315 320
   Asn Ile Leu Thr Gly Phe Gly Arg Val Glu Gly Arg Pro Val Gly Ile
   325 330 335
   Val Ala Asn Gln Pro Met Gln Phe Ala Gly Cys Leu Asp Ile Thr Ala
   340 345 350
   Ser Glu Lys Ala Ala Arg Phe Val Arg Thr Cys Asp Ala Phe Asn Val
   355 360 365
   Pro Val Leu Thr Phe Val Asp Val Pro Gly Phe Leu Pro Gly Val Asp
   370 375 380
   Gln Glu His Asp Gly Ile Ile Arg Arg Gly Ala Lys Leu Ile Phe Ala
   385 390 395 400
   Tyr Ala Glu Ala Thr Val Pro Leu Ile Thr Val Ile Thr Arg Lys Ala
   405 410 415
   Phe Gly Gly Ala Tyr Asp Val Met Gly Ser Lys His Leu Gly Ala Asp
   420 425 430
   Leu Asn Leu Ala Trp Pro Thr Ala Gln Ile Ala Val Met Gly Ala Gln
   435 440 445
   Gly Ala Val Asn Ile Leu His Arg Arg Thr Ile Ala Asp Ala Gly Asp
   450 455 460
   Asp Ala Glu Ala Thr Arg Ala Arg Leu Ile Gln Glu Tyr Glu Asp Ala
   465 470 475 480
   Leu Leu Asn Pro Tyr Thr Ala Ala Glu Arg Gly Tyr Val Asp Ala Val
   485 490 495
   Ile Met Pro Ser Asp Thr Arg Arg His Ile Val Arg Gly Leu Arg Gln
   500 505 510
   Leu Arg Thr Lys Arg Glu Ser Leu Pro Pro Lys Lys His Gly Asn Ile
   515 520 525
   Pro Leu
   530
<210> 11
   <211> 116
   <212> DNA
   <213> Streptomyces coelicolor
<400> 11
   aattgtgagc ggataacaat tgacattgtg agcggataac aagatactga gcacatcagc 60
   aggacgcact gaccgaattc aataattttg tttaacttta agaaggagat atacat 116
<210> 12
   <211> 1773
   <212> DNA
   <213> Streptomyces coelicolor
<400> 12
   atgcgcaaag tgctgattgc gaaccgtggt gaaatcgccg ttcgtgtggc acgcgcgtgt 60
   cgtgatgcag gtattgcaag tgttgcggtg tatgccgatc cggatcgcga tgcgctgcat 120
   gttcgtgcgg ccgatgaagc ctttgcactg ggcggtgata ccccggcaac gagctatctg 180
   gatattgcaa aagtgctgaa agcagcgcgc gaaagcggtg cggatgccat ccatccgggc 240
   tacggttttc tgtctgaaaa tgcagaattt gcacaggcgg ttctggatgc aggtctgatt 300
   tggatcggtc cgccgccgca tgcaattcgt gatctgggcg ataaagtggc cgcacgccac 360
   atcgcccagc gtgcaggcgc gccgctggtt gcgggcaccc cggacccggt ttctggtgca 420
   gatgaagtgg ttgcgtttgc caaagaacat ggcctgccga ttgcgatcaa agcagcattc 480
   ggcggtggcg gtcgcggtct gaaagtggcc cgtaccctgg aagaagttcc ggaactgtat 540
   gatagcgcag ttcgcgaagc ggtggcagcg tttggccgtg gtgaatgctt cgtggaacgc 600
   tacctggata aaccgcgtca tgttgaaacc cagtgtctgg cggatacgca cggcaacgtg 660
   gttgtggtta gcacccgcga ttgctctctg caacgtcgcc accagaaact ggtggaagaa 720
   gcaccggcgc cgtttctgag cgaagcccag accgaacagc tgtatagctc tagtaaagcg 780
   attctgaaag aagccggtta cgtgggcgcc ggtacggttg aatttctggt gggcatggat 840
   ggcaccatta gctttctgga agttaacacc cgtctgcaag ttgaacatcc ggtgaccgaa 900
   gaagttgcgg gcattgatct ggtgcgcgaa atgtttcgta tcgcagatgg cgaagaactg 960
   ggttacgatg atccggcgct gcgcggtcac agctttgaat ttcgtattaa tggcgaagat 1020
   ccgggccgtg gttttctgcc ggcgccgggc accgtgacgc tgttcgatgc accgaccggt 1080
   ccgggcgttc gtctggatgc cggtgtggaa agtggtagcg ttattggccc ggcatgggat 1140
   agcctgctgg cgaaactgat cgttaccggt cgtacgcgcg ccgaagcgct gcaacgtgca 1200
   gcacgtgccc tggatgaatt taccgtggaa ggcatggcga cggccattcc gtttcatcgc 1260
   accgtggttc gtgatccggc attcgcgccg gaactgaccg gctctaccga tccgttcacc 1320
   gtgcacacgc gctggatcga aaccgaattt gttaacgaaa tcaaaccgtt caccacgccg 1380
   gcggataccg aaacggatga agaaagtggt cgcgaaacgg tggttgtgga agtgggcggt 1440
   aaacgtctgg aagtttctct gccgagcagc ctgggtatga gtctggcgcg taccggtctg 1500
   gcggccggcg cccgtccgaa acgtcgcgca gcgaaaaaat ctggtccggc cgcaagcggt 1560
   gataccctgg ccagtccgat gcagggcacg attgtgaaaa tcgcagtgga agaaggtcag 1620
   gaagtgcagg aaggcgatct gattgttgtg ctggaagcga tgaaaatgga acagccgctg 1680
   aatgcccatc gtagcggcac catcaaaggc ctgacggccg aagtgggtgc atctctgacc 1740
   agtggcgcgg ccatttgcga aatcaaagat taa 1773
<210> 13
   <211> 57
   <212> DNA
   <213> Streptomyces coelicolor
<400> 13
   agatctgcgg ccgcatctag aaataatttt gtttaacttt aagaaggaga tatattc 57
<210> 14
   <211> 1593
   <212> DNA
   <213> Streptomyces coelicolor
<400> 14
   atgagtgaac cggaagaaca gcagccggat attcatacca cggcaggcaa actggcggat     60
   ctgcgtcgcc gtatcgaaga agcaacccat gcaggtagcg cacgtgcagt ggaaaaacag    120
   cacgcgaaag gtaaactgac ggcccgcgaa cgtatcgatc tgctgctgga tgaaggcagt    180
   tttgttgaac tggatgaatt tgcacgccac cgtagcacca actttggtct ggatgcgaat    240
   cgcccgtatg gcgatggtgt ggttaccggt tacggtacgg tggatggtcg tccggtggca    300
   gtttttagcc aggattttac cgtgttcggc ggtgcactgg gcgaagttta cggtcagaaa    360
   atcgtgaaag ttatggattt cgcgctgaaa acgggctgcc cggtggttgg tattaacgat    420
   agcggcggtg cccgcatcca ggaaggtgtt gcctctctgg gcgcgtatgg cgaaatcttt    480
   cgccgtaata cccatgcgag tggcgtgatt ccgcagatca gcctggtggt tggtccgtgt    540
   gcgggcggtg ccgtttactc tccggccatt accgatttta cggtgatggt tgatcagacc    600
   agtcacatgt tcattacggg cccggatgtg atcaaaaccg ttacgggcga agatgtgggt    660
   tttgaagaac tgggcggtgc acgtacccac aacagcacgt ctggcgttgc gcatcacatg    720
   gccggtgatg aaaaagatgc cgtggaatat gttaaacagc tgctgagtta cctgccgagc    780
   aacaatctgt ctgaaccgcc ggcgttcccg gaagaagcag acctggcggt gaccgatgaa    840
   gatgccgaac tggatacgat cgttccggat tctgcaaatc agccgtacga tatgcacagt    900
   gtgattgaac acgttctgga tgatgcggaa tttttcgaaa cccagccgct gtttgccccg    960
   aacattctga cgggtttcgg tcgtgtggaa ggtcgtccgg tgggtatcgt tgcaaatcag   1020
   ccgatgcagt ttgcgggttg cctggatatt accgcctctg aaaaagcggc ccgctttgtg   1080
   cgtacctgtg atgcgttcaa cgtgccggtt ctgacgtttg tggatgttcc gggcttcctg   1140
   ccgggtgttg atcaggaaca tgatggcatt atccgccgtg gtgcgaaact gatttttgcg   1200
   tatgccgaag caaccgtgcc gctgattacc gttatcacgc gcaaagcatt cggcggtgcg   1260
   tacgatgtga tgggcagcaa acatctgggt gccgatctga acctggcatg gccgaccgca   1320
   cagatcgcag tgatgggcgc gcagggtgcc gttaatattc tgcaccgccg taccatcgca 1380
   gatgcaggtg atgatgcaga agcgacgcgc gcacgtctga ttcaggaata tgaagatgcg 1440
   ctgctgaacc cgtataccgc agcggaacgt ggttacgtgg atgcggttat tatgccgagc 1500
   gatacccgcc gtcatatcgt gcgtggtctg cgtcagctgc gtacgaaacg tgaatctctg 1560
   ccgccgaaaa aacacggtaa tattccgctg taa 1593
<210> 15
   <211> 3539
   <212> DNA
   <213> Artificial Sequence
<220>
   <223> Synthetic nucleotide
<400> 15
   aattgtgagc ggataacaat tgacattgtg agcggataac aagatactga gcacatcagc 60
   aggacgcact gaccgaattc aataattttg tttaacttta agaaggagat atacatatgc 120
   gcaaagtgct gattgcgaac cgtggtgaaa tcgccgttcg tgtggcacgc gcgtgtcgtg 180
   atgcaggtat tgcaagtgtt gcggtgtatg ccgatccgga tcgcgatgcg ctgcatgttc 240
   gtgcggccga tgaagccttt gcactgggcg gtgatacccc ggcaacgagc tatctggata 300
   ttgcaaaagt gctgaaagca gcgcgcgaaa gcggtgcgga tgccatccat ccgggctacg 360
   gttttctgtc tgaaaatgca gaatttgcac aggcggttct ggatgcaggt ctgatttgga 420
   tcggtccgcc gccgcatgca attcgtgatc tgggcgataa agtggccgca cgccacatcg 480
   cccagcgtgc aggcgcgccg ctggttgcgg gcaccccgga cccggtttct ggtgcagatg 540
   aagtggttgc gtttgccaaa gaacatggcc tgccgattgc gatcaaagca gcattcggcg 600
   gtggcggtcg cggtctgaaa gtggcccgta ccctggaaga agttccggaa ctgtatgata 660
   gcgcagttcg cgaagcggtg gcagcgtttg gccgtggtga atgcttcgtg gaacgctacc 720
   tggataaacc gcgtcatgtt gaaacccagt gtctggcgga tacgcacggc aacgtggttg 780
   tggttagcac ccgcgattgc tctctgcaac gtcgccacca gaaactggtg gaagaagcac 840
   cggcgccgtt tctgagcgaa gcccagaccg aacagctgta tagctctagt aaagcgattc 900
   tgaaagaagc cggttacgtg ggcgccggta cggttgaatt tctggtgggc atggatggca 960
   ccattagctt tctggaagtt aacacccgtc tgcaagttga acatccggtg accgaagaag 1020
   ttgcgggcat tgatctggtg cgcgaaatgt ttcgtatcgc agatggcgaa gaactgggtt 1080
   acgatgatcc ggcgctgcgc ggtcacagct ttgaatttcg tattaatggc gaagatccgg 1140
   gccgtggttt tctgccggcg ccgggcaccg tgacgctgtt cgatgcaccg accggtccgg 1200
   gcgttcgtct ggatgccggt gtggaaagtg gtagcgttat tggcccggca tgggatagcc 1260
   tgctggcgaa actgatcgtt accggtcgta cgcgcgccga agcgctgcaa cgtgcagcac 1320
   gtgccctgga tgaatttacc gtggaaggca tggcgacggc cattccgttt catcgcaccg 1380
   tggttcgtga tccggcattc gcgccggaac tgaccggctc taccgatccg ttcaccgtgc 1440
   acacgcgctg gatcgaaacc gaatttgtta acgaaatcaa accgttcacc acgccggcgg 1500
   ataccgaaac ggatgaagaa agtggtcgcg aaacggtggt tgtggaagtg ggcggtaaac 1560
   gtctggaagt ttctctgccg agcagcctgg gtatgagtct ggcgcgtacc ggtctggcgg 1620
   ccggcgcccg tccgaaacgt cgcgcagcga aaaaatctgg tccggccgca agcggtgata 1680
   ccctggccag tccgatgcag ggcacgattg tgaaaatcgc agtggaagaa ggtcaggaag 1740
   tgcaggaagg cgatctgatt gttgtgctgg aagcgatgaa aatggaacag ccgctgaatg 1800
   cccatcgtag cggcaccatc aaaggcctga cggccgaagt gggtgcatct ctgaccagtg 1860
   gcgcggccat ttgcgaaatc aaagattaaa gatctgcggc cgcatctaga aataattttg 1920
   tttaacttta agaaggagat atattcatga gtgaaccgga agaacagcag ccggatattc 1980
   ataccacggc aggcaaactg gcggatctgc gtcgccgtat cgaagaagca acccatgcag 2040
   gtagcgcacg tgcagtggaa aaacagcacg cgaaaggtaa actgacggcc cgcgaacgta 2100
   tcgatctgct gctggatgaa ggcagttttg ttgaactgga tgaatttgca cgccaccgta 2160
   gcaccaactt tggtctggat gcgaatcgcc cgtatggcga tggtgtggtt accggttacg 2220
   gtacggtgga tggtcgtccg gtggcagttt ttagccagga ttttaccgtg ttcggcggtg 2280
   cactgggcga agtttacggt cagaaaatcg tgaaagttat ggatttcgcg ctgaaaacgg 2340
   gctgcccggt ggttggtatt aacgatagcg gcggtgcccg catccaggaa ggtgttgcct 2400
   ctctgggcgc gtatggcgaa atctttcgcc gtaataccca tgcgagtggc gtgattccgc 2460
   agatcagcct ggtggttggt ccgtgtgcgg gcggtgccgt ttactctccg gccattaccg 2520
   attttacggt gatggttgat cagaccagtc acatgttcat tacgggcccg gatgtgatca 2580
   aaaccgttac gggcgaagat gtgggttttg aagaactggg cggtgcacgt acccacaaca 2640
   gcacgtctgg cgttgcgcat cacatggccg gtgatgaaaa agatgccgtg gaatatgtta 2700
   aacagctgct gagttacctg ccgagcaaca atctgtctga accgccggcg ttcccggaag 2760
   aagcagacct ggcggtgacc gatgaagatg ccgaactgga tacgatcgtt ccggattctg 2820
   caaatcagcc gtacgatatg cacagtgtga ttgaacacgt tctggatgat gcggaatttt 2880
   tcgaaaccca gccgctgttt gccccgaaca ttctgacggg tttcggtcgt gtggaaggtc 2940
   gtccggtggg tatcgttgca aatcagccga tgcagtttgc gggttgcctg gatattaccg 3000
   cctctgaaaa agcggcccgc tttgtgcgta cctgtgatgc gttcaacgtg ccggttctga 3060
   cgtttgtgga tgttccgggc ttcctgccgg gtgttgatca ggaacatgat ggcattatcc 3120
   gccgtggtgc gaaactgatt tttgcgtatg ccgaagcaac cgtgccgctg attaccgtta 3180
   tcacgcgcaa agcattcggc ggtgcgtacg atgtgatggg cagcaaacat ctgggtgccg 3240
   atctgaacct ggcatggccg accgcacaga tcgcagtgat gggcgcgcag ggtgccgtta 3300
   atattctgca ccgccgtacc atcgcagatg caggtgatga tgcagaagcg acgcgcgcac 3360
   gtctgattca ggaatatgaa gatgcgctgc tgaacccgta taccgcagcg gaacgtggtt 3420
   acgtggatgc ggttattatg ccgagcgata cccgccgtca tatcgtgcgt ggtctgcgtc 3480
   agctgcgtac gaaacgtgaa tctctgccgc cgaaaaaaca cggtaatatt ccgctgtaa 3539
<210> 16
   <211> 45
   <212> DNA
   <213> Artificial Sequence
<220>
   <223> Synthetic primer
<400> 16
   aaactgcaga ggaggacagc tatgtctttt agcgaatttt atcag 45
<210> 17
   <211> 36
   <212> DNA
   <213> Artificial Sequence
<220>
   <223> Synthetic primer
<400> 17
   aaaggatccc tattcttcga tcgcctggcg aatttg 36
<210> 18
   <211> 383
   <212> PRT
   <213> Mycobacterium bovis
<400> 18
   Leu Val Glu Gly Leu Arg Glu Val Ala Asp Gly Asp Ala Leu Tyr Asp
   1               5                   10                  15
   Ala Ala Val Gly His Gly Asp Arg Gly Pro Val Trp Val Phe Ser Gly
               20                  25                  30
   Gln Gly Ser Gln Trp Ala Ala Met Gly Thr Gln Leu Leu Ala Ser Glu
           35                  40                  45
   Pro Val Phe Ala Ala Thr Ile Ala Lys Leu Glu Pro Val Ile Ala Ala
       50                  55                  60
   Glu Ser Gly Phe Ser Val Thr Glu Ala Ile Thr Ala Gln Gln Thr Val
   65                  70                  75                  80
   Thr Gly Ile Asp Lys Val Gln Pro Ala Val Phe Ala Val Gln Val Ala
                   85                  90                  95
   Leu Ala Ala Thr Met Glu Gln Thr Tyr Gly Val Arg Pro Gly Ala Val
               100                 105                 110
   Val Gly His Ser Met Gly Glu Ser Ala Ala Ala Val Val Ala Gly Ala
           115                 120                 125
   Leu Ser Leu Glu Asp Ala Ala Arg Val Ile Cys Arg Arg Ser Lys Leu
       130                 135                 140
   Met Thr Arg Ile Ala Gly Ala Gly Ala Met Gly Ser Val Glu Leu Pro
   145                 150                 155                 160
   Ala Lys Gln Val Asn Ser Glu Leu Met Ala Arg Gly Ile Asp Asp Val
                   165                 170                 175
   Val Val Ser Val Val Ala Ser Pro Gln Ser Thr Val Ile Gly Gly Thr
               180                 185                 190
   Ser Asp Thr Val Arg Asp Leu Ile Ala Arg Trp Glu Gln Arg Asp Val
           195                 200                 205
   Met Ala Arg Glu Val Ala Val Asp Val Ala Ser His Ser Pro Gln Val
       210                 215                 220
   Asp Pro Ile Leu Asp Asp Leu Ala Ala Ala Leu Ala Asp Ile Ala Pro
   225                 230                 235                 240
   Met Thr Pro Lys Val Pro Tyr Tyr Ser Ala Thr Leu Phe Asp Pro Arg
                   245                 250                 255
   Glu Gln Pro Val Cys Asp Gly Ala Tyr Trp Val Asp Asn Leu Arg Asn
               260                 265                 270
   Thr Val Gln Phe Ala Ala Ala Val Gln Ala Ala Met Glu Asp Gly Tyr
           275                 280                 285
   Arg Val Phe Ala Glu Leu Ser Pro His Pro Leu Leu Thr His Ala Val
       290                 295                 300
   Glu Gln Thr Gly Arg Ser Leu Asp Met Ser Val Ala Ala Leu Ala Gly
   305                 310                 315                 320
   Met Arg Arg Glu Gln Pro Leu Pro His Gly Leu Arg Gly Leu Leu Thr
                   325                 330                 335
   Glu Leu His Arg Ala Gly Ala Ala Leu Asp Tyr Ser Ala Leu Tyr Pro
               340                 345                 350
   Ala Gly Arg Leu Val Asp Ala Pro Leu Pro Ala Trp Thr His Ala Arg
   355 360 365
   Leu Phe Ile Asp Asp Asp Gly Gln Glu Gln Arg Ala Gln Gly Ala
   370 375 380
<210> 19
   <211> 2111
   <212> PRT
   <213> Mycobacterium bovis
<300>
   <308> GenBank / YP_97046
   <309> 2010-12-14
   <313> (1) .. (2111)
<400> 19
   Met Glu Ser Arg Val Thr Pro Val Ala Val Ile Gly Met Gly Cys Arg
   1               5                   10                  15
   Leu Pro Gly Gly Ile Asn Ser Pro Asp Lys Leu Trp Glu Ser Leu Leu
               20                  25                  30
   Arg Gly Asp Asp Leu Val Thr Glu Ile Pro Pro Asp Arg Trp Asp Ala
           35                  40                  45
   Asp Asp Tyr Tyr Asp Pro Glu Pro Gly Val Pro Gly Arg Ser Val Ser
       50                  55                  60
   Arg Trp Gly Gly Phe Leu Asp Asp Val Ala Gly Phe Asp Ala Glu Phe
   65                  70                  75                  80
   Phe Gly Ile Ser Glu Arg Glu Ala Thr Ser Ile Asp Pro Gln Gln Arg
                   85                  90                  95
   Leu Leu Leu Glu Thr Ser Trp Glu Ala Ile Glu His Ala Gly Leu Asp
               100                 105                 110
   Pro Ala Ser Leu Ala Gly Ser Ser Thr Ala Val Phe Thr Gly Leu Thr
           115                 120                 125
   His Glu Asp Tyr Leu Val Leu Thr Thr Thr Ala Gly Gly Leu Ala Ser
       130                 135                 140
   Pro Tyr Val Val Thr Gly Leu Asn Asn Ser Val Ala Ser Gly Arg Ile
   145                 150                 155                 160
   Ala His Thr Leu Gly Leu His Gly Pro Ala Met Thr Phe Asp Thr Ala
                   165                 170                 175
   Cys Ser Ser Gly Leu Met Ala Val His Leu Ala Cys Arg Ser Leu His
               180                 185                 190
   Asp Gly Glu Ala Asp Leu Ala Leu Ala Gly Gly Cys Ala Val Leu Leu
           195                 200                 205
   Glu Pro His Ala Cys Val Ala Ala Ser Ala Gln Gly Met Leu Ser Ser
       210                 215                 220
   Thr Gly Arg Cys His Ser Phe Asp Ala Asp Ala Asp Gly Phe Val Arg
   225                 230                 235                 240
   Ser Glu Gly Cys Ala Met Val Leu Leu Lys Arg Leu Pro Asp Ala Leu
                   245                 250                 255
   Arg Asp Gly Asn Arg Ile Phe Ala Val Val Arg Gly Thr Ala Thr Asn
               260                 265                 270
   Gln Asp Gly Arg Thr Glu Thr Leu Thr Met Pro Ser Glu Asp Ala Gln
           275                 280                 285
   Val Ala Val Tyr Arg Ala Ala Leu Ala Ala Ala Gly Val Gln Pro Glu
       290                 295                 300
   Thr Val Gly Val Val Glu Ala His Gly Thr Gly Thr Pro Ile Gly Asp
   305                 310                 315                 320
   Pro Ile Glu Tyr Arg Ser Leu Ala Arg Val Tyr Gly Ala Gly Thr Pro
                   325                 330                 335
   Cys Ala Leu Gly Ser Ala Lys Ser Asn Met Gly His Ser Thr Ala Ser
               340                 345                 350
   Ala Gly Thr Val Gly Leu Ile Lys Ala Ile Leu Ser Leu Arg His Gly
           355                 360                 365
   Val Val Pro Pro Leu Leu His Phe Asn Arg Leu Pro Asp Glu Leu Ser
       370                 375                 380
   Asp Val Glu Thr Gly Leu Phe Val Pro Gln Ala Val Thr Pro Trp Pro
   385                 390                 395                 400
   Asn Gly Asn Asp His Thr Pro Lys Arg Val Ala Val Ser Ser Phe Gly
                   405                 410                 415
   Met Ser Gly Thr Asn Val His Ala Ile Val Glu Glu Ala Pro Ala Glu
               420                 425                 430
   Ala Ser Ala Pro Glu Ser Ser Pro Gly Asp Ala Glu Val Gly Pro Arg
           435                 440                 445
   Leu Phe Met Leu Ser Ser Thr Ser Ser Asp Ala Leu Arg Gln Thr Ala
   450 455 460
   Arg Gln Leu Ala Thr Trp Val Glu Glu His Gln Asp Cys Val Ala Ala
   465 470 475 480
   Ser Asp Leu Ala Tyr Thr Leu Ala Arg Gly Arg Ala His Arg Pro Val
   485 490 495
   Arg Thr Ala Val Val Ala Ala Asn Leu Pro Glu Leu Val Glu Gly Leu
   500 505 510
   Arg Glu Val Ala Asp Gly Asp Ala Leu Tyr Asp Ala Ala Val Gly His
   515 520 525
   Gly Asp Arg Gly Pro Val Trp Val Phe Ser Gly Gln Gly Ser Gln Trp
   530 535 540
   Ala Ala Met Gly Thr Gln Leu Leu Ala Ser Glu Pro Val Phe Ala Ala
   545 550 555 560
   Thr Ile Ala Lys Leu Glu Pro Val Ile Ala Ala Glu Ser Gly Phe Ser
   565 570 575
   Val Thr Glu Ala Ile Thr Ala Gln Gln Thr Val Thr Gly Ile Asp Lys
   580 585 590
   Val Gln Pro Ala Val Phe Ala Val Gln Val Ala Leu Ala Ala Thr Met
   595 600 605
   Glu Gln Thr Tyr Gly Val Arg Pro Gly Ala Val Val Gly His Ser Met
   610 615 620
   Gly Glu Ser Ala Ala Ala Val Val Ala Gly Ala Leu Ser Leu Glu Asp
   625 630 635 640
   Ala Ala Arg Val Ile Cys Arg Arg Ser Lys Leu Met Thr Arg Ile Ala
   645 650 655
   Gly Ala Gly Ala Met Gly Ser Val Glu Leu Pro Ala Lys Gln Val Asn
   660 665 670
   Ser Glu Leu Met Ala Arg Gly Ile Asp Asp Val Val Val Ser Val Val
   675 680 685
   Ala Ser Pro Gln Ser Thr Val Ile Gly Gly Thr Ser Asp Thr Val Arg
   690 695 700
   Asp Leu Ile Ala Arg Trp Glu Gln Arg Asp Val Met Ala Arg Glu Val
   705 710 715 720
   Ala Val Asp Val Ala Ser His Ser Pro Gln Val Asp Pro Ile Leu Asp
   725 730 735
   Asp Leu Ala Ala Ala Leu Ala Asp Ile Ala Pro Met Thr Pro Lys Val
   740 745 750
   Pro Tyr Tyr Ser Ala Thr Leu Phe Asp Pro Arg Glu Gln Pro Val Cys
   755 760 765
   Asp Gly Ala Tyr Trp Val Asp Asn Leu Arg Asn Thr Val Gln Phe Ala
   770 775 780
   Ala Ala Val Gln Ala Ala Met Glu Asp Gly Tyr Arg Val Phe Ala Glu
   785 790 795 800
   Leu Ser Pro His Pro Leu Leu Thr His Ala Val Glu Gln Thr Gly Arg
   805 810 815
   Ser Leu Asp Met Ser Val Ala Ala Leu Ala Gly Met Arg Arg Glu Gln
   820 825 830
   Pro Leu Pro His Gly Leu Arg Gly Leu Leu Thr Glu Leu His Arg Ala
   835 840 845
   Gly Ala Ala Leu Asp Tyr Ser Ala Leu Tyr Pro Ala Gly Arg Leu Val
   850 855 860
   Asp Ala Pro Leu Pro Ala Trp Thr His Ala Arg Leu Phe Ile Asp Asp
   865 870 875 880
   Asp Gly Gln Glu Gln Arg Ala Gln Gly Ala Cys Thr Ile Thr Val His
   885 890 895
   Pro Leu Leu Gly Ser His Val Arg Leu Thr Glu Glu Pro Glu Arg His
   900 905 910
   Val Trp Gln Gly Asp Val Gly Thr Ser Val Leu Ser Trp Leu Ser Asp
   915 920 925
   His Gln Val His Asn Val Ala Ala Leu Pro Gly Ala Ala Tyr Cys Glu
   930 935 940
   Met Ala Leu Ala Ala Ala Ala Glu Val Phe Gly Glu Ala Ala Glu Val
   945 950 955 960
   Arg Asp Ile Thr Phe Glu Gln Met Leu Leu Leu Asp Glu Gln Thr Pro
   965 970 975
   Ile Asp Ala Val Ala Ser Ile Asp Ala Pro Gly Val Val Asn Phe Thr
   980 985 990
   Val Glu Thr Asn Arg Asp Gly Glu Thr Thr Arg His Ala Thr Ala Ala
   995 1000 1005
   Leu Arg Ala Ala Glu Asp Asp Cys Pro Pro Pro Gly Tyr Asp Ile
   1010 1015 1020
   Thr Ala Leu Leu Gln Ala His Pro His Ala Val Asn Gly Thr Ala
   1025 1030 1035
   Met Arg Glu Ser Phe Ala Glu Arg Gly Val Thr Leu Gly Ala Ala
   1040 1045 1050
   Phe Gly Gly Leu Thr Thr Ala His Thr Ala Glu Ala Gly Ala Ala
   1055 1060 1065
   Thr Val Leu Ala Glu Val Ala Leu Pro Ala Ser Ile Arg Phe Gln
   1070 1075 1080
   Gln Gly Ala Tyr Arg Ile His Pro Ala Leu Leu Asp Ala Cys Phe
   1085 1090 1095
   Gln Ser Val Gly Ala Gly Val Gln Ala Gly Thr Ala Thr Gly Gly
   1100 1105 1110
   Leu Leu Leu Pro Leu Gly Val Arg Ser Leu Arg Ala Tyr Gly Pro
   1115 1120 1125
   Thr Arg Asn Ala Arg Tyr Cys Tyr Thr Arg Leu Thr Lys Ala Phe
   1130 1135 1140
   Asn Asp Gly Thr Arg Gly Gly Glu Ala Asp Leu Asp Val Leu Asp
   1145 1150 1155
   Glu His Gly Thr Val Leu Leu Ala Val Arg Gly Leu Arg Met Gly
   1160 1165 1170
   Thr Gly Thr Ser Glu Arg Asp Glu Arg Asp Arg Leu Val Ser Glu
   1175 1180 1185
   Arg Leu Leu Thr Leu Gly Trp Gln Gln Arg Ala Leu Pro Glu Val
   1190 1195 1200
   Gly Asp Gly Glu Ala Gly Ser Trp Leu Leu Ile Asp Thr Ser Asn
   1205 1210 1215
   Ala Val Asp Thr Pro Asp Met Leu Ala Ser Thr Leu Thr Asp Ala
   1220 1225 1230
   Leu Lys Ser His Gly Pro Gln Gly Thr Glu Cys Ala Ser Leu Ser
   1235 1240 1245
   Trp Ser Val Gln Asp Thr Pro Pro Asn Asp Gln Ala Gly Leu Glu
   1250 1255 1260
   Lys Leu Gly Ser Gln Leu Arg Gly Arg Asp Gly Val Val Ile Val
   1265 1270 1275
   Tyr Gly Pro Arg Val Gly Asp Pro Asp Glu His Ser Leu Leu Ala
   1280 1285 1290
   Gly Arg Glu Gln Val Arg His Leu Val Arg Ile Thr Arg Glu Leu
   1295 1300 1305
   Ala Glu Phe Glu Gly Glu Leu Pro Arg Leu Phe Val Val Thr Arg
   1310 1315 1320
   Gln Ala Gln Ile Val Lys Pro His Asp Ser Gly Glu Arg Ala Asn
   1325 1330 1335
   Leu Glu Gln Ala Gly Leu Arg Gly Leu Leu Arg Val Ile Ser Ser
   1340 1345 1350
   Glu His Pro Met Leu Arg Thr Thr Leu Ile Asp Val Asp Glu His
   1355 1360 1365
   Thr Asp Val Glu Arg Val Ala Gln Gln Leu Leu Ser Gly Ser Glu
   1370 1375 1380
   Glu Asp Glu Thr Ala Trp Arg Asn Gly Asp Trp Tyr Val Ala Arg
   1385 1390 1395
   Leu Thr Pro Ser Pro Leu Gly His Glu Glu Arg Arg Thr Ala Val
   1400 1405 1410
   Leu Asp Pro Asp His Asp Gly Met Arg Val Gln Val Arg Arg Pro
   1415 1420 1425
   Gly Asp Leu Gln Thr Leu Glu Phe Val Ala Ser Asp Arg Val Pro
   1430 1435 1440
   Pro Gly Pro Gly Gln Ile Glu Val Ala Val Ser Met Ser Ser Ile
   1445 1450 1455
   Asn Phe Ala Asp Val Leu Ile Ala Phe Gly Arg Phe Pro Ile Ile
   1460 1465 1470
   Asp Asp Arg Glu Pro Gln Leu Gly Met Asp Phe Val Gly Val Val
   1475 1480 1485
   Thr Ala Val Gly Glu Gly Val Thr Gly His Gln Val Gly Asp Arg
   1490 1495 1500
   Val Gly Gly Phe Ser Glu Gly Gly Cys Trp Arg Thr Phe Leu Thr
   1505 1510 1515
   Cys Asp Ala Asn Leu Ala Val Thr Leu Pro Pro Gly Leu Thr Asp
   1520 1525 1530
   Glu Gln Ala Ile Thr Ala Ala Thr Ala His Ala Thr Ala Trp Tyr
   1535 1540 1545
   Gly Leu Asn Asp Leu Ala Gln Ile Lys Ala Gly Asp Lys Val Leu
   1550 1555 1560
   Ile His Ser Ala Thr Gly Gly Val Gly Gln Ala Ala Ile Ser Ile
   1565 1570 1575
   Ala Arg Ala Lys Gly Ala Glu Ile Phe Ala Thr Ala Gly Asn Pro
   1580 1585 1590
   Ala Lys Arg Ala Met Leu Arg Asp Met Gly Val Glu His Val Tyr
   1595 1600 1605
   Asp Ser Arg Ser Val Glu Phe Ala Glu Gln Ile Arg Arg Asp Thr
   1610 1615 1620
   Asp Gly Tyr Gly Val Asp Ile Val Leu Asn Ser Leu Thr Gly Ala
   1625 1630 1635
   Ala Gln Arg Ala Gly Leu Glu Leu Leu Ala Phe Gly Gly Arg Phe
   1640 1645 1650
   Val Glu Ile Gly Lys Ala Asp Val Tyr Gly Asn Thr Arg Leu Gly
   1655 1660 1665
   Leu Phe Pro Phe Arg Arg Gly Leu Thr Phe Tyr Tyr Leu Asp Leu
   1670 1675 1680
   Ala Leu Met Ser Val Thr Gln Pro Asp Arg Val Arg Glu Leu Leu
   1685 1690 1695
   Ala Thr Val Phe Lys Leu Thr Ala Asp Gly Val Leu Thr Ala Pro
   1700 1705 1710
   Gln Cys Thr His Tyr Pro Leu Ala Glu Ala Ala Asp Ala Ile Arg
   1715 1720 1725
   Ala Met Ser Asn Ala Glu His Thr Gly Lys Leu Val Leu Asp Val
   1730 1735 1740
   Pro Arg Ser Gly Arg Arg Ser Val Ala Val Thr Pro Glu Gln Ala
   1745 1750 1755
   Pro Leu Tyr Arg Arg Asp Gly Ser Tyr Ile Ile Thr Gly Gly Leu
   1760 1765 1770
   Gly Gly Leu Gly Leu Phe Phe Ala Ser Lys Leu Ala Ala Ala Gly
   1775 1780 1785
   Cys Gly Arg Ile Val Leu Thr Ala Arg Ser Gln Pro Asn Pro Lys
   1790 1795 1800
   Ala Arg Gln Thr Ile Glu Gly Leu Arg Ala Ala Gly Ala Asp Ile
   1805 1810 1815
   Val Val Glu Cys Gly Asn Ile Ala Glu Pro Asp Thr Ala Asp Arg
   1820 1825 1830
   Leu Val Ser Ala Ala Thr Ala Thr Gly Leu Pro Leu Arg Gly Val
   1835 1840 1845
   Leu His Ser Ala Ala Val Val Glu Asp Ala Thr Leu Thr Asn Ile
   1850 1855 1860
   Thr Asp Glu Leu Ile Asp Arg Asp Trp Ser Pro Lys Val Phe Gly
   1865 1870 1875
   Ser Trp Asn Leu His Arg Ala Thr Leu Gly Gln Pro Leu Asp Trp
   1880 1885 1890
   Phe Cys Leu Phe Ser Ser Gly Ala Ala Leu Leu Gly Ser Pro Gly
   1895 1900 1905
   Gln Gly Ala Tyr Ala Ala Ala Asn Ser Trp Val Asp Val Phe Ala
   1910 1915 1920
   His Trp Arg Arg Ala Gln Gly Leu Pro Val Ser Ala Ile Ala Trp
   1925 1930 1935
   Gly Ala Trp Gly Glu Val Gly Arg Ala Thr Phe Leu Ala Glu Gly
       1940                1945                1950
   Gly Glu  Ile Met Ile Thr Pro  Glu Glu Gly Ala Tyr  Ala Phe Glu
       1955                1960                1965
   Thr Leu  Val Arg His Asp Arg  Ala Tyr Ser Gly Tyr  Ile Pro Ile
       1970                1975                1980
   Leu Gly  Ala Pro Trp Leu Ala  Asp Leu Val Arg Arg  Ser Pro Trp
       1985                1990                1995
   Gly Glu  Met Phe Ala Ser Thr  Gly Gln Arg Ser Arg  Gly Pro Ser
       2000                2005                2010
   Lys Phe  Arg Met Glu Leu Leu  Ser Leu Pro Gln Asp  Glu Trp Ala
       2015                2020                2025
   Gly Arg  Leu Arg Arg Leu Leu  Val Glu Gln Ala Ser  Val Ile Leu
       2030                2035                2040
   Arg Arg  Thr Ile Asp Ala Asp  Arg Ser Phe Ile Glu  Tyr Gly Leu
       2045                2050                2055
   Asp Ser  Leu Gly Met Leu Glu  Met Arg Thr His Val  Glu Thr Glu
       2060                2065                2070
   Thr Gly  Ile Arg Leu Thr Pro  Lys Val Ile Ala Thr  Asn Asn Thr
       2075                2080                2085
   Ala Arg  Ala Leu Ala Gln Tyr  Leu Ala Asp Thr Leu  Ala Glu Glu
       2090                2095                2100
   Gln Ala  Ala Ala Pro Ala Ala  Ser
       2105                2110
<210> 20
   <211> 1149
   <212> DNA
   <213> Mycobacterium bovis
<400> 20
   ctggtggaag gcctgcgtga agttgccgat ggtgatgcac tgtatgatgc agcagtgggt 60
   catggcgatc gtggtccggt ttgggtgttt agcggccagg gttctcagtg ggcagcgatg 120
   ggcacccagc tgctggcaag cgaaccggtt tttgccgcaa cgattgcaaa actggaaccg 180
   gtgatcgcgg ccgaaagtgg cttcagcgtt accgaagcaa ttacggcgca gcagaccgtg 240
   acgggtatcg ataaagtgca gccggccgtt ttcgcagttc aggtggcgct ggcagcgacg 300
   atggaacaga cgtacggcgt tcgtccgggt gcagtggttg gtcacagtat gggtgaaagc 360
   gccgcagcgg tggttgcagg cgccctgagt ctggaagatg ccgcacgtgt gatttgccgt 420
   cgcagcaaac tgatgacccg tatcgcaggt gcaggtgcga tgggcagcgt ggaactgccg 480
   gcaaaacagg ttaactctga actgatggcg cgcggtattg atgatgtggt tgtgtctgtt 540
   gtggcgtctc cgcagagtac cgtgattggc ggcaccagtg atacggttcg tgatctgatc 600
   gcgcgttggg aacagcgcga tgtgatggcg cgcgaagttg ccgtggatgt tgcaagccat 660
   tctccgcagg ttgatccgat tctggatgat ctggcggcgg cactggcaga tattgcaccg 720
   atgaccccga aagtgccgta ttacagcgcg acgctgtttg atccgcgtga acagccggtg 780
   tgtgatggcg cctattgggt tgataacctg cgcaataccg tgcagtttgc ggcggcagtt 840
   caggcggcga tggaagatgg ttaccgtgtg ttcgcggaac tgtctccgca tccgctgctg 900
   acccacgcag tggaacagac gggtcgctct ctggatatga gtgttgcagc actggccggt 960
   atgcgtcgcg aacagccgct gccgcatggc ctgcgtggtc tgctgaccga actgcaccgt 1020
   gcaggtgcag cactggatta tagcgcactg tacccggcag gtcgtctggt ggatgcaccg 1080
   ctgccggcat ggacgcacgc acgtctgttc atcgatgatg atggccagga acagcgcgca 1140
   cagggtgcg 1149
<210> 21
   <211> 1149
   <212> DNA
   <213> Mycobacterium bovis
<400> 21
   ctcgtcgagg gtttgcgcga ggtggccgac ggtgacgccc tctatgacgc ggcggtggga 60
   cacggtgatc gaggaccggt ctgggtcttc tccgggcaag ggtcgcagtg ggcggcgatg 120
   ggcacgcaat tgctcgccag cgaaccagtg ttcgcggcca ccatcgccaa gctggagccg 180
   gtgatcgccg cagaatcggg attctcggtg accgaggcga taacggcgca gcagaccgtg 240
   accggaatcg acaaagtgca gccggcagtg ttcgccgttc aggtcgcgtt ggccgccacc 300
   atggagcaaa cctacggagt gcggccgggc gcggtcgtcg gacactcgat gggtgagtcg 360
   gccgcggccg tcgtcgcggg ggcactgtcg ctcgaggacg cggcgcgcgt catttgccgc 420
   cgctcgaagc tgatgacccg catagccggt gctggtgcca tgggctcggt ggaattgccc 480
   gccaagcaag tgaattcgga gctgatggca cgcggaatcg acgatgttgt ggtctcggtg 540
   gtggcgtccc cgcaatccac ggtgatcggc ggtacgagcg acaccgttcg tgacctcatc 600
   gcccgttggg agcagcggga cgtgatggcg cgcgaggtgg ccgtcgacgt ggcgtcgcac 660
   tcgcctcaag tcgatccgat actcgacgat ttggccgcgg cgctggcgga cattgctccg 720
   atgacgccca aggtgccgta ctactcggcg accctgttcg acccgcgcga gcagccggtg 780
   tgcgatggcg cttactgggt ggacaatctg cgcaacacgg tgcagttcgc cgcggcggtg 840
   caggctgcga tggaggacgg ctaccgggtc ttcgcggagc tgtcgcccca cccgctgctt 900
   acccacgccg tcgaacagac gggccgaagc ctcgacatgt cggtcgccgc cctggccggc 960
   atgcggcgag agcagcctct gccgcatggt ctgcgcggct tgctgacgga gctgcaccgc 1020
   gcgggcgccg ctttggacta ttcggcgctg tatcccgctg ggcggctggt ggatgcgccg 1080
   ctgccggcgt ggacccacgc ccgcctattc atcgacgatg atgggcaaga acagcgggca 1140
   caaggtgcc 1149
<210> 22
   <211> 628
   <212> PRT
   <213> Salmonella enterica
<300>
   <308> GenBank / AAC44817
   <309> 1999-08-05
   <313> (1)..(628)
<400> 22
   Met Ser Phe Ser Glu Phe Tyr Gln Arg Ser Ile Asn Glu Pro Glu Ala
   1               5                   10                  15
   Phe Trp Ala Glu Gln Ala Arg Arg Ile Asp Trp Arg Gln Pro Phe Thr
               20                  25                  30
   Gln Thr Leu Asp His Ser Arg Pro Pro Phe Ala Arg Trp Phe Cys Gly
           35                  40                  45
   Gly Thr Thr Asn Leu Cys His Asn Ala Val Asp Arg Trp Arg Asp Lys
       50                  55                  60
   Gln Pro Glu Ala Leu Ala Leu Ile Ala Val Ser Ser Glu Thr Asp Glu
   65                  70                  75                  80
   Glu Arg Thr Phe Thr Phe Ser Gln Leu His Asp Glu Val Asn Ile Val
                   85                  90                  95
   Ala Ala Met Leu Leu Ser Leu Gly Val Gln Arg Gly Asp Arg Val Leu
               100                 105                 110
   Val Tyr Met Pro Met Ile Ala Glu Ala Gln Ile Thr Leu Leu Ala Cys
           115                 120                 125
   Ala Arg Ile Gly Ala Ile His Ser Val Val Phe Gly Gly Phe Ala Ser
       130                 135                 140
   His Ser Val Ala Ala Arg Ile Asp Asp Ala Arg Pro Ala Leu Ile Val
   145                 150                 155                 160
   Ser Ala Asp Ala Gly Ala Arg Gly Gly Lys Ile Leu Pro Tyr Lys Lys
                   165                 170                 175
   Leu Leu Asp Asp Ala Ile Ala Gln Ala Gln His Gln Pro Lys His Val
   180 185 190
   Leu Leu Val Asp Arg Gly Leu Ala Lys Met Ala Trp Val Asp Gly Arg
   195 200 205
   Asp Leu Asp Phe Ala Thr Leu Arg Gln Gln His Leu Gly Ala Ser Val
   210 215 220
   Pro Val Ala Trp Leu Glu Ser Asn Glu Thr Ser Cys Ile Leu Tyr Thr
   225 230 235 240
   Ser Gly Thr Thr Gly Lys Pro Lys Gly Val Gln Arg Asp Val Gly Gly
   245 250 255
   Tyr Ala Val Ala Leu Ala Thr Ser Met Asp Thr Ile Phe Gly Gly Lys
   260 265 270
   Ala Gly Gly Val Phe Phe Cys Ala Ser Asp Ile Gly Trp Val Val Gly
   275 280 285
   His Ser Tyr Ile Val Tyr Ala Pro Leu Leu Ala Gly Met Ala Thr Ile
   290 295 300
   Val Tyr Glu Gly Leu Pro Thr Tyr Pro Asp Cys Gly Val Trp Trp Lys
   305 310 315 320
   Ile Val Glu Lys Tyr Gln Val Asn Arg Met Phe Ser Ala Pro Thr Ala
   325 330 335
   Ile Arg Val Leu Lys Lys Phe Pro Thr Ala Gln Ile Arg Asn His Asp
   340 345 350
   Leu Ser Ser Leu Glu Ala Leu Tyr Leu Ala Gly Glu Pro Leu Asp Glu
   355 360 365
   Pro Thr Ala Ser Trp Val Thr Glu Thr Leu Gly Val Pro Val Ile Asp
   370 375 380
   Asn Tyr Trp Gln Thr Glu Ser Gly Trp Pro Ile Met Ala Leu Ala Arg
   385 390 395 400
   Ala Leu Asp Asp Arg Pro Ser Arg Leu Gly Ser Pro Gly Val Pro Met
   405 410 415
   Tyr Gly Tyr Asn Val Gln Leu Leu Asn Glu Val Thr Gly Glu Pro Cys
   420 425 430
   Gly Ile Asn Glu Lys Gly Met Leu Val Ile Glu Gly Pro Leu Pro Pro
           435                     440                     445
   Gly Cys Ile Gln Thr Ile Trp Gly Asp Asp Ala Arg Phe Val Lys Thr
       450                     455                     460
   Tyr Trp Ser Leu Phe Asn Arg Gln Val Tyr Ala Thr Phe Asp Trp Gly
   465                     470                     475                     480
   Ile Arg Asp Ala Glu Gly Tyr Tyr Phe Ile Leu Gly Arg Thr Asp Asp
                   485                     490                     495
   Val Ile Asn Ile Ala Gly His Arg Leu Gly Thr Arg Glu Ile Glu Glu
               500                     505                     510
   Ser Ile Ser Ser Tyr Pro Asn Val Ala Glu Val Ala Val Val Gly Ile
           515                     520                     525
   Lys Asp Ala Leu Lys Gly Gln Val Ala Val Ala Phe Val Ile Pro Lys
       530                     535                     540
   Gln Ser Asp Thr Leu Ala Asp Arg Glu Ala Ala Arg Asp Glu Glu Asn
   545                     550                     555                     560
   Ala Ile Met Ala Leu Val Asp Asn Gln Ile Gly His Phe Gly Arg Pro
                   565                     570                     575
   Ala His Val Trp Phe Val Ser Gln Leu Pro Lys Thr Arg Ser Gly Lys
               580                     585                     590
   Met Leu Arg Arg Thr Ile Gln Ala Ile Cys Glu Gly Arg Asp Pro Gly
           595                     600                     605
   Asp Leu Thr Thr Ile Asp Asp Pro Ala Ser Leu Gln Gln Ile Arg Gln
       610                     615                     620
   Ala Ile Glu Glu
   625
<210> 23
   <211> 1884
   <212> DNA
   <213> Salmonella enterica
<400> 23
   atgtctttta gcgaatttta tcagcgttcc attaacgaac cggaggcgtt ctgggccgag 60
   caggcccggc gtatcgactg gcgacagccg tttacgcaga cgctggatca tagccgtcca 120
   ccgtttgccc gctggttttg cggcggcacc actaacttat gtcataacgc cgtcgaccgc 180
   tggcgggata aacagccgga ggcgctggcg ctgattgccg tctcatcaga gaccgatgaa      240
   gagcgcacat ttaccttcag ccagttgcat gatgaagtca acattgtggc cgccatgttg      300
   ctgtcgctgg gcgtgcagcg tggcgatcgc gtattggtct atatgccgat gattgccgaa      360
   gcgcagataa ccctgctggc ctgcgcgcgc attggcgcga tccattcggt ggtctttggc      420
   ggttttgcct cgcacagcgt ggcggcgcgc attgacgatg ccagaccggc gctgattgtg      480
   tcggcggatg ccggagcgcg gggcggtaaa atcctgccgt ataaaaagct gctcgatgac      540
   gctattgcgc aggcgcagca tcagccgaaa cacgttctgc tggtggacag agggctggcg      600
   aaaatggcat gggtggatgg gcgcgatctg gattttgcca cgttgcgcca gcagcatctc      660
   ggcgcgagcg tgccggtggc gtggctggaa tccaacgaaa cctcgtgcat tctttacacc      720
   tccggcacta ccggcaaacc gaaaggcgtc cagcgcgacg tcggcggtta tgcggtggcg      780
   ctggcaacct cgatggacac catttttggc ggcaaggcgg gcggcgtatt cttttgcgca      840
   tcggatatcg gctgggtcgt cggccactcc tatatcgttt acgcgccgtt gctggcaggc      900
   atggcgacta ttgtttacga aggactgccg acgtacccgg actgcggggt ctggtggaaa      960
   attgtcgaga aataccaggt taaccggatg ttttccgccc cgaccgcgat tcgcgtgctg     1020
   aaaaaattcc cgacggcgca aatccgcaat cacgatctct cctcgctgga ggcgctttat     1080
   ctggccggtg agccgctgga cgagccgacg gccagttggg taacggagac gctgggcgta     1140
   ccggtcatcg acaattattg gcagacggag tccggctggc cgatcatggc gctggcccgc     1200
   gcgctggacg acaggccgtc gcgtctggga agtcccggcg tgccgatgta cggttataac     1260
   gtccagctac tcaatgaagt caccggcgaa ccttgcggca taaatgaaaa ggggatgctg     1320
   gtgatcgaag ggccgctgcc gccgggctgt attcagacta tttggggcga cgatgcgcgt     1380
   tttgtgaaga cttactggtc gctgtttaac cgtcaggttt atgccacttt cgactgggga     1440
   atccgcgacg ccgaggggta ttactttatt ctgggccgta ccgatgatgt gattaatatt     1500
   gcgggtcatc ggctggggac gcgagaaata gaagaaagta tctccagcta cccgaacgta     1560
   gcggaagtgg cggtagtggg gataaaagac gctctgaaag ggcaggtagc ggtggcgttt     1620
   gtcattccga agcagagcga tacgctggcg gatcgcgagg cggcgcgcga cgaggaaaac     1680
   gcgattatgg cgctggtgga caaccagatc ggtcactttg gtcgtccggc gcatgtctgg     1740
   tttgtttcgc agctccccaa aacgcgttcc ggaaagatgc ttcgccgcac gatccaggcg     1800
   atctgcgaag gccgcgatcc gggcgatctg acaaccattg acgatcccgc gtcgttgcag     1860
   caaattcgcc aggcgatcga agaa                                            1884
<210> 24
   <211> 616
   <212> PRT
   <213> Streptomyces cinnamonensis
<400> 24
   Met Thr Val Leu Pro Asp Asp Gly Leu Ser Leu Ala Ala Glu Phe Pro
   1               5                   10                  15
   Asp Ala Thr His Glu Gln Trp His Arg Leu Val Glu Gly Val Val Arg
               20                  25                  30
   Lys Ser Gly Lys Asp Val Ser Gly Thr Ala Ala Glu Glu Ala Leu Ser
           35                  40                  45
   Thr Thr Leu Glu Asp Gly Leu Thr Thr Arg Pro Leu Tyr Thr Ala Arg
       50                  55                  60
   Asp Ala Ala Pro Asp Ala Gly Phe Pro Gly Phe Ala Pro Phe Val Arg
   65                  70                  75                  80
   Gly Ser Val Pro Glu Gly Asn Thr Pro Gly Gly Trp Asp Val Arg Gln
                   85                  90                  95
   Arg Tyr Ala Ser Ala Asp Pro Ala Arg Thr Asn Glu Ala Val Leu Thr
               100                 105                 110
   Asp Leu Glu Asn Gly Val Thr Ser Leu Trp Leu Thr Leu Gly Ser Ala
           115                 120                 125
   Gly Leu Pro Val Thr Gly Leu Glu Arg Ala Leu Asp Gly Val Tyr Leu
       130                 135                 140
   Asp Leu Val Pro Val Ala Leu Asp Ala Gly Ser Glu Ala Ala Thr Ala
   145                 150                 155                 160
   Ala Arg Glu Leu Leu Arg Leu Tyr Glu Ala Ala Gly Val Ala Asp Asp
                   165                 170                 175
   Ala Val Arg Gly Thr Leu Gly Ala Asp Pro Leu Gly His Glu Ala Arg
               180                 185                 190
   Thr Gly Glu Lys Ser Thr Ser Phe Ala Ala Val Ala Glu Leu Ala Arg
           195                 200                 205
   Leu Cys Gly Glu Arg Tyr Pro Gly Leu Arg Ala Leu Thr Val Asp Ala
       210                 215                 220
   Leu Pro Tyr His Glu Ala Gly Ala Ser Ala Ala Gln Glu Leu Gly Ala
   225                 230                 235                 240
   Ser Leu Ala Thr Gly Val Glu Tyr Leu Arg Ala Leu His Asp Lys Gly
                   245                 250                 255
   Leu Gly Val Glu Lys Ala Phe Ala Gln Leu Glu Phe Arg Phe Ala Ala
               260                 265                 270
   Thr Ala Asp Gln Phe Leu Thr Ile Ala Lys Leu Arg Ala Ala Arg Arg
           275                 280                 285
   Leu Trp Ala Arg Val Ala Glu Val Ser Gly Val Pro Ala Ala Gly Ala
       290                 295                 300
   Gln Arg Gln His Ala Val Thr Ser Pro Val Met Met Thr Arg Arg Asp
   305                 310                 315                 320
   Pro Trp Val Asn Met Leu Arg Thr Thr Val Ala Cys Leu Gly Ala Gly
                   325                 330                 335
   Val Gly Gly Ala Asp Ala Val Thr Val Leu Pro Phe Asp His Glu Leu
               340                 345                 350
   Gly Leu Pro Asp Ala Phe Ala Arg Arg Ile Ala Arg Asn Thr Ser Thr
           355                 360                 365
   Ile Leu Leu Glu Glu Ser His Leu Ala Arg Val Ile Asp Pro Ala Gly
       370                 375                 380
   Gly Ser Trp Tyr Val Glu Arg Leu Thr Asp Glu Leu Ala His Ala Ala
   385                 390                 395                 400
   Trp Asp Phe Phe Lys Glu Ile Glu Arg Ala Asp Gly Gln Val Ala Ala
                   405                 410                 415
   Leu Arg Ser Gly Leu Val Gly Asp Arg Ile Ala Ala Thr Trp Ala Glu
               420                 425                 430
   Arg Arg Lys Lys Leu Ala Arg Arg Arg Glu Pro Ile Thr Gly Val Ser
           435                 440                 445
   Glu Phe Pro Leu Leu Thr Glu Arg Pro Val Glu Arg Glu Pro Ala Pro
       450                 455                 460
   Ala Ala Pro Pro Gly Gly Leu Pro Arg Val Arg Arg Asp Glu Ala Tyr
   465                 470                 475                 480
   Glu Glu Leu Arg Gly Arg Ser Asp Ala His Leu Glu Ala Thr Gly Ala
                   485                 490                 495
   Arg Pro Lys Val Phe Ile Ala Ala Leu Gly Pro Ala Ala Ala His Thr
               500                 505                 510
   Ala Arg Ala Thr Phe Ala Ala Asn Leu Phe Met Ala Gly Gly Val Glu
               515                 520                 525
   Pro Val His Asp Pro Val Ser Val Asp Ala Glu Thr Ala Ala Glu Ala
       530                 535                 540
   Phe Ala Ala Ser Gly Ala Thr Val Ala Cys Leu Cys Ser Ser Asp Val
   545                 550                 555                 560
   Leu Tyr Ala Glu Gln Ala Glu Ala Val Ala Arg Ala Leu Lys Ser Ala
                   565                 570                 575
   Gly Ala Leu Arg Val Phe Leu Ala Gly Arg Gly Glu Phe Ala Asp Ile
               580                 585                 590
   Asp Glu Tyr Val Phe Ala Gly Cys Asp Ala Val Ala Val Leu Thr Ser
           595                 600                 605
   Thr Leu Asp Arg Met Gly Val Ala
       610                 615
<210> 25
   <211> 733
   <212> PRT
   <213> Streptomyces cinnamonensis
<400> 25
   Met Arg Ile Pro Glu Phe Asp Asp Ile Glu Leu Gly Ala Gly Gly Gly
   1               5                   10                  15
   Pro Ser Gly Ser Ala Glu Gln Trp Arg Ala Ala Val Lys Glu Ser Val
               20                  25                  30
   Gly Lys Ser Glu Ser Asp Leu Leu Trp Glu Thr Pro Glu Gly Ile Ala
           35                  40                  45
   Val Lys Pro Leu Tyr Thr Gly Ala Asp Val Glu Gly Leu Asp Phe Leu
       50                  55                  60
   Glu Thr Tyr Pro Gly Val Ala Pro Tyr Leu Arg Gly Pro Tyr Pro Thr
   65                  70                  75                  80
   Met Tyr Val Asn Gln Pro Trp Thr Ile Arg Gln Tyr Ala Gly Phe Ser
                   85                  90                  95
   Thr Ala Glu Glu Ser Asn Ala Phe Tyr Arg Arg Asn Leu Ala Ala Gly
               100                 105                 110
   Gln Lys Gly Leu Ser Val Ala Phe Asp Leu Pro Thr His Arg Gly Tyr
           115                 120                 125
   Asp Ser Asp His Pro Arg Val Thr Gly Asp Val Gly Met Ala Gly Val
       130                 135                 140
   Ala Ile Asp Ser Ile Tyr Asp Met Arg Gln Leu Phe Asp Gly Ile Pro
   145                 150                 155                 160
   Leu Asp Lys Met Thr Val Ser Met Thr Met Asn Gly Ala Val Leu Pro
                   165                 170                 175
   Val Leu Ala Leu Tyr Ile Val Ala Ala Glu Glu Gln Gly Val Pro Pro
               180                 185                 190
   Glu Lys Leu Ala Gly Thr Ile Gln Asn Asp Ile Leu Lys Glu Phe Met
           195                 200                 205
   Val Arg Asn Thr Tyr Ile Tyr Pro Pro Lys Pro Ser Met Arg Ile Ile
       210                 215                 220
   Ser Asp Ile Phe Ala Tyr Thr Ser Gln Lys Met Pro Arg Tyr Asn Ser
   225                 230                 235                 240
   Ile Ser Ile Ser Gly Tyr His Ile Gln Glu Ala Gly Ala Thr Ala Asp
                   245                 250                 255
   Leu Glu Leu Ala Tyr Thr Leu Ala Asp Gly Val Glu Tyr Leu Arg Ala
               260                 265                 270
   Gly Gln Glu Ala Gly Leu Asp Val Asp Ala Phe Ala Pro Arg Leu Ser
           275                 280                 285
   Phe Phe Trp Ala Ile Gly Met Asn Phe Phe Met Glu Val Ala Lys Leu
       290                 295                 300
   Arg Ala Ala Arg Leu Leu Trp Ala Lys Leu Val Lys Gln Phe Asp Pro
   305                 310                 315                 320
   Lys Asn Ala Lys Ser Leu Ser Leu Arg Thr His Ser Gln Thr Ser Gly
                   325                 330                 335
   Trp Ser Leu Thr Ala Gln Asp Val Phe Asn Asn Val Thr Arg Thr Cys
               340                 345                 350
   Val Glu Ala Met Ala Ala Thr Gln Gly His Thr Gln Ser Leu His Thr
           355                 360                 365
   Asn Ala Leu Asp Glu Ala Leu Ala Leu Pro Thr Asp Phe Ser Ala Arg
       370                 375                 380
   Ile Ala Arg Asn Thr Gln Leu Leu Ile Gln Gln Glu Ser Gly Thr Thr
   385 390 395 400
   Arg Thr Ile Asp Pro Trp Gly Gly Ser Ala Tyr Val Glu Lys Leu Thr
   405 410 415
   Tyr Asp Leu Ala Arg Arg Ala Trp Gln His Ile Glu Glu Val Glu Ala
   420 425 430
   Ala Gly Gly Met Ala Gln Ala Ile Asp Ala Gly Ile Pro Lys Leu Arg
   435 440 445
   Val Glu Glu Ala Ala Ala Arg Thr Gln Ala Arg Ile Asp Ser Gly Arg
   450 455 460
   Gln Pro Val Ile Gly Val Asn Lys Tyr Arg Val Asp Thr Asp Glu Gln
   465 470 475 480
   Ile Asp Val Leu Lys Val Asp Asn Ser Ser Val Arg Ala Gln Gln Ile
   485 490 495
   Glu Lys Leu Arg Arg Leu Arg Glu Glu Arg Asp Asp Ala Ala Cys Gln
   500 505 510
   Asp Ala Leu Arg Ala Leu Thr Ala Ala Ala Glu Arg Gly Pro Gly Gln
   515 520 525
   Gly Leu Glu Gly Asn Leu Leu Ala Leu Ala Val Asp Ala Ala Arg Ala
   530 535 540
   Lys Ala Thr Val Gly Glu Ile Ser Asp Ala Leu Glu Ser Val Tyr Gly
   545 550 555 560
   Arg His Ala Gly Gln Ile Arg Thr Ile Ser Gly Val Tyr Arg Thr Glu
   565 570 575
   Ala Gly Gln Ser Pro Ser Val Glu Arg Thr Arg Ala Leu Val Asp Ala
   580 585 590
   Phe Asp Glu Ala Glu Gly Arg Arg Pro Arg Ile Leu Val Ala Lys Met
   595 600 605
   Gly Gln Asp Gly His Asp Arg Gly Gln Lys Val Ile Ala Ser Ala Phe
   610 615 620
   Ala Asp Leu Gly Phe Asp Val Asp Val Gly Pro Leu Phe Gln Thr Pro
   625 630 635 640
   Ala Glu Val Ala Arg Gln Ala Val Glu Ala Asp Val His Ile Val Gly
   645 650 655
   Val Ser Ser Leu Ala Ala Gly His Leu Thr Leu Val Pro Ala Leu Arg
   660 665 670
   Glu Glu Leu Ala Ala Glu Gly Arg Asp Asp Ile Met Ile Val Val Gly
   675 680 685
   Gly Val Ile Pro Pro Gln Asp Val Glu Ala Leu His Glu Ala Gly Ala
   690 695 700
   Thr Ala Val Phe Pro Pro Gly Thr Val Ile Pro Asp Ala Ala His Asp
   705 710 715 720
   Leu Val Lys Arg Leu Ala Ala Asp Leu Gly His Glu Leu
   725 730
<210> 26
   <211> 146
   <212> PRT
   <213> Streptomyces sviceus
<400> 26
   Met Leu Thr Arg Ile Asp His Ile Gly Ile Ala Cys Phe Asp Leu Asp
   1               5                   10                  15
   Lys Thr Val Glu Phe Tyr Arg Ala Thr Tyr Gly Phe Glu Val Phe His
               20                  25                  30
   Ser Glu Val Asn Glu Glu Gln Gly Val Arg Glu Ala Met Leu Lys Ile
           35                  40                  45
   Asn Glu Thr Ser Asp Gly Gly Ala Ser Tyr Leu Gln Leu Leu Glu Pro
       50                  55                  60
   Thr Arg Pro Asp Ser Thr Val Ala Lys Trp Leu Asp Lys Asn Gly Glu
   65                  70                  75                  80
   Gly Val His His Ile Ala Phe Gly Thr Ala Asp Val Asp Gln Asp Ala
                   85                  90                  95
   Ala Asp Ile Lys Asp Lys Gly Val Arg Val Leu Tyr Glu Glu Pro Arg
               100                 105                 110
   Arg Gly Ser Met Gly Ser Arg Ile Thr Phe Leu His Pro Lys Asp Cys
           115                 120                 125
   His Gly Val Leu Thr Glu Leu Val Thr Ser Ala Pro Val Glu Ser Pro
       130                 135                 140
   Glu His
   145
<210> 27
   <211> 4553
   <212> DNA
   <213> Artificial Sequence
<220>
   <223> Synthetic nucleotide
<400> 27
   gaattcaaaa ttaagaggta tatattaatg accgtgctgc cggatgacgg tctgagtctg 60
   gcagccgaat ttccggatgc gacgcatgaa cagtggcacc gtctggttga aggcgtggtt 120
   cgcaaatcag gcaaagatgt ctcgggcacc gcagctgaag aagccctgag caccacgctg 180
   gaagacggtc tgaccacgcg tccgctgtat acggcacgtg atgcagcacc ggacgctggt 240
   tttccgggtt tcgcgccgtt tgtgcgtggc tcagttccgg agggtaacac cccgggcggt 300
   tgggatgtgc gtcaacgtta cgcatcggca gacccggcac gtaccaacga agcagtgctg 360
   acggatctgg aaaatggtgt taccagcctg tggctgacgc tgggttctgc aggtctgccg 420
   gtgaccggtc tggaacgtgc actggatggt gtttatctgg acctggtccc ggtggcactg 480
   gatgcaggta gcgaagcagc taccgcagca cgtgaactgc tgcgtctgta cgaagcagct 540
   ggtgttgctg atgacgcagt ccgtggcacg ctgggtgcag atccgctggg ccatgaagca 600
   cgcaccggtg aaaaaagtac gtcctttgca gcagtggcag aactggcacg tctgtgcggt 660
   gaacgttatc cgggtctgcg cgctctgacc gttgatgcgc tgccgtacca tgaagctggc 720
   gcgtcagcag ctcaggaact gggcgcttcg ctggcgaccg gtgtggaata tctgcgtgcg 780
   ctgcacgata aaggcctggg tgttgaaaaa gccttcgcac agctggaatt tcgcttcgcg 840
   gccaccgcgg accaatttct gacgattgcc aaactgcgtg cagctcgtcg cctgtgggca 900
   cgtgttgcag aagtcagtgg cgtgccggca gcaggtgcac agcgtcaaca tgcagtcacc 960
   tccccggtga tgatgacgcg tcgcgatccg tgggtgaaca tgctgcgtac cacggttgct 1020
   tgtctgggtg caggtgtcgg cggtgctgat gcagttaccg tcctgccgtt cgatcacgaa 1080
   ctgggtctgc cggacgcctt tgcacgtcgc attgcgcgta ataccagtac gatcctgctg 1140
   gaagaatccc atctggcccg tgtcattgat ccggcaggcg gtagctggta tgtggaacgc 1200
   ctgaccgatg aactggccca cgcagcttgg gactttttca aagaaatcga acgtgcagat 1260
   ggtcaggtcg cagcactgcg tagcggcctg gtgggtgacc gcattgcagc tacctgggca 1320
   gaacgtcgca aaaaactggc gcgtcgccgt gaaccgatca ccggtgtgtc tgaatttccg 1380
   ctgctgacgg aacgcccggt tgaacgtgaa ccggcaccgg cagcaccgcc gggcggtctg 1440
   ccgcgcgtgc gccgtgatga agcctacgaa gaactgcgtg gtcgttctga cgcacacctg 1500
   gaagctaccg gtgcacgtcc gaaagtgttc attgcagctc tgggtccggc agcagcacat 1560
   accgctcgtg cgacgttcgc tgcgaacctg tttatggcgg gcggtgttga accggtccac 1620
   gatcctgtga gcgttgacgc ggaaaccgcc gcagaagcct ttgctgcgtc tggcgccacg 1680
   gttgcatgcc tgtgtagctc tgatgtcctg tatgcggaac aagccgaagc agtcgctcgt 1740
   gcgctgaaaa gtgccggtgc actgcgtgtt ttcctggcag gccgcggtga atttgcggat 1800
   atcgacgaat acgtgtttgc aggttgcgat gctgtcgcag tgctgacctc cacgctggac 1860
   cgtatgggtg ttgcgtaatg cgtattccgg aatttgatga catcgaactg ggtgccggcg 1920
   gtggcccgtc aggttcggca gaacagtggc gtgcagcagt gaaagaaagc gttggtaaaa 1980
   gcgaatctga tctgctgtgg gaaaccccgg aaggcattgc tgttaaaccg ctgtacacgg 2040
   gtgccgatgt cgaaggcctg gacttcctgg aaacctatcc gggtgtcgca ccgtacctgc 2100
   gtggtccgta tccgaccatg tacgtgaacc agccgtggac gatccgccaa tacgcgggtt 2160
   ttagcaccgc cgaagaatct aacgcattct atcgtcgcaa tctggcagct ggccagaaag 2220
   gtctgagtgt ggcgtttgat ctgccgaccc atcgtggcta cgattccgac cacccgcgtg 2280
   tcacgggtga cgtgggtatg gccggcgtgg caattgatag catctatgac atgcgtcagc 2340
   tgttcgatgg tattccgctg gacaaaatga ccgtttctat gacgatgaac ggcgctgtgc 2400
   tgccggttct ggcgctgtat atcgtggcgg ccgaagaaca gggtgttccg ccggaaaaac 2460
   tggcgggcac catccaaaac gatatcctga aagaatttat ggttcgtaac acgtacatct 2520
   acccgccgaa accgagtatg cgcattatct ccgatatctt cgcctatacc tcacagaaaa 2580
   tgccgcgcta caacagtatc tccatctcag gttatcatat ccaagaagca ggcgctaccg 2640
   cggatctgga actggcctac acgctggcag acggtgttga atatctgcgt gctggtcagg 2700
   aagcgggcct ggatgtcgac gcctttgcac cgcgcctgag ctttttctgg gccattggca 2760
   tgaacttttt catggaagtg gcaaaactgc gtgcagctcg cctgctgtgg gcgaaactgg 2820
   ttaaacagtt tgatccgaaa aatgcgaaat cgctgagcct gcgtacccac tcccagacgt 2880
   caggttggtc gctgaccgcc caagatgttt tcaacaatgt cacccgcacg tgcgtggaag 2940
   caatggcagc aacccagggt catacgcaat cactgcacac caacgcgctg gatgaagctc 3000
   tggcgctgcc gaccgacttt tcggctcgta ttgcgcgcaa tacgcagctg ctgatccagc 3060
   aagaaagcgg caccacgcgt accattgatc cgtggggtgg ctctgcgtat gtggaaaaac 3120
   tgacgtacga cctggcacgt cgcgcatggc agcatatcga agaagttgaa gcagcgggtg 3180
   gcatggccca agcaattgat gcgggcatcc cgaaactgcg tgtggaagaa gcggcagcac 3240
   gtacccaggc acgcattgat tctggtcgtc aaccggtcat cggcgtgaac aaatatcgcg 3300
   tggatacgga cgaacagatt gatgttctga aagtcgacaa tagctctgtt cgcgcgcagc 3360
   aaatcgaaaa actgcgtcgc ctgcgtgaag aacgcgatga cgctgcgtgt caggatgctc 3420
   tgcgtgcact gaccgcagca gctgaacgtg gtccgggtca gggtctggaa ggtaatctgc 3480
   tggctctggc agtggatgca gcacgtgcca aagcaaccgt tggcgaaatt tcagacgcac    3540
   tggaatcggt ctacggtcgt catgcgggcc agattcgcac catcagtggt gtgtatcgca    3600
   cggaagcggg ccaatctccg agtgtcgaac gtacccgcgc cctggtggat gcatttgacg    3660
   aagctgaagg tcgtcgcccg cgtattctgg ttgccaaaat gggtcaggat ggccacgacc    3720
   gcggccaaaa agtcatcgct tccgcgtttg ccgatctggg tttcgatgtc gacgtgggtc    3780
   cgctgttcca gaccccggcc gaagtggcac gtcaagctgt ggaagcggat gttcatattg    3840
   ttggtgtcag ttccctggca gctggtcacc tgacgctggt tccggcactg cgtgaagaac    3900
   tggcggccga aggtcgcgat gacattatga tcgtggttgg tggcgtcatt ccgccgcagg    3960
   atgtggaagc cctgcatgaa gcaggtgcta ccgcggtttt tccgccgggc acggtcatcc    4020
   cggatgcagc tcatgacctg gtgaaacgtc tggcagcaga tctgggtcac gaactgtaaa    4080
   agcttaaaat taagaggtat atattaatgc tgacccgcat cgatcacatt ggcatcgcat    4140
   gctttgatct ggataaaacc gtagagttct atcgcgccac ctacggcttt gaggtgtttc    4200
   atagcgaagt aaacgaagaa cagggcgtgc gtgaagccat gctgaaaatc aacgaaacta    4260
   gtgatggtgg ggcgagctat ctgcaactgc tggaaccgac acgcccggac tctacagttg    4320
   ctaagtggct ggacaagaat ggcgaaggcg ttcatcacat tgcgttcggt acggctgatg    4380
   tggatcaaga cgcggcagat attaaagata agggtgtgcg tgttctgtac gaggagccac    4440
   gccgtggtag catgggtagc cgtattacgt tcctgcaccc taaagactgt catggtgtgc    4500
   tgactgagct ggtcacctct gccccggtcg aaagtccgga acattaaggt acc           4553
<210> 28
   <211> 717
   <212> PRT
   <213> Escherichia coli
<400> 28
   Met Ser Arg Met Ser Asn Val Gln Glu Trp Gln Gln Leu Ala Asn Lys
   1               5                   10                  15
   Glu Leu Ser Arg Arg Glu Lys Thr Val Asp Ser Leu Val His Gln Thr
               20                  25                  30
   Ala Glu Gly Ile Ala Ile Lys Pro Leu Tyr Thr Glu Ala Asp Leu Asp
           35                  40                  45
   Asn Leu Glu Val Thr Gly Thr Leu Pro Gly Leu Pro Pro Tyr Val Arg
       50                  55                  60
   Gly Pro Arg Ala Thr Met Tyr Thr Ala Gln Pro Trp Thr Ile Arg Gln
   65                  70                  75                  80
   Tyr Ala Gly Phe Ser Thr Ala Lys Glu Ser Asn Ala Phe Tyr Arg Arg
   85 90 95
   Asn Leu Ala Ala Gly Gln Lys Gly Leu Ser Val Ala Phe Asp Leu Ala
   100 105 110
   Thr His Arg Gly Tyr Asp Ser Asp Asn Pro Arg Val Ala Gly Asp Val
   115 120 125
   Gly Lys Ala Gly Val Ala Ile Asp Thr Val Glu Asp Met Lys Val Leu
   130 135 140
   Phe Asp Gln Ile Pro Leu Asp Lys Met Ser Val Ser Met Thr Met Asn
   145 150 155 160
   Gly Ala Val Leu Pro Val Leu Ala Phe Tyr Ile Val Ala Ala Glu Glu
   165 170 175
   Gln Gly Val Thr Pro Asp Lys Leu Thr Gly Thr Ile Gln Asn Asp Ile
   180 185 190
   Leu Lys Glu Tyr Leu Cys Arg Asn Thr Tyr Ile Tyr Pro Pro Lys Pro
   195 200 205
   Ser Met Arg Ile Ile Ala Asp Ile Ile Ala Trp Cys Ser Gly Asn Met
   210 215 220
   Pro Arg Phe Asn Thr Ile Ser Ile Ser Gly Tyr His Met Gly Glu Ala
   225 230 235 240
   Gly Ala Asn Cys Val Gln Gln Val Ala Phe Thr Leu Ala Asp Gly Ile
   245 250 255
   Glu Tyr Ile Lys Ala Ala Ile Ser Ala Gly Leu Lys Ile Asp Asp Phe
   260 265 270
   Ala Pro Arg Leu Ser Phe Phe Phe Gly Ile Gly Met Asp Leu Phe Met
   275 280 285
   Asn Val Ala Met Leu Arg Ala Ala Arg Tyr Leu Trp Ser Glu Ala Val
   290 295 300
   Ser Gly Phe Gly Ala Gln Asp Pro Lys Ser Leu Ala Leu Arg Thr His
   305 310 315 320
   Cys Gln Thr Ser Gly Trp Ser Leu Thr Glu Gln Asp Pro Tyr Asn Asn
   325 330 335
   Val Ile Arg Thr Thr Ile Glu Ala Leu Ala Ala Thr Leu Gly Gly Thr
   340 345 350
   Gln Ser Leu His Thr Asn Ala Phe Asp Glu Ala Leu Gly Leu Pro Thr
   355 360 365
   Asp Phe Ser Ala Arg Ile Ala Arg Asn Thr Gln Ile Ile Ile Gln Glu
   370 375 380
   Glu Ser Glu Leu Cys Arg Thr Val Asp Pro Leu Ala Gly Ser Tyr Tyr
   385 390 395 400
   Ile Glu Ser Leu Thr Asp Gln Ile Val Lys Gln Ala Arg Ala Ile Ile
   405 410 415
   Gln Gln Ile Asp Glu Ala Gly Gly Met Ala Lys Ala Ile Glu Ala Gly
   420 425 430
   Leu Pro Lys Arg Met Ile Glu Glu Ala Ser Ala Arg Glu Gln Ser Leu
   435 440 445
   Ile Asp Gln Gly Lys Arg Val Ile Val Gly Val Asn Lys Tyr Lys Leu
   450 455 460
   Asp His Glu Asp Glu Thr Asp Val Leu Glu Ile Asp Asn Val Met Val
   465 470 475 480
   Arg Asn Glu Gln Ile Ala Ser Leu Glu Arg Ile Arg Ala Thr Arg Asp
   485 490 495
   Asp Ala Ala Val Thr Ala Ala Leu Asn Ala Leu Thr His Ala Ala Gln
   500 505 510
   His Asn Glu Asn Leu Leu Ala Ala Ala Val Asn Ala Ala Arg Val Arg
   515 520 525
   Ala Thr Leu Gly Glu Ile Ser Asp Ala Leu Glu Val Ala Phe Asp Arg
   530 535 540
   Tyr Leu Val Pro Ser Gln Cys Val Thr Gly Val Ile Ala Gln Ser Tyr
   545 550 555 560
   His Gln Ser Glu Lys Ser Ala Ser Glu Phe Asp Ala Ile Val Ala Gln
   565 570 575
   Thr Glu Gln Phe Leu Ala Asp Asn Gly Arg Arg Pro Arg Ile Leu Ile
   580 585 590
   Ala Lys Met Gly Gln Asp Gly His Asp Arg Gly Ala Lys Val Ile Ala
   595 600 605
   Ser Ala Tyr Ser Asp Leu Gly Phe Asp Val Asp Leu Ser Pro Met Phe
       610                 615                 620
   Ser Thr Pro Glu Glu Ile Ala Arg Leu Ala Val Glu Asn Asp Val His
   625                 630                 635                 640
   Val Val Gly Ala Ser Ser Leu Ala Ala Gly His Lys Thr Leu Ile Pro
                   645                 650                 655
   Glu Leu Val Glu Ala Leu Lys Lys Trp Gly Arg Glu Asp Ile Cys Val
               660                 665                 670
   Val Ala Gly Gly Val Ile Pro Pro Gln Asp Tyr Ala Phe Leu Gln Glu
           675                 680                 685
   Arg Gly Val Ala Ala Ile Tyr Gly Pro Gly Thr Pro Met Leu Asp Ser
       690                 695                 700
   Val Arg Asp Val Leu Asn Leu Ile Ser Gln His His Asp
   705                 710                 715
<210> 29
   <211> 2166
   <212> DNA
   <213> Artificial Sequence
<220>
   <223> Synthetic nucleotide
<400> 29
   ggatccatgt ctagaatgag caacgtgcag gaatggcagc agctggcgaa taaagaactg 60
   agccgtcgcg aaaaaacggt tgattctctg gtgcatcaga ccgccgaagg tatcgcaatt 120
   aaaccgctgt ataccgaagc ggatctggat aacctggaag tgaccggtac gctgccgggt 180
   ctgccgccgt atgttcgtgg tccgcgtgcg accatgtaca cggcacagcc gtggacgatt 240
   cgtcagtatg cgggcttcag caccgccaaa gaatctaacg cattttaccg tcgcaatctg 300
   gcggcgggtc agaaaggtct gagcgtggcg tttgatctgg ccacccaccg tggttacgat 360
   tctgataacc cgcgcgttgc gggcgatgtg ggtaaagcag gcgttgcgat cgatacggtg 420
   gaagatatga aagttctgtt cgatcagatt ccgctggata aaatgagtgt tagcatgacc 480
   atgaatggcg cggttctgcc ggtgctggcc ttttatatcg tggcagcgga agaacagggt 540
   gttacgccgg ataaactgac cggcacgatc cagaacgata ttctgaaaga atacctgtgc 600
   cgtaatacct atatttaccc gccgaaaccg tctatgcgca ttatcgcaga tattatcgcg 660
   tggtgtagtg gtaacatgcc gcgtttcaat acgatctcta ttagtggcta tcatatgggt 720
   gaagccggcg caaactgcgt tcagcaggtg gcctttaccc tggcagatgg tatcgaatac 780
   attaaagccg caatcagtgc gggcctgaaa attgatgatt tcgccccgcg cctgagcttt 840
   ttctttggca ttggtatgga tctgtttatg aatgtggcca tgctgcgtgc ggcccgctat 900
   ctgtggagcg aagcagtttc tggctttggc gcgcaggacc cgaaaagcct ggcactgcgt 960
   acccattgcc agacgagtgg ttggagcctg accgaacagg acccgtacaa caatgtgatc 1020
   cgcaccacga ttgaagcgct ggcagcaacc ctgggtggta cgcagagcct gcacaccaac 1080
   gcgttcgatg aagccctggg tctgccgacg gattttagcg cccgtatcgc acgcaatacc 1140
   cagattatca ttcaggaaga atctgaactg tgtcgtacgg ttgatccgct ggcgggcagt 1200
   tattacatcg aaagcctgac cgatcagatt gttaaacagg cgcgtgcgat cattcagcag 1260
   attgatgaag caggcggtat ggcaaaagcg atcgaagcgg gcctgccgaa acgtatgatt 1320
   gaagaagcct ctgcacgcga acagagtctg atcgatcagg gtaaacgtgt gattgttggc 1380
   gtgaacaaat acaaactgga tcatgaagat gaaaccgatg tgctggaaat cgataacgtt 1440
   atggtgcgta atgaacagat cgccagcctg gaacgtattc gcgcaacccg cgatgatgcc 1500
   gcagttacgg cggccctgaa cgcactgacc catgcagcgc agcacaacga aaatctgctg 1560
   gccgcagcgg tgaatgccgc acgtgttcgc gcgacgctgg gtgaaatttc tgatgcactg 1620
   gaagtggcgt tcgatcgcta tctggttccg agtcagtgcg ttaccggcgt gatcgcccag 1680
   agttaccatc agagcgaaaa aagcgcatct gaatttgatg cgattgtggc ccagaccgaa 1740
   cagtttctgg cagataacgg ccgtcgcccg cgtatcctga ttgccaaaat gggtcaggat 1800
   ggccacgatc gcggtgcgaa agtgatcgcg tctgcctata gtgatctggg cttcgatgtt 1860
   gatctgtctc cgatgtttag tacgccggaa gaaattgcac gtctggcggt tgaaaatgat 1920
   gtgcatgtgg ttggtgccag ctctctggcg gcgggtcaca aaaccctgat tccggaactg 1980
   gtggaagcgc tgaaaaaatg gggtcgcgaa gatatctgtg tggttgcggg cggtgtgatt 2040
   ccgccgcagg attatgcgtt tctgcaagaa cgtggtgttg cagcaatcta cggtccgggc 2100
   accccgatgc tggatagtgt tcgcgatgtg ctgaatctga ttagccagca tcacgattaa 2160
   gagctc 2166
<210> 30
   <211> 1206
   <212> DNA
   <213> Artificial Sequence
<220>
   <223> Synthetic nucleotide
<400> 30
   ggatccatga aaataaaaac aggtgcacgc atcctcgcat tatccgcatt aacgacgatg 60
   atgttttccg cctcggctct cgccaaaatc gaagaaggta aactggtaat ctggattaac 120
   ggcgataaag gctataacgg tctcgctgaa gtcggtaaga aattcgagaa agataccgga 180
   attaaagtca ccgttgagca tccggataaa ctggaagaga aattcccaca ggttgcggca 240
   actggcgatg gccctgacat tatcttctgg gcacacgacc gctttggtgg ctacgctcaa 300
   tctggcctgt tggctgaaat caccccggac aaagcgttcc aggacaagct gtatccgttt 360
   acctgggatg ccgtacgtta caacggcaag ctgattgctt acccgatcgc tgttgaagcg 420
   ttatcgctga tttataacaa agacctgctg ccgaacccgc caaaaacctg ggaagagatc 480
   ccggcgctgg ataaagaact gaaagcgaaa ggtaagagcg cgctgatgtt caacctgcaa 540
   gaaccgtact tcacctggcc gctgattgct gctgacgggg gttatgcgtt caagtatgaa 600
   aacggcaagt acgacattaa agacgtgggc gtggataacg ctggcgcgaa agcgggtctg 660
   accttcctgg ttgacctgat taaaaacaaa cacatgaatg cagacaccga ttactccatc 720
   gcagaagctg cctttaataa aggcgaaaca gcgatgacca tcaacggccc gtgggcatgg 780
   tccaacatcg acaccagcaa agtgaattat ggtgtaacgg tactgccgac cttcaagggt 840
   caaccatcca aaccgttcgt tggcgtgctg agcgcaggta ttaacgccgc cagtccgaac 900
   aaagagctgg cgaaagagtt cctcgaaaac tatctgctga ctgatgaagg tctggaagcg 960
   gttaataaag acaaaccgct gggtgccgta gcgctgaagt cttacgagga agagttggcg 1020
   aaagatccac gtattgccgc caccatggaa aacgcccaga aaggtgaaat catgccgaac 1080
   atcccgcaga tgtccgcttt ctggtatgcc gtgcgtactg cggtgatcaa cgccgccagc 1140
   ggtcgtcaga ctgtcgatga agccctgaaa gacgcgcaga ctcgtatcac caagtctaga 1200
   gagctc 1206
<210> 31
   <211> 38
   <212> DNA
   <213> Artificial Sequence
<220>
   <223> Synthetic primer
<400> 31
   gagaggtacc atggggggtt ctcatcatca tcatcatc 38
<210> 32
   <211> 38
   <212> DNA
   <213> Artificial Sequence
<220>
   <223> Synthetic primer
<400> 32
   cagccaagct tttattacgc accctgtgcg cgctgttc 38
<210> 33
   <211> 1263
   <212> DNA
   <213> Artificial Sequence
<220>
   <223> Synthetic nucleotide
<400> 33
   atggggggtt ctcatcatca tcatcatcat ggtatggcta gcatgactgg tggacagcaa        60
   atgggtcggg atctgtacga cgatgacgat aaggatcgat ggggatccct ggtggaaggc       120
   ctgcgtgaag ttgccgatgg tgatgcactg tatgatgcag cagtgggtca tggcgatcgt       180
   ggtccggttt gggtgtttag cggccagggt tctcagtggg cagcgatggg cacccagctg       240
   ctggcaagcg aaccggtttt tgccgcaacg attgcaaaac tggaaccggt gatcgcggcc       300
   gaaagtggct tcagcgttac cgaagcaatt acggcgcagc agaccgtgac gggtatcgat       360
   aaagtgcagc cggccgtttt cgcagttcag gtggcgctgg cagcgacgat ggaacagacg       420
   tacggcgttc gtccgggtgc agtggttggt cacagtatgg gtgaaagcgc cgcagcggtg       480
   gttgcaggcg ccctgagtct ggaagatgcc gcacgtgtga tttgccgtcg cagcaaactg       540
   atgacccgta tcgcaggtgc aggtgcgatg ggcagcgtgg aactgccggc aaaacaggtt       600
   aactctgaac tgatggcgcg cggtattgat gatgtggttg tgtctgttgt ggcgtctccg       660
   cagagtaccg tgattggcgg caccagtgat acggttcgtg atctgatcgc gcgttgggaa       720
   cagcgcgatg tgatggcgcg cgaagttgcc gtggatgttg caagccattc tccgcaggtt       780
   gatccgattc tggatgatct ggcggcggca ctggcagata ttgcaccgat gaccccgaaa       840
   gtgccgtatt acagcgcgac gctgtttgat ccgcgtgaac agccggtgtg tgatggcgcc       900
   tattgggttg ataacctgcg caataccgtg cagtttgcgg cggcagttca ggcggcgatg       960
   gaagatggtt accgtgtgtt cgcggaactg tctccgcatc cgctgctgac ccacgcagtg      1020
   gaacagacgg gtcgctctct ggatatgagt gttgcagcac tggccggtat gcgtcgcgaa      1080
   cagccgctgc cgcatggcct gcgtggtctg ctgaccgaac tgcaccgtgc aggtgcagca      1140
   ctggattata gcgcactgta cccggcaggt cgtctggtgg atgcaccgct gccggcatgg      1200
   acgcacgcac gtctgttcat cgatgatgat ggccaggaac agcgcgcaca gggtgcgtaa      1260
   taa                                                                    1263
<210> 34
   <211> 419
   <212> PRT
   <213> Mycobacterium bovis
<400> 34
   Met Gly Gly Ser His His His His His His Gly Met Ala Ser Met Thr
   1 5 10 15
   Gly Gly Gln Gln Met Gly Arg Asp Leu Tyr Asp Asp Asp Asp Lys Asp
   20 25 30
   Arg Trp Gly Ser Leu Val Glu Gly Leu Arg Glu Val Ala Asp Gly Asp
   35 40 45
   Ala Leu Tyr Asp Ala Ala Val Gly His Gly Asp Arg Gly Pro Val Trp
   50 55 60
   Val Phe Ser Gly Gln Gly Ser Gln Trp Ala Ala Met Gly Thr Gln Leu
   65 70 75 80
   Leu Ala Ser Glu Pro Val Phe Ala Ala Thr Ile Ala Lys Leu Glu Pro
   85 90 95
   Val Ile Ala Ala Glu Ser Gly Phe Ser Val Thr Glu Ala Ile Thr Ala
   100 105 110
   Gln Gln Thr Val Thr Gly Ile Asp Lys Val Gln Pro Ala Val Phe Ala
   115 120 125
   Val Gln Val Ala Leu Ala Ala Thr Met Glu Gln Thr Tyr Gly Val Arg
   130 135 140
   Pro Gly Ala Val Val Gly His Ser Met Gly Glu Ser Ala Ala Ala Val
   145 150 155 160
   Val Ala Gly Ala Leu Ser Leu Glu Asp Ala Ala Arg Val Ile Cys Arg
   165 170 175
   Arg Ser Lys Leu Met Thr Arg Ile Ala Gly Ala Gly Ala Met Gly Ser
   180 185 190
   Val Glu Leu Pro Ala Lys Gln Val Asn Ser Glu Leu Met Ala Arg Gly
   195 200 205
   Ile Asp Asp Val Val Val Ser Val Val Ala Ser Pro Gln Ser Thr Val
   210 215 220
   Ile Gly Gly Thr Ser Asp Thr Val Arg Asp Leu Ile Ala Arg Trp Glu
   225 230 235 240
   Gln Arg Asp Val Met Ala Arg Glu Val Ala Val Asp Val Ala Ser His
   245 250 255
   Ser Pro Gln Val Asp Pro Ile Leu Asp Asp Leu Ala Ala Ala Leu Ala
   260 265 270
   Asp Ile Ala Pro Met Thr Pro Lys Val Pro Tyr Tyr Ser Ala Thr Leu
   275 280 285
   Phe Asp Pro Arg Glu Gln Pro Val Cys Asp Gly Ala Tyr Trp Val Asp
   290 295 300
   Asn Leu Arg Asn Thr Val Gln Phe Ala Ala Ala Val Gln Ala Ala Met
   305 310 315 320
   Glu Asp Gly Tyr Arg Val Phe Ala Glu Leu Ser Pro His Pro Leu Leu
   325 330 335
   Thr His Ala Val Glu Gln Thr Gly Arg Ser Leu Asp Met Ser Val Ala
   340 345 350
   Ala Leu Ala Gly Met Arg Arg Glu Gln Pro Leu Pro His Gly Leu Arg
   355 360 365
   Gly Leu Leu Thr Glu Leu His Arg Ala Gly Ala Ala Leu Asp Tyr Ser
   370 375 380
   Ala Leu Tyr Pro Ala Gly Arg Leu Val Asp Ala Pro Leu Pro Ala Trp
   385 390 395 400
   Thr His Ala Arg Leu Phe Ile Asp Asp Asp Gly Gln Glu Gln Arg Ala
   405 410 415
   Gln Gly Ala
<210> 35
   <211> 464
   <212> DNA
   <213> Kribbella flavida DSM
<400> 35
   gagctcagga ggaattaacc atggaacacc tgacggcgac ccagaccctg tttgaagcga 60
   ttgaccacgt tggcgttgca gttgcggatt ttgatgaagc agtgcgtttt tatgcagaaa 120
   ccttcggcat gacggtggct catgaagaag ttaacgaaga acagggtgtt cgtgaagcaa 180
   tgctgtcaat tggcgattcg ggtagctcta tccaactgct ggcgccgctg tccgatagtt 240
   ccccgattgc caaatttctg gaccgcaatg gcccgggtat ccagcaactg gcctatcgtg 300
   tccgcgatct ggacgcagtg agcgcaaccc tgcgtgaacg tggcgcgcaa ctgctgtacg 360
   acgaaccgcg tcgcggcacg gctggttctc gtattaactt cattcatccg aaatcggcgg 420
   gcggcgtcct ggtggaactg gtggaaccgg ctcgctaact gcag 464
<210> 36
   <211> 145
   <212> PRT
   <213> Kribbella flavida DSM
<400> 36
   Met Glu His Leu Thr Ala Thr Gln Thr Leu Phe Glu Ala Ile Asp His
   1 5 10 15
   Val Gly Val Ala Val Ala Asp Phe Asp Glu Ala Val Arg Phe Tyr Ala
   20 25 30
   Glu Thr Phe Gly Met Thr Val Ala His Glu Glu Val Asn Glu Glu Gln
   35 40 45
   Gly Val Arg Glu Ala Met Leu Ser Ile Gly Asp Ser Gly Ser Ser Ile
   50 55 60
   Gln Leu Leu Ala Pro Leu Ser Asp Ser Ser Pro Ile Ala Lys Phe Leu
   65 70 75 80
   Asp Arg Asn Gly Pro Gly Ile Gln Gln Leu Ala Tyr Arg Val Arg Asp
   85 90 95
   Leu Asp Ala Val Ser Ala Thr Leu Arg Glu Arg Gly Ala Gln Leu Leu
   100 105 110
   Tyr Asp Glu Pro Arg Arg Gly Thr Ala Gly Ser Arg Ile Asn Phe Ile
   115 120 125
   His Pro Lys Ser Ala Gly Gly Val Leu Val Glu Leu Val Glu Pro Ala
   130 135 140
   Arg
   145
<210> 37
   <211> 545
   <212> DNA
   <213> Sorangium cellulosum
<400> 37
   gagctcagga ggaattaacc atggctccgc cggcaacgcg tccggctccg gctgcaccga        60
   cgggcctgcc gacccaacgt gaaccgatga aagaccagat tccgggcttt ctgttcattg       120
   atcatatcgc gatggccgtg ccggcaggcc aactggacgc acaagttaaa gcctatgaaa       180
   tgctgggctt tcgtgaagtt catcgcgaag aagtccgtgg tgcggatcag gtgcgcgaag       240
   ttatgctgcg tattggtgat agcgacaacc acgtccaact gctggaaccg ctgagcccgg       300
   aatctccggt tcaaaaactg atcgagaaaa acggcggtcg cggcggtttc gcacatgtgg       360
   cttaccgtgt cagtgatgtg caagcggcct ttgacgaact gaaagcgcgt ggcttccgca       420
   ttatcgatgc agctccgcgt ccgggcagcc gtggcaccac gattttcttt gttcacccgc       480
   gctcacgcga cgatgccccg ttcggtcacc tgattgaagt tgtccagtca catggctaac       540
   tgcag                                                                   545
<210> 38
   <211> 172
   <212> PRT
   <213> Sorangium cellulosum
<400> 38
   Met Ala Pro Pro Ala Thr Arg Pro Ala Pro Ala Ala Pro Thr Gly Leu
   1               5                   10                  15
   Pro Thr Gln Arg Glu Pro Met Lys Asp Gln Ile Pro Gly Phe Leu Phe
               20                  25                  30
   Ile Asp His Ile Ala Met Ala Val Pro Ala Gly Gln Leu Asp Ala Gln
           35                  40                  45
   Val Lys Ala Tyr Glu Met Leu Gly Phe Arg Glu Val His Arg Glu Glu
       50                  55                  60
   Val Arg Gly Ala Asp Gln Val Arg Glu Val Met Leu Arg Ile Gly Asp
   65                  70                  75                  80
   Ser Asp Asn His Val Gln Leu Leu Glu Pro Leu Ser Pro Glu Ser Pro
                   85                  90                  95
   Val Gln Lys Leu Ile Glu Lys Asn Gly Gly Arg Gly Gly Phe Ala His
               100                 105                 110
   Val Ala Tyr Arg Val Ser Asp Val Gln Ala Ala Phe Asp Glu Leu Lys
           115                 120                 125
   Ala Arg Gly Phe Arg Ile Ile Asp Ala Ala Pro Arg Pro Gly Ser Arg
       130                 135                 140
   Gly Thr Thr Ile Phe Phe Val His Pro Arg Ser Arg Asp Asp Ala Pro
   145                 150                 155                 160
   Phe Gly His Leu Ile Glu Val Val Gln Ser His Gly
                   165                 170
<210> 39
   <211> 26
   <212> DNA
   <213> Artificial Sequence
<220>
   <223> Synthetic primer
<400> 39
   taagagctca ggaggaatta accatg
<210> 40
   <211> 566
<210> 41
   <211> 32
   <212> DNA
   <213> Artificial Sequence
<220>
   <223> Synthetic primer
<400> 41
   catgccatgg cggacacgtt attgattctg gg 32
<210> 42
   <211> 9
   <212> PRT
   <213> Artificial Sequence
<220>
   <223> Synthetic peptide
<400> 42
   Met Ala Asp Thr Leu Leu Ile Leu Gly
   1 5
<210> 43
   <211> 36
   <212> DNA
   <213> Artificial Sequence
<220>
   <223> Synthetic primer
<400> 43
   gcggatcctt atgagtcatg atttactaaa ggctgc 36
<210> 44
   <211> 9
<210> 45
   <211> 183
   <212> PRT
   <213> Escherichia coli
<400> 45
   Met Ala Asp Thr Leu Leu Ile Leu Gly Asp Ser Leu Ser Ala Gly Tyr
   1               5                   10                  15
   Arg Met Ser Ala Ser Ala Ala Trp Pro Ala Leu Leu Asn Asp Lys Trp
               20                  25                  30
   Gln Ser Lys Thr Ser Val Val Asn Ala Ser Ile Ser Gly Asp Thr Ser
           35                  40                  45
   Gln Gln Gly Leu Ala Arg Leu Pro Ala Leu Leu Lys Gln His Gln Pro
       50                  55                  60
   Arg Trp Val Leu Val Glu Leu Gly Gly Asn Asp Gly Leu Arg Gly Phe
   65                  70                  75                  80
   Gln Pro Gln Gln Thr Glu Gln Thr Leu Arg Gln Ile Leu Gln Asp Val
                   85                  90                  95
   Lys Ala Ala Asn Ala Glu Pro Leu Leu Met Gln Ile Arg Leu Pro Ala
               100                 105                 110
   Asn Tyr Gly Arg Arg Tyr Asn Glu Ala Phe Ser Ala Ile Tyr Pro Lys
           115                 120                 125
   Leu Ala Lys Glu Phe Asp Val Pro Leu Leu Pro Phe Phe Met Glu Glu
       130                 135                 140
   Val Tyr Leu Lys Pro Gln Trp Met Gln Asp Asp Gly Ile His Pro Asn
   145                 150                 155                 160
   Arg Asp Ala Gln Pro Phe Ile Ala Asp Trp Met Ala Lys Gln Leu Gln
                   165                 170                 175
   Pro Leu Val Asn His Asp Ser
               180
<210> 46
   <211> 33
   <212> DNA
   <213> Artificial Sequence
<220>
   <223> Synthetic primer
<400> 46
   cattactcga gcgcactccc gttctggata atg 33
<210> 47
   <211> 35
   <212> DNA
   <213> Artificial Sequence
<220>
   <223> Synthetic primer
<400> 47
   gggaagctta tgagtcatga tttactaaag gctgc 35
<210> 48
   <211> 9
   <212> PRT
   <213> Artificial Sequence
<220>
   <223> Synthetic peptide
<400> 48
   Ser Asp His Asn Val Leu Pro Gln Leu
   1 5
<210> 49
   <211> 15
   <212> DNA
   <213> Artificial Sequence
<220>
   <223> Synthetic nucleotide
<400> 49
   ggatccatgt ctaga 15
<210> 50
   <211> 34
   <212> PRT
   <213> Artificial Sequence
<220>
   <223> Synthetic peptide
<400> 50
   Met Gly Gly Ser His His His His His His Gly Met Ala Ser Met Thr
   1 5 10 15
   Gly Gly Gln Gln Met Gly Arg Thr Asp Asp Asp Asp Lys Asp Arg Trp
   20 25 30
   Gly Ser
<210> 51
   <211> 655
   <212> PRT
   <213> Ehrlichia chaffeensis
<300>
   <308> NCBI / YP_507303 <309> 2010-05-14
<313> (1)..(655)
<400> 51
   Met Ile Lys Lys Ile Leu Ile Ala Asn Arg Gly Glu Ile Ala Cys Arg
   1               5                   10                  15
   Val Met Arg Thr Ala Arg Lys Met Gly Ile Ser Cys Val Ala Val Tyr
               20                  25                  30
   Ser Asn Ala Asp Val Tyr Ser Leu His Val Leu Ser Ala Glu Glu Ala
           35                  40                  45
   Val Asn Ile Gly Pro Ala Pro Val Asn Gln Ser Tyr Leu Asn Met Glu
       50                  55                  60
   Lys Ile Cys Glu Val Ala Cys Asn Thr Gly Val Asp Ala Val His Pro
   65                  70                  75                  80
   Gly Tyr Gly Phe Leu Ser Glu Asn Ala Asp Phe Pro Glu Lys Leu Glu
                   85                  90                  95
   Gln Tyr Asn Ile Lys Phe Ile Gly Pro Ser Ser Thr Ser Ile Arg Met
               100                 105                 110
   Met Ala Asp Lys Ile Thr Ser Lys Lys Ile Ala Glu Ser Ala Lys Val
           115                 120                 125
   Asn Ile Ile Pro Gly Tyr Met Gly Ile Val Asp Ser Val His Glu Ala
       130                 135                 140
   Lys Glu Ile Ala Lys Ser Ile Gly Phe Pro Val Met Ile Lys Ala Thr
   145                 150                 155                 160
   Ala Gly Gly Gly Gly Lys Gly Met Arg Ile Val Lys Ser Ser Glu Glu
                   165                 170                 175
   Ile Glu Gln Ala Phe Thr Ser Ala Thr Asn Glu Ala Ala Lys Asn Phe
               180                 185                 190
   Arg Asp Gly Arg Ile Phe Ile Glu Lys Tyr Val Glu Leu Pro Arg His
           195                 200                 205
   Ile Glu Ile Gln Ile Ile Ala Asp Lys His Gly Asn Ile Val Cys Leu
       210                 215                 220
   Gly Glu Arg Glu Cys Ser Ile Gln Arg His Asn Gln Lys Val Ile Glu
   225                 230                 235                 240
   Glu Thr Pro Ser Pro Phe Leu Asp Glu Glu Thr Arg Gln Lys Met Tyr
                   245                 250                 255
   Gln Gln Cys Val Asn Leu Ala Lys Lys Val Gly Tyr Tyr Ser Ala Gly
               260                 265                 270
   Thr Ile Glu Phe Ile Val Asp Gln Asp Lys Gln Phe Tyr Phe Leu Glu
           275                 280                 285
   Met Asn Thr Arg Leu Gln Val Glu His Pro Val Thr Glu Leu Val Thr
       290                 295                 300
   Gly Ile Asp Ile Val Glu Glu Met Ile Arg Ile Ala Asp Gly Glu Glu
   305                 310                 315                 320
   Leu Arg Phe Thr Gln Gln Asp Val Lys Phe Thr Gly Ser Ala Ile Glu
                   325                 330                 335
   Ala Arg Val Tyr Ala Glu Asn Pro Thr Lys Asn Phe Leu Pro Ser Ser
               340                 345                 350
   Gly Arg Ile Ala Tyr Tyr Ser Ala Pro Met Pro Asn Asp Asn Leu Arg
           355                 360                 365
   Ile Asp Ser Gly Val Phe Glu Gly Ala Glu Val Ser Met Phe Tyr Asp
       370                 375                 380
   Pro Met Ile Ala Lys Val Cys Thr Tyr Gly Lys Asn Arg Asp Glu Ala
   385                 390                 395                 400
   Val Ser Phe Met Gln Arg Tyr Leu Asn Glu Phe Tyr Ile Gly Gly Ile
                   405                 410                 415
   Ala Asn Asn Ile Asp Phe Leu Leu Ser Val Phe His His Pro Val Phe
               420                 425                 430
   Ile Ser Gly Asn Ile Asn Thr Lys Phe Ile Glu Gln Phe Tyr Phe Asp
   435 440 445
   Gly Phe Gln Gly Asn Pro Leu Thr Lys Ala Cys Ile Lys Leu Phe Ile
   450 455 460
   Leu Thr Ser Leu Cys Ile Phe Phe Gln Asp Glu Tyr Gly Ile His Gly
   465 470 475 480
   Val Glu Leu Cys Glu Asn Arg Glu Leu Ala Val Tyr Val Asp Gly Gln
   485 490 495
   Lys Tyr Leu Ile Ser Ala Lys Tyr Glu Asn Gly Arg Val Leu Ala Ile
   500 505 510
   Tyr Asp Gln Cys Glu Tyr Leu Val Val Ser Thr Trp Asn Val Asn Phe
   515 520 525
   Lys Ile Leu Gln Ile Gln Val Asn Asn Asp Glu Val Phe His Val Lys
   530 535 540
   Val Asp Ser Arg Leu Asn Lys Tyr Gln Leu Lys Tyr Ser Ala Met Ser
   545 550 555 560
   Ala Leu Cys Ala Val Tyr Lys Pro Cys Val Ser Asp Leu Leu Pro Ile
   565 570 575
   Met Pro Gln Ile Ser Gly Glu Glu Leu Tyr Ser Ser Asn Val Cys Ser
   580 585 590
   Pro Ile Ser Gly Met Ile Val Lys Ile Tyr Val Lys Gln Gly Glu Glu
   595 600 605
   Val Gln Pro Gly Gln Pro Leu Leu Val Ile Glu Ala Met Lys Met Glu
   610 615 620
   Asn Val Ile Tyr Ser Asp Val Lys Ser Ile Val Lys Ser Val Leu Phe
   625 630 635 640
   Ser Glu Gly Asn Ser Val Ala Thr Gly Asp Val Ile Ile Glu Phe
   645 650 655
<210> 52
   <211> 510
   <212> PRT
   <213> Ehrlichia chaffeensis
<300>
   <308> NCBI / YP_507410
   <309> 2010-05-14
   <313> (1)..(510)
<400> 52
   Met Asn Phe Ala Gly Leu Gln Asp Leu Asn Asn Arg Gln Ser Lys Ser
   1               5                   10                  15
   Tyr Asn Gly Gly Gly Leu Ser Arg Ile Glu Lys Gln His Leu Lys Gly
               20                  25                  30
   Lys Leu Thr Ala Arg Glu Arg Leu Thr Val Leu Leu Asp Asp Asn Ser
           35                  40                  45
   Phe Glu Glu Tyr Gly Ala Phe Val Glu His Arg Cys Val Asn Phe Ser
       50                  55                  60
   Met Asp Lys Ser Lys Ile Pro Gly Asp Gly Val Val Val Gly Tyr Gly
   65                  70                  75                  80
   Thr Ile Asn Gly Arg Lys Val Cys Ile Tyr Ser Gln Asp Phe Thr Val
                   85                  90                  95
   Phe Gly Gly Ser Leu Ser Glu Ser Asn Ala Lys Lys Ile Cys Asn Ile
               100                 105                 110
   Met Asp Lys Ala Ala Ser Leu Gly Ile Pro Ile Ile Gly Ile Asn Asp
           115                 120                 125
   Ser Gly Gly Ala Arg Ile Gln Glu Gly Val Asp Ser Leu Ser Gly Tyr
       130                 135                 140
   Gly Glu Ile Phe Gln Arg Asn Val Asn Leu Ser Gly Val Val Pro Gln
   145                 150                 155                 160
   Ile Ser Leu Ile Met Gly Pro Cys Ala Gly Gly Ala Val Tyr Ser Pro
                   165                 170                 175
   Ala Leu Thr Asp Phe Ile Phe Met Val Arg Asn Thr Ser Tyr Met Phe
               180                 185                 190
   Val Thr Gly Pro Asp Val Ile Lys Lys Val Thr Tyr Glu Glu Val Thr
           195                 200                 205
   Gln Glu Asp Leu Gly Gly Ala Lys Val His Ala Ser Lys Thr Gly Ile
       210                 215                 220
   Ala Asp Leu Val Phe His Asn Glu Ile Glu Ala Leu Leu Gln Val Arg
   225                 230                 235                 240
   Arg Phe Met Asn Phe Ile Pro Ser Asn Asn Met Glu Ser Ile Gly Ser
                   245                 250                 255
   Gln Ser Ala Ser Asn Phe Ile Asn Met Glu Asp Leu Ser Leu Asn Thr
   260 265 270
   Leu Val Pro Lys Asn Ser Thr Thr Pro Tyr Asn Met Tyr Glu Leu Leu
   275 280 285
   Glu Lys Val Cys Asp Glu Arg Leu Phe Tyr Glu Ile Lys Pro Asp Phe
   290 295 300
   Ala Arg Asn Ile Ile Ile Gly Phe Gly Lys Ile Gly Gly Tyr Asn Val
   305 310 315 320
   Gly Leu Val Ala Asn Gln Pro Leu His Leu Ala Gly Cys Leu Asp Ile
   325 330 335
   Asp Ala Ser Arg Lys Gly Ala Arg Phe Ile Arg Phe Cys Asp Ala Phe
   340 345 350
   Asn Ile Pro Val Ile Thr Phe Ile Asp Val Pro Gly Phe Met Pro Gly
   355 360 365
   Val Asn Gln Glu His Ser Gly Ile Ile Ala His Gly Ala Lys Leu Leu
   370 375 380
   Tyr Ala Tyr Ala Glu Ala Thr Val Pro Lys Ile Ser Val Ile Val Arg
   385 390 395 400
   Lys Ala Tyr Gly Gly Ala Tyr Ile Val Met Asn Ser Lys His Leu Cys
   405 410 415
   Gly Asp Val Asn Tyr Ala Trp Gln Asp Ala Glu Ile Ala Val Met Gly
   420 425 430
   Ala Glu Gly Ala Val Glu Ile Ile Phe Arg Asn Glu Lys Asp Lys Asp
   435 440 445
   Lys Ile Gln His Ile Ile Asp Glu Tyr Arg Thr Thr Ile Val Asn Pro
   450 455 460
   Tyr Val Ala Ala Ser Arg Gly Tyr Ile Asp Asp Ile Ile Val Pro Ser
   465 470 475 480
   Arg Thr Arg Glu His Leu Phe Lys Ser Leu Gln Phe Leu Glu Lys Lys
   485 490 495
   Lys Val His Lys Ile Met Arg Lys His Asp Asn Leu Pro Leu
   500 505 510
<210> 53
   <211> 666
   <212> PRT
   <213> Agrobacterium vitis
<300>
   <308> NCBI / YP_002547482
   <309> 2010-04-01
   <313> (1) .. (666)
<400> 53
   Met Ala Ile Ser Lys Ile Leu Ile Ala Asn Arg Gly Glu Ile Ala Cys
   1 5 10 15
   Arg Val Ile Lys Thr Ala Lys Arg Met Gly Ile Ala Thr Val Ala Val
   20 25 30
   Tyr Ser Asp Ala Asp Ala Asn Ala Leu His Val Lys Leu Ala Asp Glu
   35 40 45
   Ala Val His Ile Gly Pro Ser Pro Ser Asn Gln Ser Tyr Ile Val Ile
   50 55 60
   Asp Lys Ile Leu Glu Ala Ile Arg Gln Thr Gly Ala Asp Ala Val His
   65 70 75 80
   Pro Gly Tyr Gly Phe Leu Ser Glu Asn Ala Ala Phe Ala Glu Ala Leu
   85 90 95
   Asp Lys Ala Gly Val Ala Phe Ile Gly Pro Pro Val Gly Ala Ile Lys
   100 105 110
   Ala Met Gly Asp Lys Ile Thr Ser Lys Lys Leu Ala Ala Glu Ala Gly
   115 120 125
   Val Ser Thr Val Pro Gly His Met Gly Leu Ile Ala Asp Ala Asp Glu
   130 135 140
   Ala Val Lys Ile Ala Ala Gln Ile Gly Tyr Pro Val Met Ile Lys Ala
   145 150 155 160
   Ser Ala Gly Gly Gly Gly Lys Gly Met Arg Ile Ala Trp Asn Asp Ala
   165 170 175
   Glu Ala Arg Glu Gly Phe Gln Ser Ser Lys Asn Glu Ala Met Asn Ser
   180 185 190
   Phe Gly Asp Asp Arg Ile Phe Ile Glu Lys Phe Val Asp Gln Pro Arg
   195 200 205
   His Ile Glu Ile Gln Val Leu Gly Asp Lys His Gly Asn Val Leu Tyr
       210                 215                 220
   Leu Gly Glu Arg Glu Cys Ser Ile Gln Arg Arg Asn Gln Lys Val Ile
   225                 230                 235                 240
   Glu Glu Ala Pro Ser Pro Phe Leu Asp Ala Asp Thr Arg Lys Ala Met
                   245                 250                 255
   Gly Glu Gln Ala Val Ala Leu Ala Lys Ala Val Gly Tyr Tyr Ser Ala
               260                 265                 270
   Gly Thr Val Glu Phe Ile Val Asp Gly Asn Arg Asn Phe Tyr Phe Leu
           275                 280                 285
   Glu Met Asn Thr Arg Leu Gln Val Glu His Pro Val Thr Glu Leu Ile
       290                 295                 300
   Thr Gly Leu Asp Leu Val Glu Gln Met Ile Arg Val Ala Ser Gly Glu
   305                 310                 315                 320
   Thr Leu Ala Leu Ala Gln Gly Asp Val Thr Leu Thr Gly Trp Ala Val
                   325                 330                 335
   Glu Ser Arg Leu Tyr Ala Glu Asp Pro Tyr Arg Asn Phe Leu Pro Ser
               340                 345                 350
   Ile Gly Arg Leu Ser Arg Tyr Arg Pro Pro Ser Glu Gly Gln Gln Ala
           355                 360                 365
   Asp Gly Thr Val Val Arg Asn Asp Thr Gly Val Phe Glu Gly Gly Glu
       370                 375                 380
   Ile Ser Met Tyr Tyr Asp Pro Met Val Ala Lys Leu Cys Thr Trp Gly
   385                 390                 395                 400
   Pro Asp Arg Ile Thr Ala Ile Asp Ala Met Ser Ala Ala Leu Asp Arg
                   405                 410                 415
   Phe Glu Val Glu Gly Ile Gly His Asn Leu Pro Phe Leu Ser Ala Val
               420                 425                 430
   Met Gln His Pro Arg Phe Arg Ser Gly Lys Ile Thr Thr Ala Phe Ile
           435                 440                 445
   Ala Glu Glu Phe Pro Glu Gly Phe Ser Gly Val Glu Pro Asp Glu Met
       450                 455                 460
   Ala Gly Lys Thr Leu Ala Ala Ile Ala Ala Leu Val His Gln Arg Arg
   465                 470                 475                 480
   Glu Ala Arg Ala Ala Gln Val Ser Gly Thr Met Gly Asn His Ala Arg
   485 490 495
   Thr Ile Gly Arg Asp Trp Val Val Gly Leu Ala Glu Gln Asn Tyr Pro
   500 505 510
   Leu Thr Leu Ser Thr Asp Pro Gly Ser Met Met Phe Ala Asp Gly Asn
   515 520 525
   Val Leu Ser Val Asp Gly Val Trp Gln Pro Gly Gln Thr Leu Ala Ile
   530 535 540
   Phe Thr Val Asn Gly Gln Ser Ile Gly Leu Lys Ile Asp Leu Lys Gly
   545 550 555 560
   Pro Ala Ile Arg Leu Arg Trp Arg Gly Met Asp Val Val Ala His Val
   565 570 575
   Arg Asn Pro Arg Val Ala Glu Leu Ala Arg Leu Met Pro Arg Lys Leu
   580 585 590
   Pro Pro Asp Thr Ser Lys Met Leu Leu Cys Pro Met Pro Gly Val Val
   595 600 605
   Thr Gly Ile Ala Val Ala Glu Gly Asp Ala Val Glu Ala Gly Gln Ala
   610 615 620
   Leu Ala Thr Val Glu Ala Met Lys Met Glu Asn Ile Leu Lys Ala Glu
   625 630 635 640
   Arg Arg Gly Val Val Lys Arg Leu Val Ala Lys Ala Gly Gln Ser Leu
   645 650 655
   Ala Val Asp Glu Leu Ile Met Glu Phe Glu
   660 665
<210> 54
   <211> 510
   <212> PRT
   <213> Agrobacterium vitis
<300>
   <308> NCBI / YP_002547479
   <309> 2010-04-01
   <313> (1)..(510)
<400> 54
   Met Pro Thr Ile Leu Asp Gln Leu Glu Ser Arg Arg Ala Glu Ala Arg
   1               5                   10                  15
   Leu Gly Gly Gly Glu Lys Arg Ile Asp Ala Gln His Ala Lys Gly Lys
               20                  25                  30
   Leu Thr Ala Arg Glu Arg Ile Glu Ile Leu Leu Asp Glu Gly Ser Phe
           35                  40                  45
   Glu Glu Tyr Asp Met Tyr Val Thr His Arg Cys Ala Asp Phe Gly Met
       50                  55                  60
   Asp Gly Gln Lys Val Ala Gly Asp Gly Val Val Thr Gly Trp Gly Thr
   65                  70                  75                  80
   Ile Asn Gly Arg Gln Val Tyr Val Phe Ser Gln Asp Phe Thr Val Leu
                   85                  90                  95
   Gly Gly Ser Leu Ser Glu Thr His Ala Gln Lys Ile Cys Lys Ile Met
               100                 105                 110
   Asp Met Ala Val Arg Val Gly Ala Pro Val Ile Gly Ile Asn Asp Ser
           115                 120                 125
   Gly Gly Ala Arg Ile Gln Glu Gly Val Ala Ser Leu Ala Gly Tyr Ala
       130                 135                 140
   Glu Val Phe Arg Arg Asn Ala Glu Val Ser Gly Val Ile Pro Gln Ile
   145                 150                 155                 160
   Ser Val Ile Met Gly Pro Cys Ala Gly Gly Ala Val Tyr Ser Pro Ala
                   165                 170                 175
   Met Thr Asp Phe Ile Phe Met Val Arg Asp Thr Ser Tyr Met Phe Val
               180                 185                 190
   Thr Gly Pro Asp Val Val Lys Thr Val Thr Asn Glu Ile Val Thr Ala
           195                 200                 205
   Glu Glu Leu Gly Gly Ala Gly Thr His Thr Lys Lys Ser Ser Val Ala
       210                 215                 220
   Asp Gly Ala Phe Glu Asn Asp Val Glu Ala Leu Glu Gln Val Arg Leu
   225                 230                 235                 240
   Leu Phe Asp Phe Leu Pro Leu Asn Asn Arg Glu Lys Pro Pro Lys Arg
                   245                 250                 255
   Pro Phe Tyr Asp Asp Pro Ala Arg Leu Glu Met Arg Leu Asp Thr Leu
               260                 265                 270
   Ile Pro Asp Ser Ser Thr Lys Pro Tyr Asp Met Lys Glu Leu Ile His
           275                 280                 285
   Ala Leu Ala Asp Glu Gly Asp Phe Phe Glu Leu Gln Glu Ala Phe Ala
       290                 295                 300
   Lys Asn Ile Ile Thr Gly Phe Ile Arg Leu Glu Gly Gln Thr Val Gly
   305                 310                 315                 320
   Val Val Ala Asn Gln Pro Met Val Leu Ala Gly Cys Leu Asp Ile Asp
                   325                 330                 335
   Ser Ser Arg Lys Ala Ala Arg Phe Val Arg Phe Cys Asp Ala Phe Ser
               340                 345                 350
   Ile Pro Ile Leu Thr Leu Val Asp Val Pro Gly Phe Leu Pro Gly Val
           355                 360                 365
   Ala Gln Glu Tyr Gly Gly Val Ile Lys His Gly Ala Lys Leu Leu Phe
       370                 375                 380
   Ala Tyr Ser Glu Ala Thr Val Pro Met Val Thr Leu Ile Thr Arg Lys
   385                 390                 395                 400
   Ala Tyr Gly Gly Ala Tyr Asp Val Met Ala Ser Lys His Ile Gly Ala
                   405                 410                 415
   Asp Val Asn Tyr Ala Trp Pro Thr Ala Glu Ile Ala Val Met Gly Ala
               420                 425                 430
   Lys Gly Ala Thr Glu Ile Leu Tyr Arg Ser Glu Leu Ala Asp Pro Glu
           435                 440                 445
   Lys Ile Ala Ala Arg Thr Arg Glu Tyr Glu Glu Arg Phe Ala Asn Pro
       450                 455                 460
   Phe Val Ala Ala Glu Arg Gly Phe Ile Asp Glu Val Ile Met Pro His
   465                 470                 475                 480
   Ser Ser Arg Lys Arg Ile Ala Arg Ala Phe Ala Ser Leu Arg Gly Lys
                   485                 490                 495
   Gln Val Ala Thr His Trp Lys Lys His Asp Thr Ile Pro Leu
               500                 505                 510
<210> 55
   <211> 667
   <212> PRT
   <213> Methylobacterium extorquens
<300>
   <308> NCBI / YP_003069256
   <309> 2010-04-16
   <313> (1)..(667)
<400> 55
   Met Phe Asp Lys Ile Leu Ile Ala Asn Arg Gly Glu Ile Ala Cys Arg
   1 5 10 15
   Ile Ile Lys Thr Ala Gln Lys Met Gly Ile Lys Thr Val Ala Val Tyr
   20 25 30
   Ser Asp Ala Asp Arg Asp Ala Val His Val Ala Met Ala Asp Glu Ala
   35 40 45
   Val Asn Ile Gly Pro Ala Pro Ala Ala Gln Ser Tyr Leu Leu Ile Glu
   50 55 60
   Lys Ile Ile Asp Ala Cys Lys Gln Thr Gly Ala Gln Ala Val His Pro
   65 70 75 80
   Gly Tyr Gly Phe Leu Ser Glu Arg Glu Ser Phe Pro Lys Ala Leu Ala
   85 90 95
   Glu Ala Gly Ile Val Phe Ile Gly Pro Asn Pro Gly Ala Ile Ala Ala
   100 105 110
   Met Gly Asp Lys Ile Glu Ser Lys Lys Ala Ala Ala Ala Ala Glu Val
   115 120 125
   Ser Thr Val Pro Gly Phe Leu Gly Val Ile Glu Ser Pro Glu His Ala
   130 135 140
   Val Thr Ile Ala Asp Glu Ile Gly Tyr Pro Val Met Ile Lys Ala Ser
   145 150 155 160
   Ala Gly Gly Gly Gly Lys Gly Met Arg Ile Ala Glu Ser Ala Asp Glu
   165 170 175
   Val Ala Glu Gly Phe Ala Arg Ala Lys Ser Glu Ala Ser Ser Ser Phe
   180 185 190
   Gly Asp Asp Arg Val Phe Val Glu Lys Phe Ile Thr Asp Pro Arg His
   195 200 205
   Ile Glu Ile Gln Val Ile Gly Asp Lys His Gly Asn Val Ile Tyr Leu
   210 215 220
   Gly Glu Arg Glu Cys Ser Ile Gln Arg Arg Asn Gln Lys Val Ile Glu
   225 230 235 240
   Glu Ala Pro Ser Pro Leu Leu Asp Glu Glu Thr Arg Arg Lys Met Gly
   245 250 255
   Glu Gln Ala Val Ala Leu Ala Lys Ala Val Asn Tyr Asp Ser Ala Gly
   260 265 270
   Thr Val Glu Phe Val Ala Gly Gln Asp Lys Ser Phe Tyr Phe Leu Glu
   275 280 285
   Met Asn Thr Arg Leu Gln Val Glu His Pro Val Thr Glu Met Ile Thr
   290 295 300
   Gly Leu Asp Leu Val Glu Leu Met Ile Arg Val Ala Ala Gly Glu Thr
   305 310 315 320
   Leu Pro Leu Thr Gln Asp Gln Val Lys Leu Asp Gly Trp Ala Val Glu
   325 330 335
   Ser Arg Val Tyr Ala Glu Asp Pro Thr Arg Asn Phe Leu Pro Ser Ile
   340 345 350
   Gly Arg Leu Thr Thr Tyr Gln Pro Pro Glu Glu Gly Pro Leu Gly Gly
   355 360 365
   Ala Ile Val Arg Asn Asp Thr Gly Val Glu Glu Gly Gly Glu Ile Ala
   370 375 380
   Ile His Tyr Asp Pro Met Ile Ala Lys Leu Val Thr Trp Ala Pro Thr
   385 390 395 400
   Arg Leu Glu Ala Ile Asp Ala Gln Ala Thr Ala Leu Asp Ala Phe Ala
   405 410 415
   Ile Glu Gly Ile Arg His Asn Ile Pro Phe Leu Ala Thr Leu Met Ala
   420 425 430
   His Pro Arg Trp Arg Asp Gly Arg Leu Ser Thr Gly Phe Ile Lys Glu
   435 440 445
   Glu Phe Pro Glu Gly Phe Ile Ala Pro Glu Pro Glu Gly Pro Val Ala
   450 455 460
   His Arg Leu Ala Ala Val Ala Ala Ala Ile Asp His Lys Leu Asn Ile
   465 470 475 480
   Arg Lys Arg Gly Ile Ser Gly Gln Met Arg Asp Pro Ser Leu Leu Thr
   485 490 495
   Phe Gln Arg Glu Arg Val Val Val Leu Ser Gly Gln Arg Phe Asn Val
   500 505 510
   Thr Val Asp Pro Asp Gly Asp Asp Leu Leu Val Thr Phe Asp Asp Gly
   515 520 525
   Thr Thr Ala Pro Val Arg Ser Ala Trp Arg Pro Gly Ala Pro Val Trp
   530 535 540
   Ser Gly Thr Val Gly Asp Gln Ser Ile Ala Ile Gln Val Arg Pro Leu
   545 550 555 560
   Leu Asn Gly Val Phe Leu Gln His Ala Gly Ala Ala Ala Glu Ala Arg
   565 570 575
   Val Phe Thr Arg Arg Glu Ala Glu Leu Ala Asp Leu Met Pro Val Lys
   580 585 590
   Glu Asn Ala Gly Ser Gly Lys Gln Leu Leu Cys Pro Met Pro Gly Leu
   595 600 605
   Val Lys Gln Ile Met Val Ser Glu Gly Gln Glu Val Lys Asn Gly Glu
   610 615 620
   Pro Leu Ala Ile Val Glu Ala Met Lys Met Glu Asn Val Leu Arg Ala
   625 630 635 640
   Glu Arg Asp Gly Thr Ile Ser Lys Ile Ala Ala Lys Glu Gly Asp Ser
   645 650 655
   Leu Ala Val Asp Ala Val Ile Leu Glu Phe Ala
   660 665
<210> 56
   <211> 510
   <212> PRT
   <213> Methylobacterium extorquens
<300>
   <308> NCBI / YP_003065890
   <309> 2010-04-16
   <313> (1)..(510)
<400> 56
   Met Lys Asp Ile Leu Glu Lys Leu Glu Glu Arg Arg Ala Gln Ala Arg
   1 5 10 15
   Leu Gly Gly Gly Glu Lys Arg Leu Glu Ala Gln His Thr Arg Gly Lys
   20 25 30
   Leu Thr Ala Arg Glu Arg Ile Glu Leu Leu Leu Asp His Gly Ser Phe
   35 40 45
   Glu Glu Phe Asp Met Phe Val Gln His Arg Ser Thr Asp Phe Gly Met
   50 55 60
   Glu Lys Gln Lys Ile Pro Gly Asp Gly Val Val Thr Gly Trp Gly Thr
   65 70 75 80
   Val Asn Gly Arg Thr Val Phe Leu Phe Ser Lys Asp Phe Thr Val Phe
   85 90 95
   Gly Gly Ser Leu Ser Glu Ala His Ala Ala Lys Ile Val Lys Val Gln
   100 105 110
   Asp Met Ala Leu Lys Met Arg Ala Pro Ile Ile Gly Ile Phe Asp Ala
   115 120 125
   Gly Gly Ala Arg Ile Gln Glu Gly Val Ala Ala Leu Gly Gly Tyr Gly
   130 135 140
   Glu Val Phe Arg Arg Asn Val Ala Ala Ser Gly Val Ile Pro Gln Ile
   145 150 155 160
   Ser Val Ile Met Gly Pro Cys Ala Gly Gly Asp Val Tyr Ser Pro Ala
   165 170 175
   Met Thr Asp Phe Ile Phe Met Val Arg Asp Thr Ser Tyr Met Phe Val
   180 185 190
   Thr Gly Pro Asp Val Val Lys Thr Val Thr Asn Glu Val Val Thr Ala
   195 200 205
   Glu Glu Leu Gly Gly Ala Lys Val His Thr Ser Lys Ser Ser Ile Ala
   210 215 220
   Asp Gly Ser Phe Glu Asn Asp Val Glu Ala Ile Leu Gln Ile Arg Arg
   225 230 235 240
   Leu Leu Asp Phe Leu Pro Ala Asn Asn Ile Glu Gly Val Pro Glu Ile
   245 250 255
   Glu Ser Phe Asp Asp Val Asn Arg Leu Asp Lys Ser Leu Asp Thr Leu
   260 265 270
   Ile Pro Asp Asn Pro Asn Lys Pro Tyr Asp Met Gly Glu Leu Ile Arg
   275 280 285
   Arg Val Val Asp Glu Gly Asp Phe Phe Glu Ile Gln Ala Ala Tyr Ala
   290 295 300
   Arg Asn Ile Ile Thr Gly Phe Gly Arg Val Glu Gly Arg Thr Val Gly
   305 310 315 320
   Phe Val Ala Asn Gln Pro Leu Val Leu Ala Gly Val Leu Asp Ser Asp
   325 330 335
   Ala Ser Arg Lys Ala Ala Arg Phe Val Arg Phe Cys Asn Ala Phe Ser
   340 345 350
   Ile Pro Ile Val Thr Phe Val Asp Val Pro Gly Phe Leu Pro Gly Thr
   355 360 365
   Ala Gln Glu Tyr Gly Gly Leu Ile Lys His Gly Ala Lys Leu Leu Phe
   370 375 380
   Ala Tyr Ser Gln Ala Thr Val Pro Leu Val Thr Ile Ile Thr Arg Lys
   385 390 395 400
   Ala Phe Gly Gly Ala Tyr Asp Val Met Ala Ser Lys His Val Gly Ala
   405 410 415
   Asp Leu Asn Tyr Ala Trp Pro Thr Ala Gln Ile Ala Val Met Gly Ala
   420 425 430
   Lys Gly Ala Val Glu Ile Ile Phe Arg Ala Glu Ile Gly Asp Ala Asp
   435 440 445
   Lys Ile Ala Glu Arg Thr Lys Glu Tyr Glu Asp Arg Phe Leu Ser Pro
   450 455 460
   Phe Val Ala Ala Glu Arg Gly Tyr Ile Asp Glu Val Ile Met Pro His
   465 470 475 480
   Ser Thr Arg Lys Arg Ile Ala Arg Ala Leu Gly Met Leu Arg Thr Lys
   485 490 495
   Glu Met Glu Gln Pro Trp Lys Lys His Asp Asn Ile Pro Leu
   500 505 510
<210> 57
   <211> 670
   <212> PRT
   <213> Sinorhizobium meliloti
<300>
   <308> NCBI / NP_437988
   <309> 2010-04-01
   <313> (1)..(670)
<400> 57
   Met Gly His Met Phe Lys Lys Ile Leu Ile Ala Asn Arg Gly Glu Ile
   1               5                   10                  15
   Ala Cys Arg Val Ile Arg Thr Thr Lys Ala Leu Gly Ile Pro Thr Val
               20                  25                  30
   Ala Val Tyr Ser Asp Ala Asp Arg Asp Ala Met His Val Arg Met Ala
           35                  40                  45
   Asp Glu Ala Val His Ile Gly Pro Ser Pro Ser Ser Gln Ser Tyr Ile
       50                  55                  60
   Val Ile Glu Asn Ile Leu Ala Ala Ile Arg Arg Thr Gly Ala Asp Ala
   65                  70                  75                  80
   Val His Pro Gly Tyr Gly Phe Leu Ser Glu Asn Ala Ala Phe Ala Glu
                   85                  90                  95
   Ala Leu Glu Lys Asp Gly Val Thr Phe Ile Gly Pro Pro Val Arg Ala
               100                 105                 110
   Ile Glu Ala Met Gly Asp Lys Ile Thr Ser Lys Lys Leu Ala Ala Glu
           115                 120                 125
   Ala Gly Val Phe Thr Val Pro Gly His Met Gly Leu Ile Glu Asp Ala
       130                 135                 140
   Asp Glu Ala Ala Arg Ile Ala Ala Glu Ile Gly Phe Pro Val Met Ile
   145                 150                 155                 160
   Lys Ala Ser Ala Gly Gly Gly Gly Lys Gly Met Arg Ile Ala Trp Asn
                   165                 170                 175
   Glu Arg Glu Ala Arg Glu Gly Phe Gln Ser Ser Arg Asn Glu Ala Lys
               180                 185                 190
   Ser Ser Phe Gly Asp Asp Arg Ile Phe Ile Glu Lys Phe Val Thr Glu
           195                 200                 205
   Pro Arg His Ile Glu Ile Gln Val Leu Gly Asp Lys His Gly Asn Ile
       210                 215                 220
   Leu Tyr Leu Gly Glu Arg Glu Cys Ser Ile Gln Arg Arg Asn Gln Lys
   225                 230                 235                 240
   Val Ile Glu Glu Ala Pro Ser Pro Phe Leu Asp Glu Lys Thr Arg Arg
                   245                 250                 255
   Ala Met Gly Glu Gln Ala Val Ala Leu Ala Lys Ala Val Gly Tyr His
   260 265 270
   Ser Ala Gly Thr Val Glu Phe Ile Val Asp Ala Gly Arg Asn Phe Tyr
   275 280 285
   Phe Leu Glu Met Asn Thr Arg Leu Gln Val Glu His Pro Val Thr Glu
   290 295 300
   Leu Val Thr Gly Leu Asp Leu Val Glu Gln Met Ile Arg Val Ala Ala
   305 310 315 320
   Gly Ala Lys Leu Ala Phe Ala Gln Lys Asp Val Lys Leu Asp Gly Trp
   325 330 335
   Ala Ile Glu Ser Arg Leu Tyr Ala Glu Asp Pro Tyr Arg Thr Phe Leu
   340 345 350
   Pro Ser Ile Gly Arg Leu Thr Arg Tyr Arg Pro Pro Glu Glu Gly Thr
   355 360 365
   Gln Ala Asp Gly Thr Val Ile Arg Asn Asp Thr Gly Val Phe Glu Gly
   370 375 380
   Gly Glu Ile Ser Met Tyr Tyr Asp Pro Met Ile Ala Lys Leu Cys Thr
   385 390 395 400
   Trp Gly Pro Asp Arg Leu Thr Ala Val Arg Ala Met Ala Asp Ala Leu
   405 410 415
   Asp Ala Phe Glu Val Glu Gly Ile Gly His Asn Leu Pro Phe Leu Ala
   420 425 430
   Ala Val Met Gln Gln Glu Arg Phe His Glu Gly Arg Leu Thr Thr Ala
   435 440 445
   Tyr Ile Ala Glu Glu Phe Ala Gly Gly Phe His Gly Val Ala Leu Asp
   450 455 460
   Asp Ala Ser Ala Arg Lys Leu Ala Ala Val Ala Ala Thr Val Asn Gln
   465 470 475 480
   Thr Leu Gln Glu Arg Ala Ser Arg Ile Ser Gly Thr Ile Gly Asn His
   485 490 495
   Arg Arg Val Val Gly His Glu Trp Val Thr Ser Leu Asp Gly His Glu
   500 505 510
   Ile Gln Val Thr Cys Glu Val Ser Ala Asp Gly Thr Tyr Val Arg Phe
           515                 520                 525
   Ala Asp Gly Thr Ser Val Ser Val Ala Thr Asp Trp Ala Pro Gly Arg
       530                 535                 540
   Thr Arg Ala Ala Phe Asn Ile Asp Asn Gln Pro Met Ser Val Lys Val
   545                 550                 555                 560
   Glu Leu Ala Gly Pro Gly Ile Arg Leu Arg Trp Arg Gly Ile Asp Val
                   565                 570                 575
   Val Ala Arg Val Arg Ser Pro Arg Ile Ala Glu Leu Ala Arg Leu Met
               580                 585                 590
   Pro Lys Lys Leu Pro Pro Asp Thr Ser Lys Met Leu Leu Cys Pro Met
           595                 600                 605
   Pro Gly Val Val Thr Ser Ile Thr Val Lys Ala Gly Glu Thr Val Glu
       610                 615                 620
   Ala Gly Gln Ala Ile Ala Val Val Glu Ala Met Lys Met Glu Asn Ile
   625                 630                 635                 640
   Leu Arg Ala Glu Lys Arg Ala Ile Val Lys Arg Val Ala Ile Glu Ala
                   645                 650                 655
   Gly Ala Ser Leu Ala Val Asp Glu Leu Ile Met Glu Phe Glu
               660                 665                 670
<210> 58
   <211> 510
   <212> PRT
   <213> Sinorhizobium meliloti
<300>
   <308> NCBI / NP_437987
   <309> 2010-04-01
   <313> (1)..(510)
<400> 58
   Met Arg Ala Val Leu Glu Gln Val Glu Ala Arg Arg Ala Glu Ala Arg
   1               5                   10                  15
   Ala Gly Gly Gly Glu Arg Arg Ile Ala Ala Gln His Gly Lys Gly Lys
               20                  25                  30
   Leu Thr Ala Arg Glu Arg Ile Asp Val Leu Leu Asp Glu Gly Ser Phe
           35                  40                  45
   Glu Glu Tyr Asp Met Tyr Val Thr His Arg Ser Val Asp Phe Gly Met
   50 55 60
   Ala Gly Gln Lys Ile Pro Gly Asp Gly Val Val Thr Gly Trp Gly Thr
   65 70 75 80
   Ile Asn Gly Arg Gln Val Tyr Val Phe Ser Gln Asp Phe Thr Val Leu
   85 90 95
   Gly Gly Ser Leu Ser Glu Thr His Ala Gln Lys Ile Cys Lys Ile Met
   100 105 110
   Asp Met Ala Ala Arg Asn Gly Ala Pro Val Ile Gly Leu Asn Asp Ser
   115 120 125
   Gly Gly Ala Arg Ile Gln Glu Gly Val Ala Ser Leu Ala Gly Tyr Ala
   130 135 140
   Glu Val Phe Arg Arg Asn Ala Glu Val Ser Gly Val Ile Pro Gln Ile
   145 150 155 160
   Ser Val Ile Met Gly Pro Cys Ala Gly Gly Ala Val Tyr Ser Pro Ala
   165 170 175
   Met Thr Asp Phe Ile Phe Met Val Arg Asp Ser Ser Tyr Met Phe Val
   180 185 190
   Thr Gly Pro Asp Val Val Lys Thr Val Thr Asn Glu Ile Val Thr Ala
   195 200 205
   Glu Glu Leu Gly Gly Ala Arg Thr His Thr Thr Lys Ser Ser Val Ala
   210 215 220
   Asp Gly Ala Tyr Glu Asn Asp Ile Glu Ala Leu Glu His Val Arg Leu
   225 230 235 240
   Leu Phe Asp Phe Leu Pro Leu Asn Asn Arg Glu Lys Pro Pro Val Arg
   245 250 255
   Pro Phe His Asp Asp Pro Gly Arg Leu Glu Met Arg Leu Asp Ser Leu
   260 265 270
   Ile Pro Asp Ser Ala Ala Lys Pro Tyr Asp Met Lys Glu Leu Ile Leu
   275 280 285
   Ala Ile Ala Asp Glu Ala Asp Phe Phe Glu Leu Gln Ala Ser Phe Ala
   290 295 300
   Arg Asn Ile Ile Thr Gly Phe Ile Arg Ile Glu Gly Gln Thr Val Gly
   305 310 315 320
   Val Ile Ala Asn Gln Pro Met Val Leu Ala Gly Cys Leu Asp Ile Asp
   325 330 335
   Ser Ser Arg Lys Ala Ala Arg Phe Val Arg Phe Cys Asp Ala Phe Ser
   340 345 350
   Ile Pro Ile Leu Thr Leu Val Asp Val Pro Gly Phe Leu Pro Gly Thr
   355 360 365
   Ala Gln Glu Tyr Gly Gly Val Ile Lys His Gly Ala Lys Leu Leu Phe
   370 375 380
   Ala Tyr Ser Gln Ala Thr Val Pro Met Val Thr Leu Ile Thr Arg Lys
   385 390 395 400
   Ala Tyr Gly Gly Ala Tyr Asp Val Met Ala Ser Lys His Ile Gly Ala
   405 410 415
   Asp Val Asn Tyr Ala Trp Pro Thr Ala Glu Ile Ala Val Met Gly Ala
   420 425 430
   Lys Gly Ala Thr Glu Ile Leu Tyr Arg Ser Glu Leu Gly Asp Pro Ala
   435 440 445
   Lys Ile Ala Ala Arg Thr Lys Glu Tyr Glu Glu Arg Phe Ala Asn Pro
   450 455 460
   Phe Val Ala Ala Glu Arg Gly Phe Ile Asp Glu Val Ile Met Pro His
   465 470 475 480
   Ser Ser Arg Arg Arg Ile Ala Arg Ala Phe Ala Ser Leu Arg Asn Lys
   485 490 495
   Gln Val Glu Thr Arg Trp Arg Lys His Asp Thr Ile Pro Leu
   500 505 510
<210> 59
   <211> 681
   <212> PRT
   <213> Ruegeria pomeroyi
<300>
   <308> NCBI / YP_166352
   <309> 2010-06-29
   <313> (1)..(681)
<400> 59
   Met Phe Asn Lys Ile Leu Ile Ala Asn Arg Gly Glu Ile Ala Cys Arg
   1               5                   10                  15
   Val Ile Lys Thr Ala Arg Lys Met Gly Ile Ser Thr Val Ala Ile Tyr
               20                  25                  30
   Ser Asp Ala Asp Lys Gln Ala Leu His Val Gln Met Ala Asp Glu Ala
           35                  40                  45
   Val His Ile Gly Pro Pro Pro Ala Asn Gln Ser Tyr Ile Val Ile Asp
       50                  55                  60
   Lys Val Met Ala Ala Ile Arg Ala Thr Gly Ala Gln Ala Val His Pro
   65                  70                  75                  80
   Gly Tyr Gly Phe Leu Ser Glu Asn Ser Lys Phe Ala Glu Ala Leu Glu
                   85                  90                  95
   Ala Glu Gly Val Ile Phe Val Gly Pro Pro Lys Gly Ala Ile Glu Ala
               100                 105                 110
   Met Gly Asp Lys Ile Thr Ser Lys Lys Ile Ala Gln Glu Ala Asn Val
           115                 120                 125
   Ser Thr Val Pro Gly Tyr Met Gly Leu Ile Glu Asp Ala Asp Glu Ala
       130                 135                 140
   Val Lys Ile Ser Asn Gln Ile Gly Tyr Pro Val Met Ile Lys Ala Ser
   145                 150                 155                 160
   Ala Gly Gly Gly Gly Lys Gly Met Arg Ile Ala Trp Asn Asp Gln Glu
                   165                 170                 175
   Ala Arg Glu Gly Phe Gln Ser Ser Lys Asn Glu Ala Ala Asn Ser Phe
               180                 185                 190
   Gly Asp Asp Arg Ile Phe Ile Glu Lys Phe Val Thr Gln Pro Arg His
           195                 200                 205
   Ile Glu Ile Gln Val Leu Cys Asp Ser His Gly Asn Gly Ile Tyr Leu
       210                 215                 220
   Gly Glu Arg Glu Cys Ser Ile Gln Arg Arg Asn Gln Lys Val Val Glu
   225                 230                 235                 240
   Glu Ala Pro Ser Pro Phe Leu Asp Glu Ala Thr Arg Arg Ala Met Gly
                   245                 250                 255
   Glu Gln Ala Val Ala Leu Ala Lys Ala Val Gly Tyr Ala Ser Ala Gly
               260                 265                 270
   Thr Val Glu Phe Ile Val Asp Gly Gln Lys Asn Phe Tyr Phe Leu Glu
   275 280 285
   Met Asn Thr Arg Leu Gln Val Glu His Pro Val Thr Glu Leu Ile Thr
   290 295 300
   Gly Val Asp Leu Val Glu Gln Met Ile Arg Val Ala Ala Gly Glu Pro
   305 310 315 320
   Leu Ser Ile Thr Gln Gly Asp Val Lys Leu Thr Gly Trp Ala Ile Glu
   325 330 335
   Asn Arg Leu Tyr Ala Glu Asp Pro Tyr Arg Gly Phe Leu Pro Ser Ile
   340 345 350
   Gly Arg Leu Thr Arg Tyr Arg Pro Pro Ala Glu Thr Ala Ala Gly Pro
   355 360 365
   Leu Leu Val Asn Gly Lys Trp Gln Gly Asp Ala Pro Ser Gly Glu Ala
   370 375 380
   Ala Val Arg Asn Asp Thr Gly Val Tyr Glu Gly Gly Glu Ile Ser Met
   385 390 395 400
   Tyr Tyr Asp Pro Met Ile Ala Lys Leu Cys Thr Trp Ala Pro Thr Arg
   405 410 415
   Ala Ala Ala Ile Glu Ala Met Arg Ile Ala Leu Asp Ser Phe Glu Val
   420 425 430
   Glu Gly Ile Gly His Asn Leu Pro Phe Leu Ser Ala Val Met Asp His
   435 440 445
   Pro Lys Phe Ile Ser Gly Asp Met Thr Thr Ala Phe Ile Ala Glu Glu
   450 455 460
   Tyr Pro Glu Gly Phe Glu Gly Val Asn Leu Pro Glu Thr Asp Leu Arg
   465 470 475 480
   Arg Val Ala Ala Ala Ala Ala Ala Met His Arg Val Ala Glu Ile Arg
   485 490 495
   Arg Thr Arg Val Ser Gly Arg Met Asp Asn His Glu Arg Arg Val Gly
   500 505 510
   Thr Glu Trp Val Val Thr Leu Gln Gly Ala Asp Phe Pro Val Thr Ile
   515 520 525
   Ala Ala Asp His Asp Gly Ser Thr Val Ser Phe Asp Asp Gly Ser Ser
   530 535 540
   Met Arg Val Thr Ser Asp Trp Thr Pro Gly Asp Gln Leu Ala Asn Leu
   545 550 555 560
   Met Val Asp Gly Ala Pro Leu Val Leu Lys Val Gly Lys Ile Ser Gly
   565 570 575
   Gly Phe Arg Ile Arg Thr Arg Gly Ala Asp Leu Lys Val His Val Arg
   580 585 590
   Thr Pro Arg Gln Ala Glu Leu Ala Arg Leu Met Pro Glu Lys Leu Pro
   595 600 605
   Pro Asp Thr Ser Lys Met Leu Leu Cys Pro Met Pro Gly Leu Ile Val
   610 615 620
   Lys Val Asp Val Glu Val Gly Gln Glu Val Gln Glu Gly Gln Ala Leu
   625 630 635 640
   Cys Thr Ile Glu Ala Met Lys Met Glu Asn Ile Leu Arg Ala Glu Lys
   645 650 655
   Lys Gly Val Val Ala Lys Ile Asn Ala Ser Ala Gly Asn Ser Leu Ala
   660 665 670
   Val Asp Asp Val Ile Met Glu Phe Glu
   675 680
<210> 60
   <211> 510
   <212> PRT
   <213> Ruegeria pomeroyi
<300>
   <308> NCBI / YP_166345
   <309> 2010-06-29
   <313> (1)..(510)
<400> 60
   Met Lys Asp Ile Leu Ser Glu Leu Glu Thr Arg Arg Glu Ala Ala Arg
   1               5                   10                  15
   Leu Gly Gly Gly Gln Lys Arg Ile Asp Ala Gln His Ala Arg Gly Lys
               20                  25                  30
   Leu Thr Ala Arg Glu Arg Ile Glu Leu Leu Leu Asp Glu Asp Ser Phe
           35                  40                  45
   Glu Glu Phe Asp Met Phe Val Ser His Arg Cys Thr Asp Phe Gly Met
       50                  55                  60
   Glu Lys Gln Arg Pro Ala Gly Asp Gly Val Val Thr Gly Trp Gly Thr
   65 70 75 80
   Ile Asn Gly Arg Met Val Tyr Val Phe Ser Gln Asp Phe Thr Val Phe
   85 90 95
   Gly Gly Ser Leu Ser Glu Thr His Ala Gln Lys Ile Cys Lys Ile Met
   100 105 110
   Asp Met Ala Val Gln Asn Gly Ala Pro Val Ile Gly Ile Asn Asp Ser
   115 120 125
   Gly Gly Ala Arg Ile Gln Glu Gly Val Ala Ser Leu Ala Gly Tyr Ala
   130 135 140
   Glu Val Phe Gln Arg Asn Ile Met Ala Ser Gly Val Val Pro Gln Ile
   145 150 155 160
   Ser Val Ile Met Gly Pro Cys Ala Gly Gly Ala Val Tyr Ser Pro Ala
   165 170 175
   Met Thr Asp Phe Ile Phe Met Val Lys Asp Thr Ser Tyr Met Phe Val
   180 185 190
   Thr Gly Pro Asp Val Val Lys Thr Val Thr Asn Glu Val Val Thr Ala
   195 200 205
   Glu Glu Leu Gly Gly Ala Ser Thr His Thr Arg Lys Ser Ser Val Ala
   210 215 220
   Asp Gly Ala Phe Glu Asn Asp Val Glu Ala Leu Ala Glu Val Arg Arg
   225 230 235 240
   Leu Val Asp Phe Leu Pro Leu Asn Asn Arg Glu Lys Pro Pro Val Arg
   245 250 255
   Pro Phe Phe Asp Glu Pro Gly Arg Ile Glu Ala Ser Leu Asp Thr Leu
   260 265 270
   Val Pro Glu Asn Ala Asn Thr Pro Tyr Asp Met Lys Glu Leu Ile Asn
   275 280 285
   Lys Ile Ala Asp Glu Gly Asp Phe Tyr Glu Ile Gln Glu Asp Phe Ala
   290 295 300
   Lys Asn Ile Ile Thr Gly Phe Ile Arg Leu Glu Gly Gln Thr Val Gly
   305 310 315 320
   Val Val Ala Asn Gln Pro Met Ile Leu Ala Gly Cys Leu Asp Ile Asp
   325 330 335
   Ser Ser Arg Lys Ala Ala Arg Phe Val Arg Phe Cys Asp Cys Phe Glu
   340 345 350
   Ile Pro Ile Leu Thr Leu Val Asp Val Pro Gly Phe Leu Pro Gly Thr
   355 360 365
   Ser Gln Glu Tyr Gly Gly Val Ile Lys His Gly Ala Lys Leu Leu Phe
   370 375 380
   Ala Tyr Gly Glu Ala Thr Val Pro Lys Val Thr Val Ile Thr Arg Lys
   385 390 395 400
   Ala Tyr Gly Gly Ala Tyr Asp Val Met Ala Ser Lys His Leu Arg Gly
   405 410 415
   Asp Phe Asn Tyr Ala Trp Pro Thr Ala Glu Ile Ala Val Met Gly Ala
   420 425 430
   Lys Gly Ala Thr Glu Ile Ile His Arg Ala Asp Leu Gly Asp Ala Asp
   435 440 445
   Lys Ile Ala Ala His Thr Lys Asp Tyr Glu Gly Arg Phe Ala Asn Pro
   450 455 460
   Phe Val Ala Ala Glu Arg Gly Phe Ile Asp Glu Val Ile Gln Pro Arg
   465 470 475 480
   Ser Thr Arg Lys Arg Val Ser Arg Ala Phe Ala Ser Leu Arg Gly Lys
   485 490 495
   Ser Leu Lys Asn Pro Trp Lys Lys His Asp Asn Ile Pro Leu
   500 505 510
<210> 61
   <211> 678
   <212> PRT
   <213> Bacillus megaterium
<300>
   <308> NCBI / YP_003564880
   <309> 2010-12-17
   <313> (1)..(678)
<400> 61
   Met Lys Thr Asn Thr Leu Ser Phe His Glu Phe Thr Arg Thr Pro Lys
   1               5                   10                  15
   Glu Asp Trp Ala Gln Glu Val Ser Lys Asn Thr Ala Ile Ser Ser Lys
   20 25 30
   Glu Thr Leu Glu Asn Ile Phe Leu Lys Pro Leu Tyr Phe Glu Ser Asp
   35 40 45
   Thr Ala His Leu Asp Tyr Leu Gln Gln Ser Pro Ala Gly Ile Asp Tyr
   50 55 60
   Leu Arg Gly Ala Gly Lys Glu Ser Tyr Ile Leu Gly Glu Trp Glu Ile
   65 70 75 80
   Thr Gln Lys Ile Asp Leu Pro Ser Ile Lys Glu Ser Asn Lys Leu Leu
   85 90 95
   Leu His Ser Leu Arg Asn Gly Gln Asn Thr Ala Ala Phe Thr Cys Ser
   100 105 110
   Glu Ala Met Arg Gln Gly Lys Asp Ile Asp Glu Ala Thr Glu Ala Glu
   115 120 125
   Val Ala Ser Gly Ala Thr Ile Ser Thr Leu Glu Asp Val Ala His Leu
   130 135 140
   Phe Gln His Val Ala Leu Glu Ala Val Pro Leu Phe Leu Asn Thr Gly
   145 150 155 160
   Cys Thr Ser Val Pro Leu Leu Ser Phe Leu Lys Ala Tyr Cys Val Asp
   165 170 175
   His Asn Phe Asn Met Arg Gln Leu Lys Gly Thr Val Gly Met Asp Pro
   180 185 190
   Leu Gly Thr Leu Ala Glu Tyr Gly Arg Val Pro Leu Ser Thr Arg Asp
   195 200 205
   Leu Tyr Asp His Leu Ala Tyr Ala Thr Arg Leu Ala His Ser Asn Val
   210 215 220
   Pro Glu Leu Lys Thr Ile Ile Val Ser Ser Ile Pro Tyr His Asn Ser
   225 230 235 240
   Gly Ala Asn Ala Val Gln Glu Leu Ala Tyr Met Leu Ala Thr Gly Val
   245 250 255
   Gln Tyr Ile Asp Glu Cys Ile Lys Arg Gly Leu Ser Leu His Gln Val
   260 265 270
   Leu Pro His Met Thr Phe Ser Phe Ser Val Ser Ser His Leu Phe Met
   275 280 285
   Glu Ile Ser Lys Leu Arg Ala Phe Arg Met Leu Trp Ala Asn Val Val
   290 295 300
   Arg Ala Phe Asp Asp Thr Ala Val Ser Val Pro Phe Ile His Thr Glu
   305 310 315 320
   Thr Ser His Leu Thr Gln Ser Lys Glu Asp Met Tyr Thr Asn Ala Leu
   325 330 335
   Arg Ser Thr Val Gln Ala Phe Ala Ser Ile Val Gly Gly Ala Asp Ser
   340 345 350
   Leu His Ile Glu Pro Tyr Asp Ser Val Thr Ser Ser Ser Ser Gln Phe
   355 360 365
   Ala His Arg Leu Ala Arg Asn Thr His Leu Ile Leu Gln His Glu Thr
   370 375 380
   His Ile Ser Lys Val Met Asp Pro Ala Gly Gly Ser Trp Tyr Val Glu
   385 390 395 400
   Ala Tyr Thr His Glu Leu Met Thr Lys Ala Trp Glu Leu Phe Gly Asn
   405 410 415
   Ile Glu Asp His Gly Gly Met Glu Glu Ala Leu Lys Gln Gly Arg Ile
   420 425 430
   Gln Asp Glu Val Glu Gln Met Lys Val Lys Arg Gln Glu Asp Ile Glu
   435 440 445
   Cys Arg Ile Glu Arg Leu Ile Gly Val Thr His Tyr Ala Pro Lys Gln
   450 455 460
   Gln Asp Ala Ser Gln Glu Ile Lys Ser Thr Pro Phe Lys Lys Glu Glu
   465 470 475 480
   Ile Lys Met Asp Lys Tyr Ser Asp Gln Asn Ala Ser Glu Phe Ser Ser
   485 490 495
   Asn Leu Ser Leu Glu Asp Tyr Thr Lys Leu Ala Ser Lys Gly Val Thr
   500 505 510
   Ala Gly Trp Met Leu Lys Gln Met Ala Lys Gln Thr Gln Pro Asp Ser
   515 520 525
   Val Val Pro Leu Thr Lys Trp Arg Ala Ala Glu Lys Phe Glu Lys Ile
   530 535 540
   Arg Val Tyr Thr Lys Gly Met Ser Ile Gly Ile Met Glu Leu Thr Asp
   545 550 555 560
   Pro Ser Ser Arg Lys Lys Ala Glu Ile Ala Arg Ser Leu Phe Glu Ser
   565 570 575
   Ala Gly Phe Ala Cys Glu Thr Ile Lys Asn Ile Asp Ser Tyr Val Glu
   580 585 590
   Ile Ala Asp Trp Met Asn Glu Gln Lys His Glu Ala Tyr Val Ile Cys
   595 600 605
   Gly Ser Asp Glu Leu Val Glu Lys Leu Leu Thr Lys Ala Met Thr Tyr
   610 615 620
   Phe Glu Glu Asp Ser Val Tyr Val Tyr Val Val Gly Glu Glu His Val
   625 630 635 640
   Ser Arg Lys Thr Gln Trp Gln Gln Lys Gly Val Met Ser Val Ile His
   645 650 655
   Pro Lys Thr Asn Val Ile Gln Cys Val Lys Lys Leu Leu Cys Ala Leu
   660 665 670
   Glu Val Glu Val His Val
   675
<210> 62
   <211> 716
   <212> PRT
   <213> Bacillus megaterium
<300>
   <308> NCBI - YP_003564879
   <309> 2010-12-17
   <313> (1) .. (716)
<400> 62
   Met Tyr Lys Lys Pro Ser Phe Ser Asn Ile Pro Leu Ser Phe Ser Lys
   1               5                   10                  15
   Gln Gln Arg Glu Asp Asp Val Thr Gln Ser Ser Tyr Thr Ala Phe Gln
               20                  25                  30
   Thr Asn Glu Gln Ile Glu Leu Lys Ser Val Tyr Thr Lys Lys Asp Arg
           35                  40                  45
   Asp Asn Leu Asp Phe Ile His Phe Ala Pro Gly Val Pro Pro Phe Val
       50                  55                  60
   Arg Gly Pro Tyr Ala Thr Met Tyr Val Asn Arg Pro Trp Thr Ile Arg
   65 70 75 80
   Gln Tyr Ala Gly Tyr Ser Thr Ala Glu Glu Ser Asn Ala Phe Tyr Arg
   85 90 95
   Arg Asn Leu Ala Ala Gly Gln Lys Gly Leu Ser Val Ala Phe Asp Leu
   100 105 110
   Ala Thr His Arg Gly Tyr Asp Ser Asp His Pro Arg Val Val Gly Asp
   115 120 125
   Val Gly Lys Ala Gly Val Ala Ile Asp Ser Met Met Asp Met Lys Gln
   130 135 140
   Leu Phe Glu Gly Ile Pro Leu Asp Gln Met Ser Val Ser Met Thr Met
   145 150 155 160
   Asn Gly Ala Val Leu Pro Ile Leu Ala Phe Tyr Ile Val Thr Ala Glu
   165 170 175
   Glu Gln Gly Val Lys Lys Glu Lys Leu Ala Gly Thr Ile Gln Asn Asp
   180 185 190
   Ile Leu Lys Glu Tyr Met Val Arg Asn Thr Tyr Ile Tyr Pro Pro Glu
   195 200 205
   Met Ser Met Arg Ile Ile Ala Asp Ile Phe Lys Tyr Thr Ala Glu Tyr
   210 215 220
   Met Pro Lys Phe Asn Ser Ile Ser Ile Ser Gly Tyr His Met Gln Glu
   225 230 235 240
   Ala Gly Ala Pro Ala Asp Leu Glu Leu Ala Tyr Thr Leu Ala Asp Gly
   245 250 255
   Leu Glu Tyr Val Arg Thr Gly Leu Lys Ala Gly Ile Thr Ile Asp Ala
   260 265 270
   Phe Ala Pro Arg Leu Ser Phe Phe Trp Ala Ile Gly Met Asn Tyr Phe
   275 280 285
   Met Glu Val Ala Lys Met Arg Ala Gly Arg Leu Leu Trp Ala Lys Leu
   290 295 300
   Met Lys Gln Phe Glu Pro Asp Asn Pro Lys Ser Leu Ala Leu Arg Thr
   305 310 315 320
   His Ser Gln Thr Ser Gly Trp Ser Leu Thr Glu Gln Asp Pro Phe Asn
   325 330 335
   Asn Val Ile Arg Thr Cys Val Glu Ala Leu Ala Ala Val Ser Gly His
   340 345 350
   Thr Gln Ser Leu His Thr Asn Ala Leu Asp Glu Ala Ile Ala Leu Pro
   355 360 365
   Thr Asp Phe Ser Ala Arg Ile Ala Arg Asn Thr Gln Leu Tyr Leu Gln
   370 375 380
   Asn Glu Thr Glu Ile Cys Ser Val Ile Asp Pro Trp Gly Gly Ser Tyr
   385 390 395 400
   Tyr Val Glu Ser Leu Thr Asn Glu Leu Met Ile Lys Ala Trp Lys His
   405 410 415
   Leu Glu Glu Ile Glu Gln Leu Gly Gly Met Thr Lys Ala Ile Glu Ala
   420 425 430
   Gly Val Pro Lys Met Lys Ile Glu Glu Ala Ala Ala Arg Arg Gln Ala
   435 440 445
   Arg Ile Asp Ser Gln Ala Glu Ile Ile Val Gly Val Asn Gln Phe Gln
   450 455 460
   Pro Glu Gln Glu Glu Pro Leu Asp Ile Leu Asp Ile Asp Asn Thr Ala
   465 470 475 480
   Val Arg Met Lys Gln Leu Glu Lys Leu Lys Lys Ile Arg Ser Glu Arg
   485 490 495
   Asn Glu Gln Ala Val Ile Glu Ala Leu Asn Arg Leu Thr Asn Cys Ala
   500 505 510
   Lys Thr Gly Glu Gly Asn Leu Leu Ala Phe Ala Val Glu Ala Ala Arg
   515 520 525
   Ala Arg Ala Thr Leu Gly Glu Ile Ser Glu Ala Ile Glu Lys Val Ala
   530 535 540
   Gly Arg His Gln Ala Thr Ser Lys Ser Val Ser Gly Val Tyr Ser Ala
   545 550 555 560
   Glu Phe Val His Arg Asp Gln Ile Glu Glu Val Arg Lys Leu Thr Ala
   565 570 575
   Glu Phe Leu Glu Gly Glu Gly Arg Arg Pro Arg Ile Leu Val Ala Lys
   580 585 590
   Met Gly Gln Asp Gly His Asp Arg Gly Ser Lys Val Ile Ser Thr Ala
   595 600 605
   Phe Ala Asp Leu Gly Phe Asp Val Asp Ile Gly Pro Leu Phe Gln Thr
   610 615 620
   Pro Gln Glu Thr Ala Arg Gln Ala Val Glu Asn Asp Val His Val Ile
   625 630 635 640
   Gly Ile Ser Ser Leu Ala Ala Gly His Lys Thr Leu Leu Pro Gln Leu
   645 650 655
   Val Asp Glu Leu Lys Lys Leu Glu Arg Asp Asp Ile Val Val Ile Val
   660 665 670
   Gly Gly Val Ile Pro Lys Gln Asp Tyr Ser Phe Leu Leu Glu His Gly
   675 680 685
   Ala Ser Ala Ile Phe Gly Pro Gly Thr Val Ile Pro Lys Ala Ala Val
   690 695 700
   Ser Val Leu His Glu Ile Lys Lys Arg Leu Glu Glu
   705 710 715
<210> 63
   <211> 615
   <212> PRT
   <213> Mycobacterium tuberculosis
<300>
   <308> NCBI / YP_001282809
   <309> 2010-05-13
   <313> (1)..(615)
<400> 63
   Met Ser Ile Asp Val Pro Glu Arg Ala Asp Leu Glu Gln Val Arg Gly
   1               5                   10                  15
   Arg Trp Arg Asn Ala Val Ala Gly Val Leu Ser Lys Ser Asn Arg Thr
               20                  25                  30
   Asp Ser Ala Gln Leu Gly Asp His Pro Glu Arg Leu Leu Asp Thr Gln
           35                  40                  45
   Thr Ala Asp Gly Phe Ala Ile Arg Ala Leu Tyr Thr Ala Phe Asp Glu
       50                  55                  60
   Leu Pro Glu Pro Pro Leu Pro Gly Gln Trp Pro Phe Val Arg Gly Gly
   65                  70                  75                  80
   Asp Pro Leu Arg Asp Val His Ser Gly Trp Lys Val Ala Glu Ala Phe
                   85                  90                  95
   Pro Ala Asn Gly Ala Thr Ala Asp Thr Asn Ala Ala Val Leu Ala Ala
               100                 105                 110
   Leu Gly Glu Gly Val Ser Ala Leu Leu Ile Arg Val Gly Glu Ser Gly
           115                 120                 125
   Val Ala Pro Asp Arg Leu Thr Ala Leu Leu Ser Gly Val Tyr Leu Asn
       130                 135                 140
   Leu Ala Pro Val Ile Leu Asp Ala Gly Ala Asp Tyr Arg Pro Ala Cys
   145                 150                 155                 160
   Asp Val Met Leu Ala Leu Val Ala Gln Leu Asp Pro Gly Gln Arg Asp
                   165                 170                 175
   Thr Leu Ser Ile Asp Leu Gly Ala Asp Pro Leu Thr Ala Ser Leu Arg
               180                 185                 190
   Asp Arg Pro Ala Pro Pro Ile Glu Glu Val Val Ala Val Ala Ser Arg
           195                 200                 205
   Ala Ala Gly Glu Arg Gly Leu Arg Ala Ile Thr Val Asp Gly Pro Ala
       210                 215                 220
   Phe His Asn Leu Gly Ala Thr Ala Ala Thr Glu Leu Ala Ala Thr Val
   225                 230                 235                 240
   Ala Ala Ala Val Ala Tyr Leu Arg Val Leu Thr Glu Ser Gly Leu Val
                   245                 250                 255
   Val Ser Asp Ala Leu Arg Gln Ile Ser Phe Arg Leu Ala Ala Asp Asp
               260                 265                 270
   Asp Gln Phe Met Thr Leu Ala Lys Met Arg Ala Leu Arg Gln Leu Trp
           275                 280                 285
   Ala Arg Val Ala Glu Val Val Gly Asp Pro Gly Gly Gly Ala Ala Val
       290                 295                 300
   Val His Ala Glu Thr Ser Leu Pro Met Met Thr Gln Arg Asp Pro Trp
   305                 310                 315                 320
   Val Asn Met Leu Arg Cys Thr Leu Ala Ala Phe Gly Ala Gly Val Gly
                   325                 330                 335
   Gly Ala Asp Thr Val Leu Val His Pro Phe Asp Val Ala Ile Pro Gly
   340 345 350
   Gly Phe Pro Gly Thr Ala Ala Gly Phe Ala Arg Arg Ile Ala Arg Asn
   355 360 365
   Thr Gln Leu Leu Leu Leu Glu Glu Ser His Val Gly Arg Val Leu Asp
   370 375 380
   Pro Ala Gly Gly Ser Trp Phe Val Glu Glu Leu Thr Asp Arg Leu Ala
   385 390 395 400
   Arg Arg Ala Trp Gln Arg Phe Gln Ala Ile Glu Ala Arg Gly Gly Phe
   405 410 415
   Val Glu Ala His Asp Phe Leu Ala Gly Gln Ile Ala Glu Cys Ala Ala
   420 425 430
   Arg Arg Ala Asp Asp Ile Ala His Arg Arg Leu Ala Ile Thr Gly Val
   435 440 445
   Asn Glu Tyr Pro Asn Leu Gly Glu Pro Ala Leu Pro Pro Gly Asp Pro
   450 455 460
   Thr Ser Pro Val Arg Arg Tyr Ala Ala Gly Phe Glu Ala Leu Arg Asp
   465 470 475 480
   Arg Ser Asp His His Leu Ala Arg Thr Gly Ala Arg Pro Arg Val Leu
   485 490 495
   Leu Leu Pro Leu Gly Pro Leu Ala Glu His Asn Ile Arg Thr Thr Phe
   500 505 510
   Ala Thr Asn Leu Leu Ala Ser Gly Gly Ile Glu Ala Ile Asp Pro Gly
   515 520 525
   Thr Val Asp Ala Gly Thr Val Gly Asn Ala Val Ala Asp Ala Gly Ser
   530 535 540
   Pro Ser Val Ala Val Ile Cys Gly Thr Asp Ala Arg Tyr Arg Asp Glu
   545 550 555 560
   Val Ala Asp Ile Val Gln Ala Ala Arg Ala Ala Gly Val Ser Arg Val
   565 570 575
   Tyr Leu Ala Gly Pro Glu Lys Ala Leu Gly Asp Ala Ala His Arg Pro
   580 585 590
   Asp Glu Phe Leu Thr Ala Lys Ile Asn Val Val Gln Ala Leu Ser Asn
   595 600 605
   Leu Leu Thr Arg Leu Gly Ala
   610 615
<210> 64
   <211> 750
   <212> PRT
   <213> Mycobacterium tuberculosis
<300>
   <308> NCBI / YP_001282810
   <309> 2010-05-13
   <313> (1) .. (750)
<400> 64
<210> 65
   <211> 616
   <212> PRT
   <213> Corynebacterium glutamicum
<300>
   <308> NCBI / YP_225814
   <309> 2010-12-14
   <313> (1)..(616)
<400> 65
   Met Thr Asp Leu Thr Lys Thr Ala Val Pro Glu Glu Leu Ser Glu Asn
   1               5                   10                  15
   Leu Glu Thr Trp Tyr Lys Ala Val Ala Gly Val Phe Ala Arg Thr Gln
               20                  25                  30
   Lys Lys Asp Ile Gly Asp Ile Ala Val Asp Val Trp Lys Lys Leu Ile
           35                  40                  45
   Val Thr Thr Pro Asp Gly Val Asp Ile Asn Pro Leu Tyr Thr Arg Ala
       50                  55                  60
   Asp Glu Ser Gln Arg Lys Phe Thr Glu Val Pro Gly Glu Phe Pro Phe
   65                  70                  75                  80
   Thr Arg Gly Thr Thr Val Asp Gly Glu Arg Val Gly Trp Gly Val Thr
                   85                  90                  95
   Glu Thr Phe Gly His Asp Ser Pro Lys Asn Ile Asn Ala Ala Val Leu
               100                 105                 110
   Asn Ala Leu Asn Ser Gly Thr Thr Thr Leu Gly Phe Glu Phe Ser Glu
           115                 120                 125
   Glu Phe Thr Ala Ala Asp Leu Lys Val Ala Leu Glu Gly Val Tyr Leu
       130                 135                 140
   Asn Met Ala Pro Leu Leu Ile His Ala Gly Gly Ser Thr Ser Glu Val
   145                 150                 155                 160
   Ala Ala Ala Leu Tyr Thr Leu Ala Glu Glu Ala Gly Thr Phe Phe Ala
   165 170 175
   Ala Leu Thr Leu Gly Ser Arg Pro Leu Thr Ala Gln Val Asp Gly Ser
   180 185 190
   His Ser Asp Thr Ile Glu Glu Ala Val Gln Leu Ala Val Asn Ala Ser
   195 200 205
   Lys Arg Ala Asn Val Arg Ala Ile Leu Val Asp Gly Ser Ser Phe Ser
   210 215 220
   Asn Gln Gly Ala Ser Asp Ala Gln Glu Ile Gly Leu Ser Ile Ala Ala
   225 230 235 240
   Gly Val Asp Tyr Val Arg Arg Leu Val Asp Ala Gly Leu Ser Thr Glu
   245 250 255
   Ala Ala Leu Lys Gln Val Ala Phe Arg Phe Ala Val Thr Asp Glu Gln
   260 265 270
   Phe Ala Gln Ile Ser Lys Leu Arg Val Ala Arg Arg Leu Trp Ala Arg
   275 280 285
   Val Cys Glu Val Leu Gly Phe Pro Glu Leu Ala Val Ala Pro Gln His
   290 295 300
   Ala Val Thr Ala Arg Ala Met Phe Ser Gln Arg Asp Pro Trp Val Asn
   305 310 315 320
   Met Leu Arg Ser Thr Val Ala Ala Phe Ala Ala Gly Val Gly Gly Ala
   325 330 335
   Thr Asp Val Glu Val Arg Thr Phe Asp Asp Ala Ile Pro Asp Gly Val
   340 345 350
   Pro Gly Val Ser Arg Asn Phe Ala His Arg Ile Ala Arg Asn Thr Asn
   355 360 365
   Leu Leu Leu Leu Glu Glu Ser His Leu Gly His Val Val Asp Pro Ala
   370 375 380
   Gly Gly Ser Tyr Phe Val Glu Ser Phe Thr Asp Asp Leu Ala Glu Lys
   385 390 395 400
   Ala Trp Ala Val Phe Ser Gly Ile Glu Ala Glu Gly Gly Tyr Ser Ala
   405 410 415
   Ala Cys Ala Ser Gly Thr Val Thr Ala Met Leu Asp Gln Thr Trp Glu
   420 425 430
   Gln Thr Arg Ala Asp Val Ala Ser Arg Lys Lys Lys Leu Thr Gly Ile
   435 440 445
   Asn Glu Phe Pro Asn Leu Ala Glu Ser Pro Leu Pro Ala Asp Arg Arg
   450 455 460
   Val Glu Pro Ala Gly Val Arg Arg Trp Ala Ala Asp Phe Glu Ala Leu
   465 470 475 480
   Arg Asn Arg Ser Asp Ala Phe Leu Glu Lys Asn Gly Ala Arg Pro Gln
   485 490 495
   Ile Thr Met Ile Pro Leu Gly Pro Leu Ser Lys His Asn Ile Arg Thr
   500 505 510
   Gly Phe Thr Ser Asn Leu Leu Ala Ser Gly Gly Ile Glu Ala Ile Asn
   515 520 525
   Pro Gly Gln Leu Val Pro Gly Thr Asp Ala Phe Ala Glu Ala Ala Gln
   530 535 540
   Ala Ala Gly Ile Val Val Val Cys Gly Thr Asp Gln Glu Tyr Ala Glu
   545 550 555 560
   Thr Gly Glu Gly Ala Val Glu Lys Leu Arg Glu Ala Gly Val Glu Arg
   565 570 575
   Ile Leu Leu Ala Gly Ala Pro Lys Ser Phe Glu Gly Ser Ala His Ala
   580 585 590
   Pro Asp Gly Tyr Leu Asn Met Thr Ile Asp Ala Ala Ala Thr Leu Ala
   595 600 605
   Asp Leu Leu Asp Ala Leu Gly Ala
   610 615
<210> 66
   <211> 737
   <212> PRT
   <213> Corynebacterium glutamicum
<300>
   <308> NCBI / YP_225813
   <309> 2010-12-14
<313> (1) .. (737)
<400> 66
   Met Thr Ser Ile Pro Asn Phe Ser Asp Ile Pro Leu Thr Ala Glu Thr
   1               5                   10                  15
   Arg Ala Ser Glu Ser His Asn Val Asp Ala Gly Lys Val Trp Asn Thr
   20 25 30
   Pro Glu Gly Ile Asp Val Lys Arg Val Phe Thr Gln Ala Asp Arg Asp
   35 40 45
   Glu Ala Gln Ala Ala Gly His Pro Val Asp Ser Leu Pro Gly Gln Lys
   50 55 60
   Pro Phe Met Arg Gly Pro Tyr Pro Thr Met Tyr Thr Asn Gln Pro Trp
   65 70 75 80
   Thr Ile Arg Gln Tyr Ala Gly Phe Ser Thr Ala Ala Glu Ser Asn Ala
   85 90 95
   Phe Tyr Arg Arg Asn Leu Ala Ala Gly Gln Lys Gly Leu Ser Val Ala
   100 105 110
   Phe Asp Leu Ala Thr His Arg Gly Tyr Asp Ser Asp Asn Glu Arg Val
   115 120 125
   Val Gly Asp Val Gly Met Ala Gly Val Ala Ile Asp Ser Ile Leu Asp
   130 135 140
   Met Arg Gln Leu Phe Asp Gly Ile Asp Leu Ser Ser Val Ser Val Ser
   145 150 155 160
   Met Thr Met Asn Gly Ala Val Leu Pro Ile Leu Ala Phe Tyr Ile Val
   165 170 175
   Ala Ala Glu Glu Gln Gly Val Gly Pro Glu Gln Leu Ala Gly Thr Ile
   180 185 190
   Gln Asn Asp Ile Leu Lys Glu Phe Met Val Arg Asn Thr Tyr Ile Tyr
   195 200 205
   Pro Pro Lys Pro Ser Met Arg Ile Ile Ser Asn Ile Phe Glu Tyr Thr
   210 215 220
   Ser Leu Lys Met Pro Arg Phe Asn Ser Ile Ser Ile Ser Gly Tyr His
   225 230 235 240
   Ile Gln Glu Ala Gly Ala Thr Ala Asp Leu Glu Leu Ala Tyr Thr Leu
   245 250 255
   Ala Asp Gly Ile Glu Tyr Ile Arg Ala Gly Lys Glu Val Gly Leu Asp
   260 265 270
   Val Asp Lys Phe Ala Pro Arg Leu Ser Phe Phe Trp Gly Ile Ser Met
   275 280 285
   Tyr Thr Phe Met Glu Ile Ala Lys Leu Arg Ala Gly Arg Leu Leu Trp
   290 295 300
   Ser Glu Leu Val Ala Lys Phe Asp Pro Lys Asn Ala Lys Ser Gln Ser
   305 310 315 320
   Leu Arg Thr His Ser Gln Thr Ser Gly Trp Ser Leu Thr Ala Gln Asp
   325 330 335
   Val Tyr Asn Asn Val Ala Arg Thr Ala Ile Glu Ala Met Ala Ala Thr
   340 345 350
   Gln Gly His Thr Gln Ser Leu His Thr Asn Ala Leu Asp Glu Ala Leu
   355 360 365
   Ala Leu Pro Thr Asp Phe Ser Ala Arg Ile Ala Arg Asn Thr Gln Leu
   370 375 380
   Leu Leu Gln Gln Glu Ser Gly Thr Val Arg Pro Val Asp Pro Trp Ala
   385 390 395 400
   Gly Ser Tyr Tyr Val Glu Trp Leu Thr Asn Glu Leu Ala Asn Arg Ala
   405 410 415
   Arg Lys His Ile Asp Glu Val Glu Glu Ala Gly Gly Met Ala Gln Ala
   420 425 430
   Thr Ala Gln Gly Ile Pro Lys Leu Arg Ile Glu Glu Ser Ala Ala Arg
   435 440 445
   Thr Gln Ala Arg Ile Asp Ser Gly Arg Gln Ala Leu Ile Gly Val Asn
   450 455 460
   Arg Tyr Val Ala Glu Glu Asp Glu Glu Ile Glu Val Leu Lys Val Asp
   465 470 475 480
   Asn Thr Lys Val Arg Ala Glu Gln Leu Ala Lys Leu Ala Gln Leu Lys
   485 490 495
   Ala Glu Arg Asn Asp Ala Glu Val Lys Ala Ala Leu Asp Ala Leu Thr
   500 505 510
   Ala Ala Ala Arg Asn Glu His Lys Glu Pro Gly Asp Leu Asp Gln Asn
   515 520 525
   Leu Leu Lys Leu Ala Val Asp Ala Ala Arg Ala Lys Ala Thr Ile Gly
   530 535 540
   Glu Ile Ser Asp Ala Leu Glu Val Val Phe Gly Arg His Glu Ala Glu
   545 550 555 560
   Ile Arg Thr Leu Ser Gly Val Tyr Lys Asp Glu Val Gly Lys Glu Gly
   565 570 575
   Thr Val Ser Asn Val Glu Arg Ala Ile Ala Leu Ala Asp Ala Phe Glu
   580 585 590
   Ala Glu Glu Gly Arg Arg Pro Arg Ile Phe Ile Ala Lys Met Gly Gln
   595 600 605
   Asp Gly His Asp Arg Gly Gln Lys Val Val Ala Ser Ala Tyr Ala Asp
   610 615 620
   Leu Gly Met Asp Val Asp Val Gly Pro Leu Phe Gln Thr Pro Ala Glu
   625 630 635 640
   Ala Ala Arg Ala Ala Val Asp Ala Asp Val His Val Val Gly Met Ser
   645 650 655
   Ser Leu Ala Ala Gly His Leu Thr Leu Leu Pro Glu Leu Lys Lys Glu
   660 665 670
   Leu Ala Ala Leu Gly Arg Asp Asp Ile Leu Val Thr Val Gly Gly Val
   675 680 685
   Ile Pro Pro Gly Asp Phe Gln Asp Leu Tyr Asp Met Gly Ala Ala Ala
   690 695 700
   Ile Tyr Pro Pro Gly Thr Val Ile Ala Glu Ser Ala Ile Asp Leu Ile
   705 710 715 720
   Thr Arg Leu Ala Ala His Leu Gly Phe Asp Leu Asp Val Asp Val Asn
   725 730 735
   Glu
<210> 67
   <211> 631
   <212> PRT
   <213> Rhodococcus erythropolis
<300>
   <308> NCBI / YP_002766535
   <309> 2010-05-12
<313> (1)..(631)
<400> 67
   Met Ser Leu Ala Ser Glu Ala Glu Ala Val Glu Gln Ala Tyr Ala Glu
   1 5 10 15
   Trp Gln Arg Ser Val Ala Gly Val Leu Ala Lys Ser Arg Arg Val Asp
   20 25 30
   Ala Ala Glu Leu Gly Pro Glu Pro Gln Lys Leu Leu Glu Thr Val Thr
   35 40 45
   Tyr Asp Gly Val Thr Val Ala Pro Leu Tyr Ser Pro Arg Asp Glu Arg
   50 55 60
   Pro Glu Gln Ser Leu Pro Gly Thr Phe Pro Tyr Val Arg Gly Val Asp
   65 70 75 80
   Ala His Arg Asp Val Asn Ala Gly Trp Leu Val Ser Ala Ala Phe Gly
   85 90 95
   Thr Ala Ser Ala Ala Glu Thr Asn Arg Ala Ile Leu Asp Ala Leu Glu
   100 105 110
   Asn Gly Val Ser Ala Leu Trp Leu Lys Val Gly Ala Asp Gly Val Pro
   115 120 125
   Val Thr Asp Leu Ala Ala Ala Leu Glu Gly Val Leu Leu Asp Leu Ala
   130 135 140
   Pro Leu Thr Leu Asp Ala Gly Ala Glu Val Asn Asp Ala Ala Arg Ala
   145 150 155 160
   Leu Phe Ser Leu Leu Asp Ala Arg Gly Glu Ala Gly Asp Gly Val Ser
   165 170 175
   Asp Arg Ser Ser Ile Arg Val His Leu Gly Ala Ala Pro Leu Thr Ser
   180 185 190
   Ser Phe Ser Gly Ala Ala Asp Val Glu Phe Ala Gly Ala Val Glu Leu
   195 200 205
   Ala Ala Leu Ala Ala Ala Arg Ala Glu Thr Val His Ala Ile Thr Val
   210 215 220
   Asp Gly Thr Ala Phe His Asn Ala Gly Ala Gly Asp Ala Glu Glu Leu
   225 230 235 240
   Gly Ala Ala Ile Ala Ala Gly Leu Glu Tyr Leu Arg Ala Leu Thr Ala
   245 250 255
   Glu Ser Gly Leu Thr Ile Gly Ala Ala Leu Ser Gln Leu Ala Phe Arg
               260                 265                 270
   Tyr Ser Ala Thr Asp Asp Gln Phe Gln Thr Ile Ala Lys Phe Arg Ala
           275                 280                 285
   Ala Arg Leu Val Trp Ala Arg Ile Ala Gln Val Cys Gly Ala Ser Asp
       290                 295                 300
   Phe Gly Gly Ala Pro Gln His Ala Val Thr Ser Ala Ala Met Met Ala
   305                 310                 315                 320
   Gln Arg Asp Pro Trp Val Asn Met Leu Arg Thr Thr Leu Ala Ala Phe
                   325                 330                 335
   Gly Ala Gly Val Gly Gly Ala Asp Ala Val Thr Val Leu Pro Phe Asp
               340                 345                 350
   Val Ala Leu Ala Asp Gly Thr Leu Gly Val Ser Lys Ser Phe Ser Ser
           355                 360                 365
   Arg Ile Ala Arg Asn Thr Gln Leu Leu Leu Leu Glu Glu Ser His Leu
       370                 375                 380
   Gly Arg Val Leu Asp Pro Ser Ala Gly Ser Trp Tyr Val Glu Asp Leu
   385                 390                 395                 400
   Thr Gln Gln Ile Ala Ala Thr Ala Trp Glu Phe Phe Gln Glu Ile Glu
                   405                 410                 415
   Ala Ala Gly Gly Tyr Leu Ala Ala Leu Glu Ala Gly Ile Val Ser Gly
               420                 425                 430
   Arg Ile Ala Ala Thr Lys Ala Lys Arg Asp Ser Asp Ile Ala His Arg
           435                 440                 445
   Lys Thr Thr Val Thr Gly Val Asn Glu Phe Pro Asn Leu Gly Glu Thr
       450                 455                 460
   Pro Leu Ser Ala Glu Ala Val Glu Pro Gly Gln Ser Val Ala Arg Tyr
   465                 470                 475                 480
   Ala Ala Ala Phe Glu Ala Leu Arg Asp Arg Ser Asp Ala Phe Leu Ala
                   485                 490                 495
   Ala Gly Gly Ala Arg Pro Thr Ala Leu Leu Ala Pro Leu Gly Ser Val
               500                 505                 510
   Ala Glu His Asn Val Arg Thr Thr Phe Ala Ser Asn Leu Leu Ala Ser
   515 520 525
   Gly Gly Ile Asp Ala Val Asn Pro Gly Pro Leu Glu Val Gly Ala Glu
   530 535 540
   Ala Ile Ser Ala Ala Val Lys Ala Ser Gly Val Thr Val Ala Val Leu
   545 550 555 560
   Cys Gly Thr Asp Lys Arg Tyr Gly Glu Ser Ala Ala Ala Ala Val Ala
   565 570 575
   Glu Leu Arg Ala Ala Gly Ile Thr Lys Val Leu Leu Ala Gly Pro Glu
   580 585 590
   Lys Ala Val Ala Asp Ala Thr Gly Glu Ser Arg Pro Asp Gly Phe Leu
   595 600 605
   Thr Ala Arg Ile Asp Ala Val Ser Ala Leu Thr Glu Leu Leu Asp Phe
   610 615 620
   Ile Glu Thr Gly Ser Ser Lys
   625 630
<210> 68
   <211> 750
   <212> PRT
   <213> Rhodococcus erythropolis
<300>
   <308> NCBI / YP_002766536
   <309> 2010-05-12
<313> (1) .. (750)
<400> 68
   Met Thr Thr Arg Glu Val Lys His Val Ile Gly Ser Phe Ala Glu Val
   1               5                   10                  15
   Pro Leu Glu Asp Pro Gln Ser Pro Ala Pro Thr Pro Pro Ser Val Glu
               20                  25                  30
   Gln Ala Gln Ala Leu Ile Glu Glu Gly Ala Asn Ala Asn Asn Tyr Ala
           35                  40                  45
   Ala Glu Gln Val Val Trp Ser Thr Pro Glu Gly Ile Asp Val Lys Pro
       50                  55                  60
   Val Tyr Thr Gly Ala Asp Arg Thr Ala Ala Ala Glu Ser Gly Tyr Pro
   65                  70                  75                      80
   Leu Asp Ser Phe Pro Gly Ala Ala Pro Phe Leu Arg Gly Pro Tyr Pro
   85 90 95
   Thr Met Tyr Val Asn Gln Pro Trp Thr Ile Arg Gln Tyr Ala Gly Phe
   100 105 110
   Ser Thr Ala Ala Glu Ser Asn Ala Phe Tyr Arg Arg Asn Leu Ala Ala
   115 120 125
   Gly Gln Lys Gly Leu Ser Val Ala Phe Asp Leu Ala Thr His Arg Gly
   130 135 140
   Tyr Asp Ser Asp His Pro Arg Val Ala Gly Asp Val Gly Met Ala Gly
   145 150 155 160
   Val Ala Ile Asp Ser Ile Leu Asp Met Arg Gln Leu Phe Asp Gly Ile
   165 170 175
   Asp Leu Ser Gln Val Ser Val Ser Met Thr Met Asn Gly Ala Val Leu
   180 185 190
   Pro Ile Leu Ala Leu Tyr Val Ala Ala Ala Gly Glu Gln Gly Val Thr
   195 200 205
   Pro Asp Lys Leu Ala Gly Thr Ile Gln Asn Asp Ile Leu Lys Glu Phe
   210 215 220
   Met Val Arg Asn Thr Tyr Ile Tyr Pro Pro Lys Pro Ser Met Arg Ile
   225 230 235 240
   Ile Ser Asp Ile Phe Ala Tyr Ser Ser Ala Glu Met Pro Lys Tyr Asn
   245 250 255
   Ser Ile Ser Ile Ser Gly Tyr His Ile Gln Glu Ala Gly Ala Thr Ala
   260 265 270
   Asp Leu Glu Leu Ala Tyr Thr Leu Ala Asp Gly Val Glu Tyr Ile Arg
   275 280 285
   Ala Gly Leu Asp Ala Gly Met Asp Ile Asp Lys Phe Ala Pro Arg Leu
   290 295 300
   Ser Phe Phe Trp Ala Ile Gly Met Asn Phe Phe Met Glu Val Ala Lys
   305 310 315 320
   Leu Arg Ala Gly Arg Leu Leu Trp Ala Glu Leu Val Ala Lys Phe Asp
   325 330 335
   Pro Lys Ser Ala Lys Ser Leu Ser Leu Arg Thr His Ser Gln Thr Ser
   340 345 350
   Gly Trp Ser Leu Thr Ala Gln Asp Val Phe Asn Asn Val Pro Arg Thr
   355 360 365
   Cys Val Glu Ala Met Ala Ala Thr Gln Gly His Thr Gln Ser Leu His
   370 375 380
   Thr Asn Ala Leu Asp Glu Ala Ile Ala Leu Pro Thr Asp Phe Ser Ala
   385 390 395 400
   Arg Ile Ala Arg Asn Thr Gln Leu Leu Leu Gln Gln Glu Ser Gly Thr
   405 410 415
   Val Arg Pro Ile Asp Pro Trp Gly Gly Ser Tyr Tyr Val Glu Trp Leu
   420 425 430
   Thr Asn Glu Leu Ala Asn Arg Ala Arg Lys His Ile Glu Glu Val Glu
   435 440 445
   Glu Ala Gly Gly Met Ala Gln Ala Ile Asn Glu Gly Ile Pro Lys Leu
   450 455 460
   Arg Ile Glu Glu Ala Ala Ala Arg Thr Gln Ala Arg Ile Asp Ser Gly
   465 470 475 480
   Arg Gln Pro Leu Val Gly Val Asn Lys Tyr Val Pro Asp Glu Val Asp
   485 490 495
   Thr Ile Glu Val Leu Lys Val Glu Asn Ser Lys Val Arg Lys Glu Gln
   500 505 510
   Leu Glu Lys Leu Val Arg Leu Arg Ala Glu Arg Asp Pro Glu Ala Val
   515 520 525
   Glu Ala Ala Leu Ala Asn Leu Thr Arg Ala Ala Ala Ser Thr Glu Gly
   530 535 540
   Gly Met Glu Asn Asn Leu Leu Ala Leu Ala Val Val Ala Ala Arg Ala
   545 550 555 560
   Met Ala Thr Val Gly Glu Ile Ser Asp Ala Leu Glu Lys Val Tyr Gly
   565 570 575
   Arg His Gln Ala Glu Ile Arg Thr Ile Ser Gly Val Tyr Arg Asp Glu
   580 585 590
   Ala Gly Thr Val Ser Asn Ile Ser Lys Ala Met Glu Leu Val Glu Lys
   595 600 605
   Phe Ala Glu Asp Glu Gly Arg Arg Pro Arg Ile Leu Val Ala Lys Met
   610 615 620
   Gly Gln Asp Gly His Asp Arg Gly Gln Lys Val Ile Ser Thr Ala Phe
   625 630 635 640
   Ala Asp Ile Gly Phe Asp Val Asp Val Gly Pro Leu Phe Gln Thr Pro
   645 650 655
   Glu Glu Val Ala Asn Gln Ala Ala Asp Asn Asp Val His Val Val Gly
   660 665 670
   Val Ser Ser Leu Ala Ala Gly His Leu Thr Leu Val Pro Ala Leu Arg
   675 680 685
   Glu Ala Leu Ala Ala Ala Gly Arg Pro Asp Ile Met Ile Val Val Gly
   690 695 700
   Gly Val Ile Pro Pro Gly Asp Phe Asp Glu Leu Tyr Glu Ala Gly Ala
   705 710 715 720
   Ala Ala Ile Phe Pro Pro Gly Thr Val Ile Ala Asp Ala Ala Ser Gly
   725 730 735
   Leu Leu Glu Lys Leu Ser Ala Gln Leu Gly His Asp His Ser
   740 745 750
<210> 69
   <211> 618
   <212> PRT
   <213> Porphyromonas gingivalis
<300>
   <308> NCBI / NP_905776
   <309> 2010-06-29
<313> (1) .. (618)
<400> 69
   Met Ala Lys Glu Lys Glu Lys Leu Phe Ser Glu Phe Pro Pro Val Ser
   1               5                   10                  15
   Arg Glu Ala Trp Ile Asp Lys Ile Thr Ala Asp Leu Lys Gly Val Pro
               20                  25                  30
   Phe Glu Lys Lys Leu Val Trp Arg Thr Asn Glu Gly Phe Asn Val Asn
           35                  40                  45
   Pro Phe Tyr Arg Arg Glu Asp Ile Glu Asp Leu Lys Thr Thr Thr Ser
       50                  55                  60
   Leu Pro Asp Glu Tyr Pro Tyr Val Arg Ser Thr Arg Met His Asn Glu
   65 70 75 80
   Trp Leu Val Arg Gln Asp Ile Val Val Gly Asp Asn Val Ala Glu Ala
   85 90 95
   Asn Glu Lys Ala Leu Asp Leu Leu Asn Lys Gly Val Asp Ser Leu Gly
   100 105 110
   Phe Tyr Leu Lys Lys Val His Ile Asn Val Asp Thr Leu Ala Ala Leu
   115 120 125
   Leu Lys Asp Ile Glu Leu Thr Ala Val Glu Leu Asn Phe Asn Cys Cys
   130 135 140
   Ile Thr Arg Ala Ala Asp Leu Leu Ser Ala Phe Ser Ala Tyr Val Lys
   145 150 155 160
   Lys Val Gly Ala Asp Pro Asn Lys Cys His Gly Ser Val Ser Tyr Asp
   165 170 175
   Pro Phe Lys Lys Gln Leu Val Arg Gly Val Ser Asn Pro Asp Trp Val
   180 185 190
   Lys Met Thr Leu Pro Val Met Asp Ala Ala Arg Glu Leu Pro Ala Phe
   195 200 205
   Arg Val Leu Asn Val Asn Ala Val Asn Leu Ser Asp Ala Gly Ala Phe
   210 215 220
   Ile Thr Gln Glu Leu Gly Tyr Ala Leu Ala Trp Gly Ala Glu Leu Leu
   225 230 235 240
   Asp Lys Leu Thr Asp Ala Gly Tyr Lys Pro Glu Glu Ile Ala Ser Arg
   245 250 255
   Ile Lys Phe Asn Phe Gly Ile Gly Ser Asn Tyr Phe Met Glu Ile Ala
   260 265 270
   Lys Phe Arg Ala Ala Arg Trp Leu Trp Ala Gln Ile Val Gly Ser Tyr
   275 280 285
   Gly Asp Gln Tyr Lys Asn Glu Thr Ala Lys Ile His Gln His Ala Thr
   290 295 300
   Thr Ser Met Trp Asn Lys Thr Val Phe Asp Ala His Val Asn Leu Leu
   305 310 315 320
   Arg Thr Gln Thr Glu Thr Met Ser Ala Ala Ile Ala Gly Val Asp Ser
   325 330 335
   Ile Thr Val Leu Pro Phe Asp Val Thr Tyr Gln Gln Ser Asp Asp Phe
   340 345 350
   Ser Glu Arg Ile Ala Arg Asn Gln Gln Leu Leu Leu Lys Glu Glu Cys
   355 360 365
   His Phe Asp Lys Val Ile Asp Pro Ser Ala Gly Ser Tyr Tyr Ile Glu
   370 375 380
   Thr Leu Thr Asn Ser Ile Gly Glu Glu Ala Trp Lys Leu Phe Leu Ser
   385 390 395 400
   Val Glu Asp Ala Gly Gly Phe Thr Gln Ala Ala Glu Thr Ala Ser Ile
   405 410 415
   Gln Lys Ala Val Asn Ala Ser Asn Ile Lys Arg His Gln Ser Val Ala
   420 425 430
   Thr Arg Arg Glu Ile Phe Leu Gly Thr Asn Gln Phe Pro Asn Phe Thr
   435 440 445
   Glu Val Ala Gly Asp Lys Ile Thr Leu Ala Gln Gly Glu His Asp Cys
   450 455 460
   Asn Cys Val Lys Ser Ile Glu Pro Leu Asn Phe Ser Arg Gly Ala Ser
   465 470 475 480
   Glu Phe Glu Ala Leu Arg Leu Ala Thr Glu Lys Ser Gly Lys Thr Pro
   485 490 495
   Val Val Phe Met Leu Thr Ile Gly Asn Leu Ala Met Arg Leu Ala Arg
   500 505 510
   Ser Gln Phe Ser Ser Asn Phe Phe Gly Cys Ala Gly Tyr Lys Leu Ile
   515 520 525
   Asp Asn Leu Gly Phe Lys Ser Val Glu Glu Gly Val Asp Ala Ala Leu
   530 535 540
   Ala Ala Lys Ala Asp Ile Val Val Leu Cys Ser Ser Asp Asp Glu Tyr
   545 550 555 560
   Ala Glu Tyr Ala Pro Ala Ala Phe Asp Tyr Leu Ala Gly Arg Ala Glu
   565 570 575
   Phe Val Val Ala Gly Ala Pro Ala Cys Met Ala Asp Leu Glu Ala Lys
   580 585 590
   Gly Ile Arg Asn Tyr Val His Val Lys Ser Asn Val Leu Glu Thr Leu
   595 600 605
   Arg Ala Phe Asn Asp Lys Phe Gly Ile Arg
   610 615
<210> 70
   <211> 715
   <212> PRT
   <213> Porphyromonas gingivalis
<300>
   <308> NCBI / NP_905777
   <309> 2010-06-29
   <313> (1) .. (715)
<400> 70
   Met Lys Pro Asn Tyr Lys Asp Ile Asp Ile Lys Ser Ala Gly Phe Val
   1               5                   10                  15
   Ala Lys Asp Ala Thr Arg Trp Ala Glu Glu Lys Gly Ile Val Ala Asp
               20                  25                  30
   Trp Arg Thr Pro Glu Gln Ile Met Val Lys Pro Leu Tyr Thr Lys Asp
           35                  40                  45
   Asp Leu Glu Gly Met Glu His Leu Asp Tyr Val Ser Gly Leu Pro Pro
       50                  55                  60
   Phe Leu Arg Gly Pro Tyr Ser Gly Met Tyr Pro Met Arg Pro Trp Thr
   65                  70                  75                  80
   Ile Arg Gln Tyr Ala Gly Phe Ser Thr Ala Glu Glu Ser Asn Ala Phe
                   85                  90                  95
   Tyr Arg Arg Asn Leu Ala Ser Gly Gln Lys Gly Leu Ser Val Ala Phe
               100                 105                 110
   Asp Leu Ala Thr His Arg Gly Tyr Asp Ala Asp His Ser Arg Val Val
           115                 120                 125
   Gly Asp Val Gly Lys Ala Gly Val Ser Ile Cys Ser Leu Glu Asp Met
       130                 135                 140
   Lys Val Leu Phe Asp Gly Ile Pro Leu Ser Lys Met Ser Val Ser Met
   145                 150                 155                 160
   Thr Met Asn Gly Ala Val Leu Pro Ile Leu Ala Phe Tyr Ile Asn Ala
                   165                 170                 175
   Gly Leu Glu Gln Gly Ala Lys Leu Glu Glu Met Ala Gly Thr Ile Gln
   180 185 190
   Asn Asp Ile Leu Lys Glu Phe Met Val Arg Asn Thr Tyr Ile Tyr Pro
   195 200 205
   Pro Glu Phe Ser Met Arg Ile Ile Ala Asp Ile Phe Glu Tyr Thr Ser
   210 215 220
   Gln Asn Met Pro Lys Phe Asn Ser Ile Ser Ile Ser Gly Tyr His Met
   225 230 235 240
   Gln Glu Ala Gly Ala Thr Ala Asp Ile Glu Met Ala Tyr Thr Leu Ala
   245 250 255
   Asp Gly Met Gln Tyr Leu Lys Ala Gly Ile Asp Ala Gly Ile Asp Val
   260 265 270
   Asp Ala Phe Ala Pro Arg Leu Ser Phe Phe Trp Ala Ile Gly Val Asn
   275 280 285
   His Phe Met Glu Ile Ala Lys Met Arg Ala Ala Arg Leu Leu Trp Ala
   290 295 300
   Lys Ile Val Lys Ser Phe Gly Ala Lys Asn Pro Lys Ser Leu Ala Leu
   305 310 315 320
   Arg Thr His Ser Gln Thr Ser Gly Trp Ser Leu Thr Glu Gln Asp Pro
   325 330 335
   Phe Asn Asn Val Gly Arg Thr Cys Ile Glu Ala Met Ala Ala Ala Leu
   340 345 350
   Gly His Thr Gln Ser Leu His Thr Asn Ala Leu Asp Glu Ala Ile Ala
   355 360 365
   Leu Pro Thr Asp Phe Ser Ala Arg Ile Ala Arg Asn Thr Gln Ile Tyr
   370 375 380
   Ile Gln Glu Glu Thr Leu Val Cys Lys Glu Ile Asp Pro Trp Gly Gly
   385 390 395 400
   Ser Tyr Tyr Val Glu Ser Leu Thr Asn Glu Leu Val His Lys Ala Trp
   405 410 415
   Thr Leu Ile Lys Glu Val Gln Glu Met Gly Gly Met Ala Lys Ala Ile
   420 425 430
   Glu Thr Gly Leu Pro Lys Leu Arg Ile Glu Glu Ala Ala Ala Arg Thr
   435 440 445
   Gln Ala Arg Ile Asp Ser His Gln Gln Val Ile Val Gly Val Asn Lys
   450 455 460
   Tyr Arg Leu Pro Lys Glu Asp Pro Ile Asp Ile Leu Glu Ile Asp Asn
   465 470 475 480
   Thr Ala Val Arg Lys Gln Gln Ile Glu Arg Leu Asn Asp Leu Arg Ser
   485 490 495
   His Arg Asp Glu Lys Ala Val Gln Glu Ala Leu Glu Ala Ile Thr Lys
   500 505 510
   Cys Val Glu Thr Lys Glu Gly Asn Leu Leu Asp Leu Ala Val Lys Ala
   515 520 525
   Ala Gly Leu Arg Ala Ser Leu Gly Glu Ile Ser Asp Ala Cys Glu Lys
   530 535 540
   Val Val Gly Arg Tyr Lys Ala Val Ile Arg Thr Ile Ser Gly Val Tyr
   545 550 555 560
   Ser Ser Glu Ser Gly Glu Asp Lys Asp Phe Ala His Ala Lys Glu Leu
   565 570 575
   Ala Glu Lys Phe Ala Lys Lys Glu Gly Arg Gln Pro Arg Ile Met Ile
   580 585 590
   Ala Lys Met Gly Gln Asp Gly His Asp Arg Gly Ala Lys Val Val Ala
   595 600 605
   Thr Gly Tyr Ala Asp Cys Gly Phe Asp Val Asp Met Gly Pro Leu Phe
   610 615 620
   Gln Thr Pro Glu Glu Ala Ala Arg Gln Ala Val Glu Asn Asp Val His
   625 630 635 640
   Val Met Gly Val Ser Ser Leu Ala Ala Gly His Lys Thr Leu Ile Pro
   645 650 655
   Gln Val Ile Ala Glu Leu Glu Lys Leu Gly Arg Pro Asp Ile Leu Val
   660 665 670
   Thr Ala Gly Gly Val Ile Pro Ala Gln Asp Tyr Asp Phe Leu Tyr Gln
   675 680 685
   Ala Gly Val Ala Ala Ile Phe Gly Pro Gly Thr Pro Val Ala Tyr Ser
   690 695 700
   Ala Ala Lys Val Leu Glu Ile Leu Leu Glu Glu
   705 710 715